(19) Europäisches Patentamt / European Patent Office / Office européen des brevets

(11) **EP 3 400 928 B1**

(12) **EUROPEAN PATENT SPECIFICATION**

(45) Date of publication and mention
of the grant of the patent:
**21.04.2021 Bulletin 2021/16**

(51) Int Cl.:
***A61J 7/00*** *(2006.01)*

(21) Application number: **18181874.1**

(22) Date of filing: **21.12.2012**

(54) **SYSTEM, METHOD, AND APPARATUS FOR MONITORING, REGULATING, OR CONTROLLING FLUID FLOW**

SYSTEM, VERFAHREN UND VORRICHTUNG ZUR ÜBERWACHUNG, REGELUNG ODER STEUERUNG EINES FLÜSSIGKEITSSTROMS

SYSTÈME, PROCÉDÉ ET APPAREIL PERMETTANT DE SURVEILLER, RÉGULER OU CONTRÔLER L'ÉCOULEMENT DE FLUIDE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority:
**21.12.2011 US 201161578649 P
21.12.2011 US 201161578658 P
21.12.2011 US 201161578674 P
21.12.2011 US 201113333574
21.12.2011 PCT/US2011/066588
24.05.2012 US 201261651322 P
03.08.2012 US 201261679117 P**

(43) Date of publication of application:
**14.11.2018 Bulletin 2018/46**

(62) Document number(s) of the earlier application(s) in accordance with Art. 76 EPC:
**16167576.4 / 3 081 205
15192051.9 / 3 006 010
12826640.0 / 2 793 977**

(73) Proprietor: **DEKA Products Limited Partnership
Manchester, NH 03101 (US)**

(72) Inventors:
• **KAMEN, Dean**
  **Bedford, New Hampshire 03110 (US)**
• **PERET, Bob D**
  **Bedford, New Hampshire 03110 (US)**
• **KANE, Derek G**
  **Manchester, New Hampshire 03104 (US)**
• **YOO, Brian H**
  **Cambridge, Massachusetts 02140 (US)**
• **TRACEY, Brian D**
  **Litchfield, New Hampshire 03052 (US)**
• **SCHNELLINGER, Thomas S**
  **North Andover, Massachusetts 01845 (US)**
• **LAGENFELD, Christopher C**
  **Nashua, New Hampshire 03054 (US)**
• **MURPHY, Colin H**
  **Cambridge, Massachusetts 02138 (US)**
• **KERWIN, John M**
  **Manchester, New Hampshire 03104 (US)**
• **JOHNSON, Matthew J**
  **Dunbarton, New Hampshire 03046 (US)**
• **SLATE, Michael J**
  **Merrimack, New Hampshire 03054 (US)**

(74) Representative: **Haseltine Lake Kempner LLP
Redcliff Quay
120 Redcliff Street
Bristol BS1 6HU (GB)**

(56) References cited:
KR-A- 20050 039 780     US-A- 4 936 828
US-A- 6 159 186     US-A1- 2006 175 414

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**EP 3 400 928 B1**

**Description**

**BACKGROUND**

*Relevant Field*

The present disclosure relates to monitoring, regulating, or controlling fluid flow. More particularly, the present disclosure relates to a system, method, and apparatus for monitoring, regulating, or controlling fluid flow, for example, for use in medical applications such as intravenous infusion therapy, dialysis, transfusion therapy, peritoneal infusion therapy, bolus delivery, enteral nutrition therapy, parenteral nutrition therapy, hemoperfusion therapy, fluid resuscitation therapy, or insulin delivery, among others.

*Description of Related Art*

In many medical settings, one common mode of medical treatment involves delivering fluids into a patient, such as a human, animal, or pet. The need may arise to rapidly infuse fluid into the patient, accurately infuse the fluid into the patient, and/or slowly infuse the fluid into the patient. Saline and lactated ringers are examples of commonly used fluids. Such fluids may be used to maintain or elevate blood pressure and promote adequate perfusion. In the shock-trauma setting or in septic shock, fluid resuscitation is often a first-line therapy to maintain or improve blood pressure.

Delivery of fluid into the patient may be facilitated by use of a gravity-fed line (or tube) inserted into the patient. Typically, a fluid reservoir (e.g., an IV bag) is hung on a pole and is connected to the fluid tube. The fluid tube is sometimes coupled to a drip chamber for trapping air and estimating fluid flow. Below the fluid tube may be a manually actuated valve used to adjust the flow of fluid. For example, by counting the number of drops formed in the drip chamber within a certain amount of time, a caregiver can calculate the rate of fluid that flows through the drip chamber and adjust the valve (if needed) to achieve a desired flow rate.

Certain treatments require that the fluid delivery system strictly adhere to the flow rate set by the caregiver. Typically, such applications use an infusion pump, but such pumps may not be used in all situations or environments.

KR 2005 0039780 describes an optical sensor, a focus lens and a light emitting diode, which are installed on the outer surface of a drip chamber or a tube. There is also described a digital signal processor for converting an image captured by the optical sensor into an electrical signal by digital signal processing. A microprocessor analyses the electric signal to calculate a dropping time interval and a dropping time of a drop of Ringer's solution falling in the drip chamber, and calculates a flow rate of the Ringer's solution flowing in the tube.

US 6,159,186 describes an apparatus for controlling infusion delivery using conventional infusion fluid bags including an air bag which is pressurized by a pneumatic pump. A digital camera or imaging device is able to analyze drops passing through a drop chamber by capturing an image of the drop as it passes through the drop chamber. The output of the imaging device is fed to an image processor which calculates the volume of the captured drop using the number of pixels in the image. An infrared detector detects each time a drops passes through the drop chamber, and the outputs of the both the image processor and the infrared detector are passed to a master controller. The master controller controls the fluid delivery process by counting the number of drops passing through the drop chamber and controlling a flow brake to achieve the required flow rate.

US 4,936,828 describes a liquid drop image sensor for determining the volume of liquid drops comprising a light source and an array of optical sensors which receive light energy from the source. The array is positioned relative to the source to establish a passageway therebetween through which drops can fall. An electronic structure connected to the array sequentially activates the array for discrete episodes during the passage of a drop through the passageway. The electronic structure also generates signals which indicate the proportion of the array affected by the shadow of the drop during each episode. Additionally, the liquid drop image sensor includes structure which accumulates the signals and uses them to calculate the volume of the liquid drop.

**SUMMARY**

The invention is defined in the independent method claim 1, and the independent apparatus claim 17. Preferable embodiments of the invention are defined in the dependent claims. Briefly, and in general terms, the present disclosure relates to a system, method, and apparatus for monitoring, regulating, or controlling fluid flow, for example, for use in medical applications such as intravenous infusion therapy, dialysis, transfusion therapy, peritoneal infusion therapy, bolus delivery, enteral nutrition therapy, parenteral nutrition therapy, hemoperfusion therapy, fluid resuscitation therapy, or insulin delivery, among others. More particularly, the present disclosure relates to a fluid flow meter for monitoring the flow of fluids associated with a patient, a valve for regulating the flow of fluid associated with the patient, and/or a fluid flow meter coupled to a valve (e.g., arranged in a closed-loop, open-loop, or feedback configuration) to monitor, regulate and/or control the use of fluid associated with the patient.

In certain embodiments of the present disclosure, a method includes: capturing an image of a drip chamber using an image sensor; identifying a plurality of pixels of interest within the image; determining a subset of pixels within the plurality of pixels of interest, wherein each pixel of the plurality of pixels is determined to be within the subset of pixels when there is a path to a baseline corresponding to the drip chamber; performing a rotation operation on the subset of pixels; and estimating a volume of the drop within the drip chamber by counting a number of pixels within the rotated subset of pixels. The step of determining the subset of pixels within the plurality of pixels of interest includes determining each of the plurality of pixels of interest is within the subset of pixels if the respective pixel of the plurality of pixels of interest has a contiguous path back to the baseline corresponding to the drip chamber.

The baseline may be a predetermined set of pixels within the image sensor. The plurality of pixels of interests may be identified by comparing the image to a background image.

The method may optionally include one or more of: initializing the background image; updating the background image using the image captured by the image sensor; updating an array of variances using the image captured by the image sensor; and/or updating an array of integers in according with the image captured by the image sensor.

Each integer of the array of integers may correspond to a number of updates of a pixel of the background image. In some specific embodiments, the comparison of the image to the background image only compares pixels within the image to pixels within the background image if a respective integer of the array of integers indicates a respective pixel within the background image has been updated at least a predetermined number of times.

The method may optionally include one or more of: identifying a drop in the image and a predetermined band near an edge of the drop; and initializing the background image by setting each pixel of the background image to the image unless it is within the identified drop or the predetermined band near the edge of the drop.

The method may further include setting a pixel of the background image to a predetermined value if a corresponding pixel of the image is within the identified drop or the predetermined band near the edge of the drop. The corresponding pixel of the image has a location corresponding to the pixel of the background image.

The method may further include determining a baseline corresponding to an opening of the drip chamber.

The method may optionally include one or more of: capturing a first image using the image sensor; identifying the drop within the first image and a predetermined band near an edge of the drop; initializing the background image by setting each pixel to the first image unless it is within the identified drop or the predetermined band near the edge of the drop; setting pixels within the region of the drop or within the predetermined band to a predetermined value; initializing an array of integers; initializing an array of variances; and updating the background image, the array of integers, and the array of variances using the image.

The method may further comprise the act of remotely controlling the flow rate by a monitoring client. The monitoring client may be a tablet.

In certain embodiments of the present disclosure, a flow meter includes a coupler, a support member, an image sensor, and at least one processor. The coupler is adapted to couple to a drip chamber. The support member is operatively coupled to the coupler. The image sensor has a field of view and is operatively coupled to the support member. The image sensor is positioned to view the drip chamber within the field of view.

The at least one processor is operatively coupled to the image sensor to receive image data therefrom, and the at least one processor is configured to: capture an image of a drip chamber using the image sensor; identify a plurality of pixels of interest within the image; determine a subset of pixels within the plurality of pixels of interest, wherein each pixel of the plurality of pixels is determined to be within the subset of pixels when there is a path to a baseline corresponding to the drip chamber; perform a rotation operation on the subset of pixels; and estimate a volume of the drop within the drip chamber by counting a number of pixels within the rotated subset of pixels, wherein the at least one processor is configured to determine the subset of pixels within the plurality of pixels of interest by determining each of the plurality of pixels of interest is within the subset of pixels if the respective pixel of the plurality of pixels of interest has a contiguous path back to the baseline corresponding to the drip chamber.

## BRIEF DESCRIPTION OF THE DRAWINGS

These and other aspects will become more apparent from the following detailed description of the various embodiments of the present disclosure with reference to the drawings wherein:

Fig. 1 shows a block diagram of a system for regulating fluid flow in accordance with an embodiment of the present disclosure;

Fig. 2 shows a flowchart diagram of a method for exposing an image sensor in accordance with an embodiment of the present disclosure;

Fig. 3 shows a timing diagram illustrating an embodiment of the method of Fig. 2 in accordance with an embodiment of the present disclosure;

Figs. 4A-4B show illustrations of image data (i.e., images) captured by a flow meter of a drip chamber to illustrate an embodiment of the method for exposing an image sensor of Fig. 2 in accordance with the timing diagram of Fig. 3 in accordance with an embodiment of the present disclosure;

Fig. 5 shows a diagram of a flow meter and valve that are integrated together for coupling to a drip chamber and an IV bag in accordance with an embodiment of the present disclosure;

Fig. 6 is a block diagram of an imaging system of a flow meter for imaging a drip chamber in accordance with an embodiment of the present disclosure;

Fig. 7 is a graphic illustration of an image captured by the image sensor of the system of Fig. 6 in accordance with an embodiment of the present disclosure;

Fig. 8 is a block diagram of an imaging system of a flow meter for imaging a drip chamber utilizing a background pattern in accordance with an embodiment of the present disclosure;

Fig. 9 is a graphic illustration of an image captured by an image sensor of a flow meter disclosed herein when a free flow condition exists in accordance with an embodiment of the present disclosure;

Fig. 10 is a graphic illustration of an image captured by an image sensor of a flow meter for use as a background image in accordance with an embodiment of the present disclosure;

Fig. 11 is a graphic illustration of an image captured by an image sensor when drops are being formed within a drip chamber in accordance with an embodiment of the present disclosure;

Fig. 12 is a graphic illustration of an image captured by an image sensor for use as a background image in accordance with an embodiment of the present disclosure;

Fig. 13 is a graphic illustration of a difference between the images of Figs. 11 and 12 with additional processing in accordance with an embodiment of the present disclosure;

Fig. 14 is a graphic representation of some of the image processing performed using Figs. 11-13 to determine if a free flow condition exists in accordance with an embodiment of the present disclosure;

Fig. 15 is a graphic illustration of an image captured by the image sensor when a free flow condition exists in accordance with an embodiment of the present disclosure;

Fig. 16 is a graphic illustration of an image captured by the image sensor for use as a background image in accordance with an embodiment of the present disclosure;

Fig. 17 is a graphic illustration of a difference between the images of Figs. 15 and 16 with some additional processing for use in detecting a free flow condition in accordance with an embodiment of the present disclosure;

Fig. 18 is a graphic representation of some of the image processing performed using Figs. 15-17 to determine if a free flow condition exists in accordance with an embodiment of the present disclosure;

Fig. 19 illustrates a template for pattern matching to determine if a free flow condition exits in accordance with an embodiment of the present disclosure;

Fig. 20 is a graphic illustration of a difference between a reference image and an image containing a stream processed with edge detection and line detection for use in detecting a free flow condition in accordance with an embodiment of the present disclosure;

Fig. 21 is a graphic illustration of an image of a drip chamber captured by an image sensor when a free flow condition exists in accordance with an embodiment of the present disclosure;

Fig. 22 is a block diagram of an imaging system for use with a flow meter having a background pattern with stripes and a light source shining on the stripes from an adjacent location to an image sensor in accordance with an embodiment of the present disclosure;

Fig. 23 is a block diagram of an imaging system for use with a flow meter having a background pattern with stripes and a light source shining on the stripes from behind the background pattern relative to an opposite end of an image sensor in accordance with an embodiment of the present disclosure;

Fig. 24 illustrates an image from an image sensor when a drop distorts the background pattern of Fig. 23 in accordance with an embodiment of the present disclosure;

Fig. 25 is a block diagram of an imaging system for use with a flow meter having a background pattern with a checkerboard pattern and a light source shining on the stripes from behind the background pattern relative to an opposite end of an image sensor in accordance with an embodiment of the present disclosure;

Fig. 26 shows an image from the image sensor of Fig. 25 when a drop distorts the background pattern in accordance with an embodiment of the present disclosure;

Figs. 27-28 show a flow chart illustration of a method for estimating a volume of a drop within a drip chamber in accordance with an embodiment of the present disclosure;

Figs. 29-31 illustrate images used or generated by a flow meter to estimate a volume of a drop within a drip chamber using the method illustrated by Figs. 27-28 in accordance with an embodiment of the present disclosure;

Fig. 32 shows pseudo code for identifying a plurality of pixels of interest in accordance with the method of Figs. 27-28 in accordance with an embodiment of the present disclosure;

Figs. 33-36 illustrate additional images used or generated by a flow meter to estimate a volume of a drop within a drip chamber using the method illustrated by Figs. 27-28 in accordance with an embodiment of the present disclosure;

Fig. 37 shows pseudo code for determining a subset of pixels within the plurality of pixels of interest that corresponds to a drop in accordance with an embodiment of the present invention;

Fig. 38 shows a ray diagram illustrating the diameter of a blur circle to illustrate aspects of an image sensor of an imaging system disclosed herein in accordance with an embodiment of the present disclosure;

Fig. 39 is a graphic illustrating a calculated blur circle for a variety of lens-to-focal plane separations and lens-to-image separations for an image sensor of an imaging system disclosed herein in accordance with an embodiment of the present disclosure;

Fig. 40 is a graphic illustrating a blur circle divided by a pixel size when a 20 millimeter focal length lens of an image sensor of an imaging system disclosed herein is used in accordance with an embodiment of the present disclosure;

Fig. 41 is a graphic illustrating a blur circle divided by a pixel size when a 40 millimeter focal length lens of an image sensor of an imaging system disclosed herein is used in accordance with an embodiment of the present disclosure;

Fig. 42 shows a table illustrating the corresponding fields of view about the optical axis for the corners of two configurations of an imaging system disclosed herein in accordance with an embodiment of the present disclosure;

Fig. 43 shows a flow meter coupled to a drip chamber in accordance with an embodiment of the present disclosure;

Fig. 44 shows the flow meter and drip chamber of Fig. 43 with the door open in accordance with an embodiment of the present disclosure;

Fig. 45 shows a flow meter coupled to a drip chamber in accordance with an embodiment of the present disclosure;

Fig. 46 shows a flow meter and a pinch valve coupled to the body of the flow meter to control the flow of fluid into a patient in accordance with an embodiment of the present disclosure;

Fig. 47 shows a close-up view of the pinch valve that is coupled to the body of the flow meter of Fig. 46 in accordance with an embodiment of the present disclosure;

Fig. 48 shows a flow meter and a pinch valve wherein the flow meter includes two image sensors in accordance with another embodiment of the present disclosure;

Fig. 49 shows a flow meter and a valve including two curved, elongated support members to control the flow of fluid into a patient in accordance with an embodiment of the present disclosure;

Figs. 50A-50B show close-up views of the valve of Fig. 49 in accordance with an embodiment of the present disclosure;

Figs. 51A-51D show several views of a flow meter with a monitoring client, a valve, a drip chamber, an IV bag and a fluid tube wherein the flow meter includes a receiving portion to receive the valve in accordance with an embodiment of the present disclosure;

Figs. 52A-52D show several views of another flow meter with a valve, a drip chamber, and a tube wherein the flow meter has a receiving portion to receive the valve in accordance with an embodiment of the present disclosure;

Fig. 53A shows another view of the valve of Figs. 51A-51D and 52A-52D in accordance with an embodiment of the present disclosure;

Figs. 53B-53C show two exploded views of the valve of Fig. 53A in accordance with an embodiment of the present disclosure;

Fig. 54 shows the valve of Fig. 53 in manual use in accordance with an embodiment of the present disclosure;

Fig. 55 shows a valve that includes two flexible members in accordance with an embodiment of the present disclosure;

Figs. 56A-56C show several views of a valve having two curved, elongated support members with one of the elongated support members having a plurality of ridges adapted to engage a tube in accordance with an embodiment of the present disclosure;

Figs. 57A-57C show several views of a valve having a ratchet that engages a connecting member in accordance with an embodiment of the present disclosure;

Figs. 57D-57E show two exploded views of the valve of Figs. 57A-57C in accordance with an embodiment of the present disclosure;

Figs. 58A-58D show several views of a valve having two elongated support members, a connecting member, and a screw-type actuator in accordance with another embodiment of the present disclosure;

Figs. 59A-59C show several views of a body of a valve in accordance with an embodiment of the present disclosure;

Figs. 59D-59G show several views of a knob for use with the body shown in Figs. 59A-59C in accordance with an embodiment of the present disclosure;

Fig. 59H shows the assembled valve that includes the body shown in Figs. 59A-59C coupled to the knob of Figs. 59D-59G in accordance with an embodiment of the present disclosure;

Fig. 60 shows a valve having a guiding protrusion in accordance with an embodiment of the present disclosure;

Fig. 61 shows a motor and a valve-securing structure for coupling to the valve of Fig. 60 in accordance with an embodiment of the present disclosure;

Fig. 62 shows the valve of Fig. 60 secured to the motor and the valve-securing structure of Fig. 61 in accordance with an embodiment of the present disclosure;

Fig. 63 shows another motor and valve-securing structure for coupling to the valve of Fig. 60 in accordance with an embodiment of the present disclosure;

Fig. 64A shows a valve having a collar and several fingers for regulating fluid flow through a fluid line in accordance with an embodiment of the present disclosure;

Fig. 64B shows a cross-sectional view of the valve of Fig. 64A in accordance with an embodiment of the present disclosure;

Fig. 65 shows a cross-sectional view of a valve having two curved surfaces for positioning a fluid tube therebetween to regulate fluid flow through the fluid tube in accordance with an embodiment of the present disclosure;

Figs. 66A-66G show several views of a valve having a knob to move a connecting member which is locked into position after movement of the knob in accordance with an embodiment of the present disclosure;

Fig. 67 shows a graphic that illustrates actuation vs. flow rates for a valve in accordance with an embodiment of the present disclosure;

Fig. 68A shows a flow meter that uses binary optics in accordance with an embodiment of the present disclosure;

Figs. 69A-69F show several views of a safety valve that may be used with a flow meter in accordance with an embodiment of the present disclosure;

Fig. 70 shows a flow chart diagram illustrating a method of estimating drop growth and/or flow within a drip chamber in accordance with an embodiment of the present disclosure;

Figs. 71A-71B show images take by a flow meter with a template overlaid therein to illustrate the method of Fig. 70 in accordance with an embodiment of the present disclosure;

Fig. 72 shows a modulateable backlight assembly in accordance with an embodiment of the present disclosure;

Figs. 73A-73C show several views of a tube-restoring apparatus in accordance with an embodiment of the present disclosure;

Fig. 74 shows a system for regulating fluid flow using a valve having two flexible strips in accordance with an embodiment of the present disclosure;

Fig. 75 shows the valve of Fig. 74 in accordance with an embodiment of the present disclosure;

Fig. 76A shows a valve that utilizes a fluid-based bladder in accordance with an embodiment of the present disclosure;

Fig. 76B shows a cross-sectional view of the assembled valve of Fig. 76A with two elastomeric fillers in accordance with an embodiment of the present disclosure;

Fig. 77 shows a system for regulating fluid flow using a valve having two flexible strips actuateable by a linear actuator in accordance with an embodiment of the present disclosure;

Fig. 78 shows the system of Fig. 77 with the valve actuated in accordance with an embodiment of the present disclosure;

Fig. 79 shows a close-up of the valve of Figs. 77-78 in accordance with an embodiment of the present disclosure;

Fig. 80 shows a close-up of the valve as actuated in Fig. 78 in accordance with an embodiment of the present disclosure;

Fig. 81 shows several images for use to illustrate a method of estimating drop growth and/or fluid flow illustrated in Figs. 82A-82B in accordance with an embodiment of the present disclosure; and

Figs. 82A-82B show a flow chart diagram illustrating a method of estimating drop growth and/or fluid flow in accordance with an embodiment of the present disclosure; and

Fig. 83 shows a flow chart diagram of a method for reducing noise from condensation in accordance with an embodiment of the present disclosure.

## DETAILED DESCRIPTION

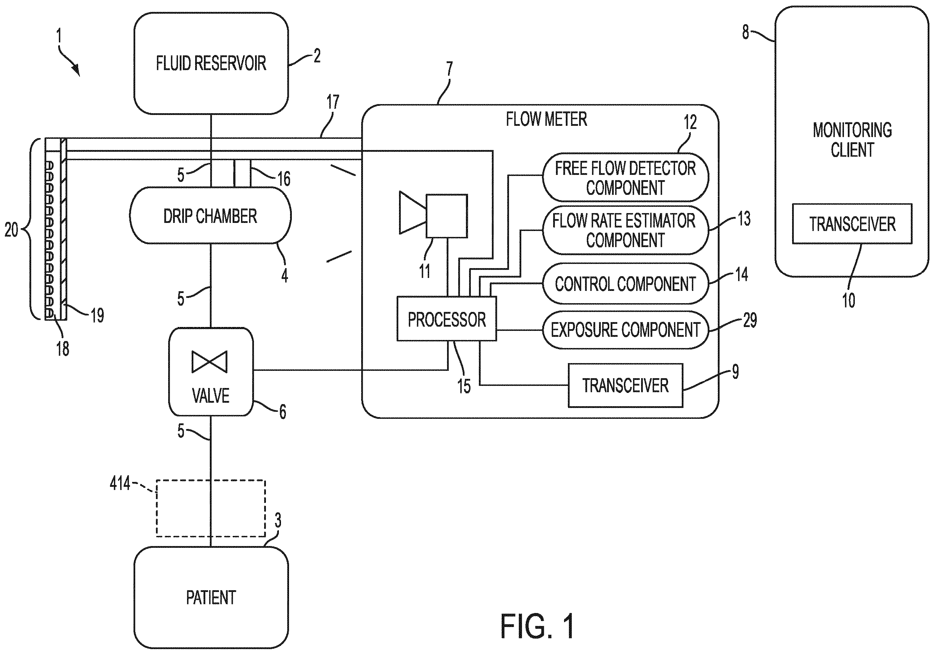

Fig. 1 shows a block diagram of a system 1 for regulating fluid flow in accordance with an embodiment of the present disclosure. For example, system 1 may regulate, monitor, and/or control the flow of fluid into a patient 3. The system 1 includes a fluid reservoir 2 for infusing fluid contained therein into the patient 3. The fluid reservoir 2 is gravity fed into a drip chamber 4 via a fluid tube 5. The fluid reservoir 2, the drip chamber 4, and the patient 3 may be considered as part of the system 1, may be considered as separate or optional work pieces for the system 1 (e.g., any fluid reservoir 2 and drip chamber 4 may be used to treat any patient 3).

A flow meter 7 monitors the drip chamber 4 to estimate a flow rate of liquid flowing through the drip chamber 4. The fluid from the drip chamber 4 is gravity-fed into a valve 6. The valve 6 regulates (i.e., varies) the flow of fluid from the fluid reservoir 2 to the patient 3 by regulating fluid flow from the drip chamber 4 to the patient 3. The valve 6 may be any valve as described herein, including a valve having two curved-shaped members, a valve having two flexible sheets, a valve that pinches (or uniformly compresses) on the tube over a significant length of the tube, or the like. The valve 6 may be an inverse-Bourdon-tube valve that works in an opposite way of a Bourdon tube in that a deformation of the fluid path causes changes in fluid flow rather than fluid flow causing deformation of the fluid path.

In alternative embodiments, the system 1 optionally includes an infusion pump 414 (e.g., a peristaltic pump, a finger pump, a linear peristaltic pump, a rotary peristaltic pump, a cassette-based pump, membrane pump, other pump, etc.) coupled to the fluid tube 5. The outlined box designated as 414 represents the optional nature of the infusion pump 414, e.g., the infusion pump may not be used in some embodiments. The infusion pump 414 may use the flow meter 7 as feedback to control the flow of fluid through the fluid tube 5. The infusion pump 414 may be in wireless communication with the flow meter 7 to receive the flow rate therefrom. The infusion pump 414 may use a feedback control algorithm (e.g., the control component 14 of Fig. 1) to adjust the flow of fluid, such as a proportional-integral-derivative ("PID"), bang-bang, neural network, and/or fuzzy logic control system. In this specific exemplary embodiment (i.e., an embodiment having the infusion pump 414), the valve 6 is optional. However, in other embodiments, the valve 6 may or may not be used, and/or is optional. The infusion pump 414 may adjust the rotation of a cam and/or a motor in accordance with measurements from the flow meter 7, such as flow rate, volume infused, total volume infused, etc. Additionally or alternatively, the infusion pump 414 may stop fluid flow (e.g., by stopping the pumping action) when the flow meter 7 communicates to the infusion pump 414 that a free flow condition exists. In yet additional embodiments, the monitoring client 8 controls the operation of the infusion pump 414 (e.g., via a wireless connection) and receives feedback from the flow meter 7.

In some embodiments, the fluid reservoir 2 is pressurized to facilitate the flow of fluid from the fluid reservoir 2 into the patient 3, e.g., in the case where the fluid reservoir 2 (e.g., an IV bag) is below the patient 3; The pressurization provides sufficient mechanical energy to cause the fluid to flow into the patient 3. A variety of pressure sources, such as physical pressure, mechanical pressure, and pneumatic pressure may be applied to the inside or outside of the fluid reservoir 2. In one such embodiment, the pressurization may be provided by a rubber band wrapped around an IV bag.

The flow meter 7 and the valve 6 may form a closed-loop system to regulate fluid flow to the patient 3. For example, the flow meter 7 may receive a target flow rate from a monitoring client 8 by communication using transceivers 9, 10. That is, the transceivers 9, 10 may be used for communication between the flow meter 7 and the monitoring client 8. The transceivers 9, 10 may communicate between each other using a modulated signal to encode various types of information such as digital data or an analog signal. Some modulation techniques used may include using carrier frequency with FM modulation, using AM modulation, using digital modulation, using analog modulation, or the like.

The flow meter 7 estimates the flow rate through the drip chamber 4 and adjusts the valve 6 to achieve the target flow rate received from the monitoring client 8. The valve 6 may be controlled by the flow meter 7 directly from communication lines coupled to an actuator of the valve 6 or via a wireless link from the flow meter 7 to onboard circuitry of the valve 6. The onboard electronics of the valve 6 may be used to control actuation of the valve 6 via an actuator coupled thereto. This closed-loop embodiment of the flow meter 7 and the valve 6 may utilize any control algorithm including a PID control algorithm, a neural network control algorithm, a fuzzy-logic control algorithm, the like, or some combination thereof.

The flow meter 7 is coupled to a support member 17 that is coupled to the drip chamber 4 via a coupler 16. The support member 17 also supports a backlight 18. The backlight 18 includes an array of LEDs 20 that provides illumination to the flow meter 7. In some specific embodiments, the backlight 18 includes a background pattern 19. In other embodiments, the backlight 18 does not include the background pattern 19. In some embodiments, the background pattern 19 is present in only the lower portion of the backlight 18 and there is no background pattern 19 on the top (e.g., away from the ground) of the backlight 18.

The flow meter 7 includes an image sensor 11, a free flow detector component 12, a flow rate estimator component 13, a control component 14, an exposure component 29, a processor 15, and a transceiver 9. The flow meter 7 may be battery operated, may be powered by an AC outlet, may include supercapacitors, and may include on-board, power-supply circuitry (not explicitly shown).

The image sensor 11 may be a CCD sensor, a CMOS sensor, or other image sensor. The image sensor 11 captures images of the drip chamber 4 and communicates image data corresponding to the captured images to the processor 15.

The processor 15 is also coupled to the free flow detector component 12, the flow rate estimator component 13, the control component 14, and the exposure component 29. The free flow detector component 12, the flow rate estimator component 13, the control component 14, and the exposure component 29 may be implemented as processor executable instructions that are executable by the processor 15 and may be stored in memory, such as a non-transitory processor-readable memory, ROM, RAM, EEPROM, a harddisk, a harddrive, a flashdrive, and the like.

The processor 15 can execute the instructions of the free flow detector component 12 to determine if a free flow condition exists within the drip chamber 4 by analyzing the image data from the image sensor 11. Various embodiments of the free flow detector component 12 for detecting a free flow condition are described below. In response to a detected free flow condition, the processor 15 can make a function call to the control component 14 to send a signal to the valve 6 to completely stop fluid flow to the patient 3. That is, if the free flow detector component 12 determines that a free flow condition exists, the flow meter 7 may instruct the valve 6 to stop fluid flow, may instruct the monitoring client 8 to stop fluid flow (which may communicate with the valve 6 or the pump 414), and/or may instruct the pump 414 to stop pumping or occlude fluid flow using an internal safety occluder.

The flow rate estimator component 13 estimates the flow rate of fluid flowing through the drip chamber 4 using the image data from the image sensor 11. The processor 15 communicates the estimated flow rate to the control component 14 (e.g., via a function call). Various embodiments of estimating the flow rate are described below. If the flow rate estimator component 13 determines that the flow rate is greater than a predetermined threshold or is outside a predetermined range, the flow meter 7 may instruct the valve 6 to stop fluid flow (which may communicate with the valve 6 or the pump 414), may instruct the monitoring client 8 to stop fluid flow (which may communicate with the valve 6 or the pump 414), and/or may instruct the pump 414 to stop pumping or occlude fluid flow using an internal safety occluder.

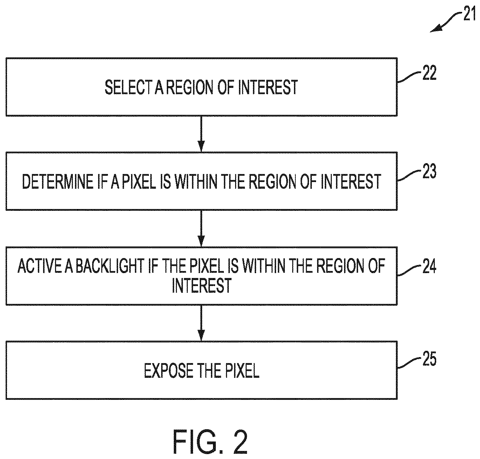

The processor 15 controls the array of LEDs 20 to provide sufficient light for the image sensor 11. For example, the exposure component 29 may be used by the processor 15 or in conjunction therewith to control the array of LEDs 20 such that the image sensor 11 captures image data sufficient for use by the free flow detector component 12 and the flow rate estimator component 13. The processor 15 may implement an exposure algorithm stored by the exposure component 29 (see Fig. 2) to control the lighting conditions and/or the exposure of the image sensor 11 when generating the image data. Additionally or alternatively, the exposure component 29 may be implemented as a circuit, an integrated circuit, a CPLD, a PAL, a PLD, a hardware-description-language-based implementation, and/or a software system.

The control component 14 calculates adjustments to make to the valve 6 in accordance with the estimated flow rate from the flow rate estimator component 13. For example and as previously mentioned, the control component 14 may implement a PID control algorithm to adjust the valve 6 to achieve the target flow rate.

The monitoring client 8, in some embodiments, monitors operation of the system 1. For example, when a free flow condition is detected by the free flow detector component 12, the monitoring client 8 may wirelessly communicate a signal to the valve 6 to interrupt fluid flow to the patient 3.

The flow meter 7 may additionally include various input/output devices to facilitate patient safety, such as various scanners, and may utilize the transceiver 9 to communicate with electronic medical records, drug error reduction systems, and/or facility services, such as inventory control systems.

In a specific exemplary embodiment, the flow meter 7 has a scanner, such as an RFID interrogator that interrogates an RFID tag attached to the fluid reservoir 2 or a barcode scanner that scans a barcode of the fluid reservoir 2. The scanner may be used to determine whether the correct fluid is within the fluid reservoir 2, it is the correct fluid reservoir 2, the treatment programmed into the flow meter 7 corresponds to the fluid within the fluid reservoir 2 and/or the fluid reservoir 2 and flow meter 7 are correct for the particular patient (e.g., as determined from a patient's barcode, a patient's RFID tag, or other patient identification).

For example, the flow meter 7 may scan the RFID tag of the fluid reservoir 2 to determine if a serial number or fluid type encoded within the RFID tag is the same as indicated by the programmed treatment stored within the flow meter 7. Additionally or alternatively, the flow meter 7 may interrogate the RFID tag of the fluid reservoir 2 for a serial number and the RFID tag of the patient 3 for a patient serial number, and also interrogate the electronic medical records using the transceiver 9 to determine if the serial number of the fluid reservoir 2 within the RFID tag attached to the fluid reservoir 2 matches the patient's serial number within the RFID tag attached to the patient 3 as indicated by the electronic medical records.

Additionally or alternatively, the monitoring client 8 may scan the RFID tag of the fluid reservoir 2 and the RFID tag of the patient 3 to determine that it is the correct fluid within the fluid reservoir 2, it is the correct fluid reservoir 2, the treatment programmed into the flow meter 7 corresponds to the fluid within the fluid reservoir 2, and/or the fluid reservoir 2 is correct for the particular patient (e.g., as determined from a patient's barcode, RFID tag, electronic medical records, or other patient identification or information). Additionally or alternatively, the monitoring client 8 or the flow meter 7 may interrogate the electronic medical records database and/or the pharmacy to verify the prescription or to download the prescription, e.g., using the serial number of the barcode on the fluid reservoir 2 or the RFID tag attached to the fluid reservoir 2.

Fig. 2 shows a flow chart diagram of a method 21 for exposing an image sensor, e.g., the image sensor 11 of Fig. 1, in accordance with an embodiment of the present disclosure. The method 21 includes acts 22, 23, 24, and 25. Method 21 may be implemented by the processor 15 of Fig. 1 (e.g., as the exposure component 29) and may be implemented as a processor-implemented method, as a set of instructions configured for execution by one or more processors, in hardware, in software, the like, or some combination thereof.

Act 22 selects a region of interest. For example, referring again to Fig. 1, the image sensor 11 includes a field of view that includes the drip chamber 4. However, the drip chamber 4 may not occupy the entire field of view of the image sensor 11. Act 22 selects only the pixels of the image sensor 11 that show, for example, the drip chamber 4.

Act 23 determines if a pixel is within the region of interest 23. If the pixel of act 23 is a pixel that images, for example, the drip chamber 4, then act 23 determines that it is within the region of interest. Likewise, in this example, if the pixel of act 23 is a pixel that does not image the drip chamber 4, act 23 determines that the pixel is not within the region of interest.

Act 24 activates a backlight, e.g., backlight 18 of Fig. 1, if the pixel is within the region of interest. Pixels of an image sensor may be exposed during different times. Thus, the backlight 18 may be activated only when pixels within the region of interest are being exposed. For example, some image sensors include vertical and horizontal sync signals. The backlight may be synchronized with these signals to turn on when a pixel of interests is being exposed.

In some embodiments of the present disclosure, a subset of LEDs of the backlight (e.g., a subset of the LED array 18, which may a 2-dimensional array) may be turned on. The subset may be a sufficient subset to sufficiently illuminate the pixel being exposed if the pixel is within the region of interest.

Act 25 exposes the pixel. If in act 23 it was determined that the pixel is within the region of interest, the pixel will be exposed with at least a portion of the backlight turned on in act 25. Additionally, if in act 23 it was determined that the pixel is not within the region of interest, the pixel will be exposed without at least a portion of the backlight turned on in act 25.

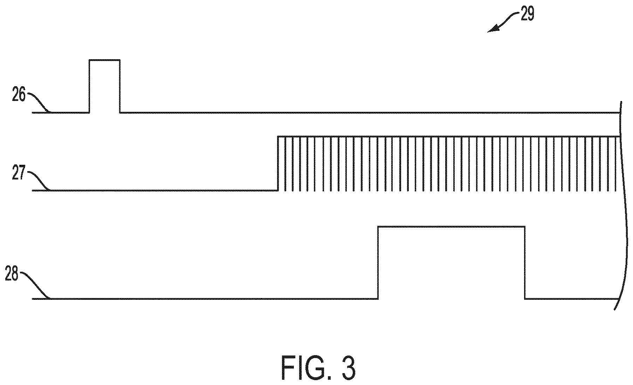

Fig. 3 shows a timing diagram 29 illustrating an embodiment of the method of Fig. 2 in accordance with an embodiment of the present disclosure. The timing diagram 29 includes traces 26, 27, and 28. Trace 26 is a vertical sync signal from an image sensor and trace 27 is a horizontal sync signal from the image sensor (e.g., image sensor 11 of Fig. 1). A circuit or software routine (e.g., the exposure component 29 found in the flow meter 7 of Fig. 1) may use the sync traces 26, 27 to generate a backlight-enable signal 28 that is used to activate a backlight or a subset thereof.

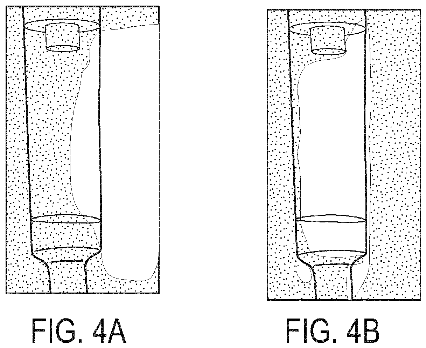

Figs. 4A-4B show illustrations of image data of a flow meter 7 illustrating an embodiment of the method of Fig. 2 in accordance with the timing diagram of Fig. 3 in accordance with an embodiment of the present disclosure. Fig. 4A illustrates the image data taken by a flow meter, such as the flow meter 7 of Fig. 1, without the use of the exposure algorithm illustrated in Figs. 2 and 3; Fig. 4B illustrates the image data taken by the flow meter with the use of the exposure algorithm illustrated in Figs. 2 and 3. Less power is needed to provide illumination during the capture of the image of Fig. 4B than to provide illumination for the capture of the image of Fig. 4A because of less use of the backlight.

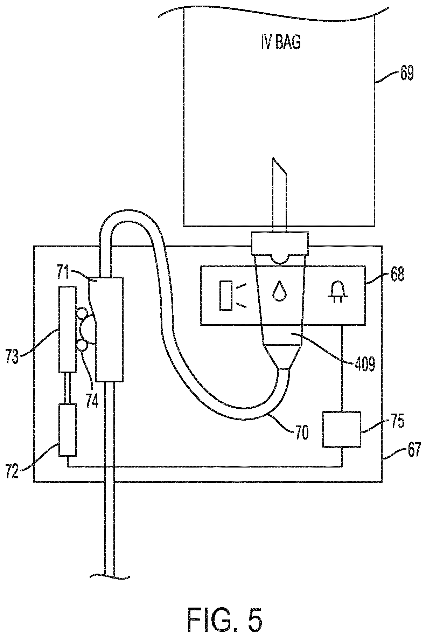

Fig. 5 shows a diagram of a flow meter 67 and a valve 71 that are integrated together for coupling to a drip chamber 409 and an IV bag 69 in accordance with an embodiment of the present disclosure. The flow meter 67 includes an optical drip counter 68 that receives fluid from the IV bag 69. The optical drip counter 68 may be an image sensor, a pair of image sensors, a capacitive drip counter, and/or the like. The flow meter 67 is coupled to a tube 70 coupled to a roller clamp 71 that is controlled by a motor 72. The motor 72 is coupled to a lead screw mechanism 73 to control a roller clamp 71 via interaction with interacting members 74.

The motor 72 may be a servo motor and may be used to adjust the flow rate through the tube 70. That is, the flow meter 67 may also function as a flow meter and regulator. For example, a processor 75 within the flow meter 67 may adjust the motor 72 such that a desired flow rate is achieved as measured by the optical drip counter 68. The processor 75 may implement a control algorithm using the optical drip counter 68 as feedback, e.g., a PID control loop with the output supplied to the motor 72 and the feedback received from the optical drip counter 68.

In alternative embodiments, the motor 72, the lead screw mechanism 73, and the roller clamp 71 may be replaced and/or supplemented by an actuator that squeezes the tube 70 (e.g., using a cam mechanism or linkage driven by a motor) or they may be replaced by any sufficient roller, screw, or slider driven by a motor. For example, in some embodiments of the present disclosure, the roller clamp 71 may be replaced by any valve as described herein, including a valve having two C-shaped members, a valve having two curve-shaped support members, a valve having two flexible sheets, a valve that pinches on the tube over a significant length of the tube, or the like.

The flow meter 67 may also optionally include a display. The display may be used to set the target flow rate, display the current flow rate, and/or provide a button, e.g., a touch screen button to stop the flow rate.

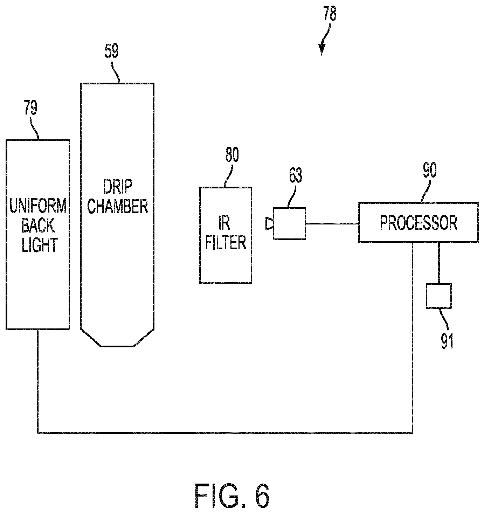

Fig. 6 is a block diagram of an imaging system 78 of a flow meter for imaging a drip chamber in accordance with an embodiment of the present disclosure. The imaging system 78 as shown in Fig. 6 may be used within any flow meter described herein, including the flow meter 7 of Fig. 1 and/or the flow meter 67 of Fig. 5.

The imaging system 78 of Fig. 6 includes an image sensor 63, a uniform backlight 79 to shine light at least partially through the drip chamber 59, and an infrared ("IR") filter 80 that receives the light from the uniform backlight 79.

System 78 also includes a processor 90 that may be operatively coupled to the image sensor 63 and/or the uniform backlight 79. The processor 90 implements an algorithm to determine when a free flow condition exists and/or to estimate a flow rate (e.g., using the free flow detector component 12 or the flow rate estimator component 13 of Fig. 1). The processor 90 may be in operative communication with a processor-readable memory 91 (e.g., a non-transitory processor-readable memory) to receive one or more instructions to implement the algorithm to determine if a free flow condition exists and/or to estimate the flow rate. The one or more instructions from the processor-readable memory 91 are configured for execution by the processor 90.

The uniform backlight 79 may be an array of light-emitting diodes ("LEDs") having the same or different colors, a light bulb, a window to receive ambient light, an incandescent light, and the like. In some embodiments, the uniform backlight 79 may include one or more point-source lights.

The processor 90 may modulate the uniform backlight 79 in accordance with the image sensor 63. For example, the processor 90 may activate the uniform backlight 79 for a predetermined amount of time and signal the image sensor 63 to capture at least one image, and thereafter signal the uniform backlight 79 to turn off. The one or more images from the image sensor 63 may be processed by the processor 90 to estimate the flow rate and/or detect free flow conditions. For example, in one embodiment of the present disclosure, the system 78 monitors the size of the drops being formed within the drip chamber 59, and counts the number of drops that flow through the drip chamber 59 within a predetermined amount of time; the processor 90 may average the periodic flow from the individual drops over a period of time to estimate the flow rate. For example, if X drops each having a volume Y flow through the drip chamber in a time Z, the flow rate may be calculated as (X*Y)/Z.

Additionally or alternatively, the system 78 may determine when the IV fluid is streaming through the drip chamber 59 (i.e., during a free flow condition). The uniform backlight 79 shines light through the drip chamber 59 to provide sufficient illumination for the image sensor 63 to image the drip chamber 59. The image sensor 63 can capture one or more images of the drip chamber 59.

Other orientations and configurations of the system 78 may be used to account for the orientation and output characteristics of the uniform backlight 79, the sensitivity and orientation of the image sensor 63, and the ambient light conditions. In some embodiments of the present disclosure, the processor 90 implements an algorithm that utilizes a uniformity of the images collected by the image sensor 63. The uniformity may be facilitated by the uniform backlight 79. For example, consistent uniform images may be captured by the image sensor 63 when a uniform backlight 79 is utilized.

Ambient lighting may cause inconsistencies in the images received from the image sensor 63; for example, direct solar illumination provides inconsistent lighting because the sun may be intermittently obscured by clouds and the sun's brightness and angle of illumination depend upon the time of the day. Therefore, in some embodiments of the present disclosure, an IR filter 80 is optionally used to filter out some of the ambient light to mitigate variations in the images captured by the image sensor 63. The IR filter 80 may be a narrow-band infrared light filter placed in front of the image sensor 63; and the uniform backlight 79 may emit light that is about the same wavelength as the center frequency of the passband of the filter 80. The IR filter 80 and the uniform backlight 79 may have a center frequency of about 850 nanometers. In some embodiments, the imaging system 78 may be surrounded by a visually translucent but IR-blocking shell. In alternative embodiments, other optical frequencies, bandwidths, center frequencies, or filter types may be utilized in the system 78.

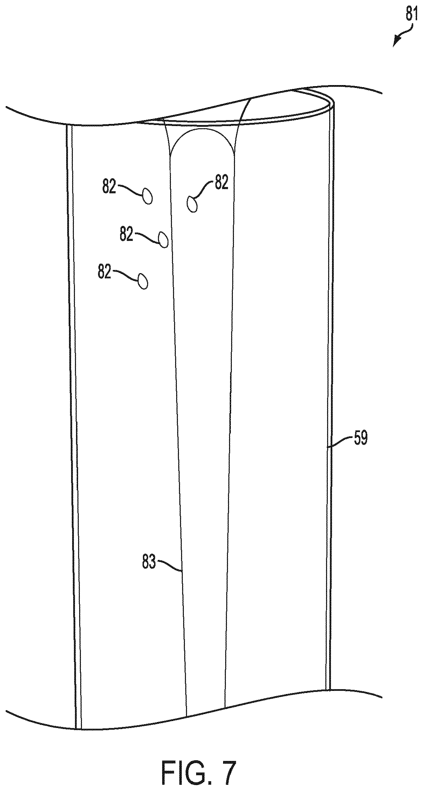

Fig. 7 is a graphic illustration of an image 81 captured by the image sensor 63 of the system 78 of Fig. 6 in accordance with an embodiment of the present disclosure. The image 81 is an image of a drip chamber 59 having condensation 82 and a stream 83 caused by a free flow condition therein. Edge detection may be used to determine the position of the stream 83 and/or the condensation 82, in some embodiments. Additionally or alternatively, a background image or pattern may be used.

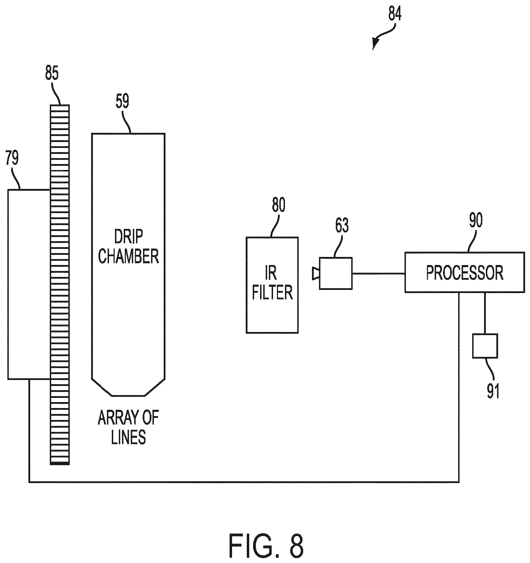

Fig. 8 is a block diagram of an imaging system 84 of a flow meter for imaging a drip chamber in accordance with an embodiment of the present disclosure. The imaging system 84 may be used with any flow meter disclosed herein, including the flow meter 7 of Fig. 1 and the flow meter 67 of Fig. 5.

System 84 includes an array of lines 85 that are opaque behind the drip chamber 59. System 84 uses the array of lines 85 to detect a free flow condition. The free flow detection algorithm (e.g., the free flow detector component 12 of Fig. 1) may use the presence or absence of drops for determining whether or not a streaming condition (e.g., a free flow condition) exists.

In some specific embodiments, the lines 85 are only present on a fraction of the image (e.g., the background pattern only occupies a fraction of the backlight 18 or the binary optics only causes the pattern to appear in a fraction of the image, such as the lower or upper half). For example, a lower fraction of the image may include a background pattern of stripes.

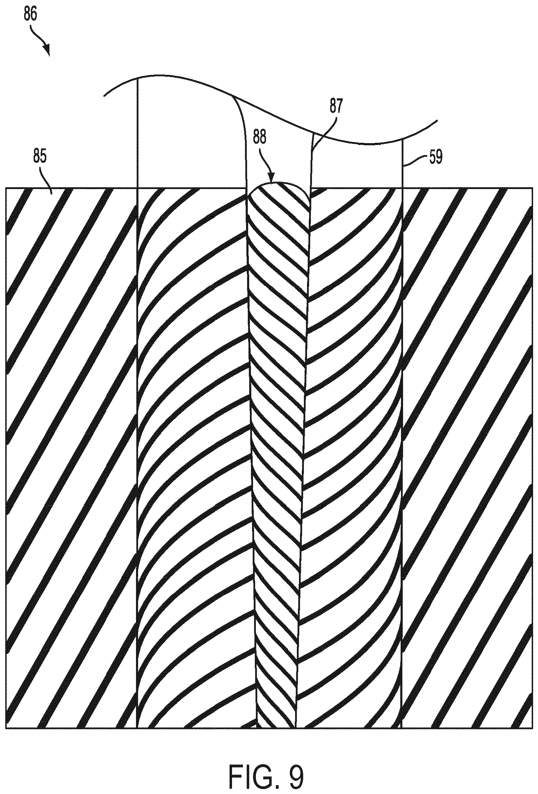

Referring now to Fig. 9, a graphic illustration of an image 86 is shown as captured by the image sensor 63 of Fig. 8 when a free flow condition exists in the drip chamber 59 in accordance with an embodiment of the present disclosure. The image 86 illustrates the condition in which the drip chamber 59 experiences a free flow condition and illustrates the effect that the stream of fluid 87 acts as a positive cylindrical lens. That is, as shown in Fig. 9, the array of lines 85 as captured in an image by the image sensor 63 are shown as a reversed line pattern 88 from the array of lines 85 as compared to a non-free flow condition. The appearance of the reversed line pattern 88 is caused by changes to the light when the light passes through the stream of fluid 87 as the light approaches the image sensor 63.

In some embodiments of the present disclosure, illumination by light having an optical wavelength of about 850 nanometers may be used to create the image 86. Some materials may be opaque in the visible spectrum and transparent in the near IR spectrum at about 850 nanometers and therefore may be used to create the array of lines 85. The array of lines 85 may be created using various rapid-prototyping plastics. For example, the array of lines 85 may be created using a rapid-prototype structure printed with an infrared-opaque ink or coated with a metal for making the array of lines 85. Additionally or alternatively, in some embodiments of the present disclosure, another method of creating the array of lines 85 is to create a circuit board with the lines laid down in copper. In another embodiment, the array of lines 85 is created by laying a piece of ribbon cable on the uniform backlight 79; the wires in the ribbon cable are opaque to the infrared spectrum, but the insulation is transparent such that the spacing of the wires may form the line for use during imaging by the image sensor 63 (see Fig. 8). In yet additional embodiments, a piece of thin EDMed metal may be utilized. Metal is opaque to light and the spaces between the metal material deposits may be very finely controlled during manufacture to allow the IR light to pass through the spaces.

The processor 90 implements an algorithm to determine when a free flow condition exists (e.g., using the free flow detector component 12 of Fig. 1). The processor 90 may be in operative communication with a processor-readable memory 91 (e.g., a non-transitory processor-readable memory) to receive one or more instructions to implement the algorithm to determine if a free flow condition exists. The one or more instructions from the processor-readable memory 91 are configured for execution by the processor 90.

Referring again to Fig. 8, blood may be used by the system 84. For example, system 84 may determine when a free flow condition of blood exists when utilizing the image sensor 63, the IR filter 80, and the uniform backlight 79 configured, for example, for use using optical light having a wavelength of 850 nanometers or 780 nanometers, e.g., when using bovine blood. The blood may appear opaque compared to the images taken using water.

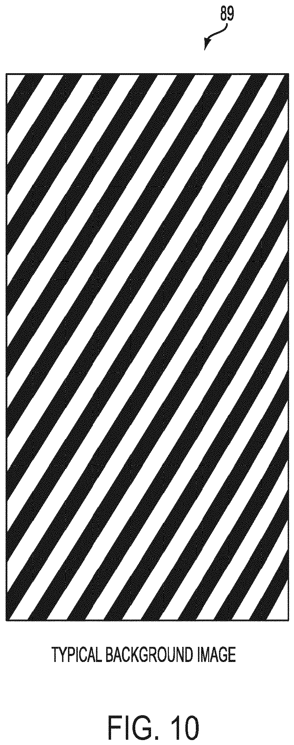

The following algorithm implemented by the processor 90 and received from the processor-readable memory 91 may be used to determine when a free flow condition exists: (1) establish a background image 89 (see Fig. 10); and (2) subtract the background image 89 from the current image. Additionally processing may be performed on the resulting image.

In some embodiments of the present disclosure, the background image 89 of Fig. 10 may be dynamically generated by the processor 90. The dynamic background image may be used to account for changing conditions, e.g. condensation or splashes 82 on the surface of the drip chamber 59 (see Fig. 7). For example, in one specific embodiment, for each new image captured by the image sensor (e.g., 63 of Fig. 8), the background image has each pixel multiplied by .96 and the current image (e.g., the most recently captured image) has a respective pixel multiplied by .04, after which the two values are added together to create a new value for a new background image for that respective pixel; this process may be repeated for all of the pixels. In yet another example, in one specific embodiment, if a pixel of the new image is at a row, x, and at a column, y, the new background image at row, x, and column, y, is the value of the previous background image at row, x, and column, y, multiplied by .96, which is added to the value of the pixel at row, x, and column, y of the new image multiplied by .04.

When the system 84 has no water flowing through the drip chamber 59 (see Fig. 8) the resulting subtraction should be almost completely back, i.e., low pixel magnitudes, thereby facilitating the algorithm to determine that the drip chamber 59 has no water flowing therethrough.

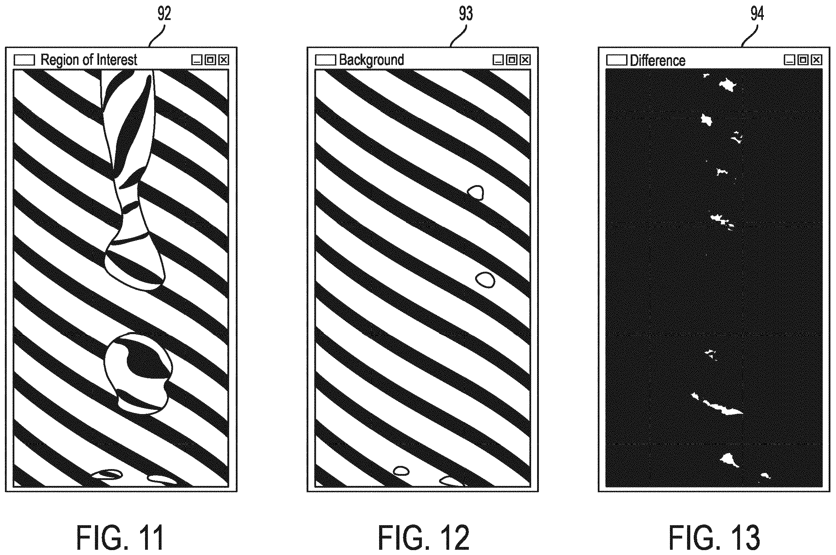

Fig. 11 shows an image 92 from the image sensor 63 when there is a drop within the drip chamber 59 (see Fig. 8). Fig. 12 shows a background image 93 used by the system 84. When the system 83 has a drop as shown in image 92 of Fig. 11, the system 84 of Fig. 8 has a few high contrast-spots where the image of the array of lines is warped by the lensing of the droplet as illustrated by an image 94 of Fig. 13. Image 94 of Fig. 13 is generated by taking, for each respective pixel, the absolute value of the subtraction of the image 92 of Fig. 11 from image 93 of Fig. 12, and converting each respective pixel to a white pixel if the value is above a predetermined threshold or otherwise converts the pixel to a black pixel when the value is below the predetermined threshold. Each white pixel within the image 94 of Fig. 13 is a result of there being a difference for that pixel location between the images 92 and 93 that is greater than a predetermined threshold.

For example, consider three respective pixels of Figs. 11, 12, and 13 having a location of row x and column y. To determine the pixel of row x and column y for the image 94 of Fig. 13, the pixel at row x and column y of image 92 of Fig. 11 is subtracted from the pixel at row x and column y of image 92 of Fig. 12, then the absolute value of the result of the subtraction is taken; and if the absolute value of the result is above a predetermined threshold (e.g., above a grayscale value of 128, for example), the pixel at the location of row x and column y of image 94 of Fig. 13 is white, otherwise the pixel at the location of row x and column y of image 94 of Fig. 13 is black.

When it is determined that a few high-contrast spots exist within the image 94 of Fig. 13, the processor 90 of system 84 (see Fig. 8) determines that drops are being formed within the drip chamber 59 and no free flow condition exists. The images of the drops may be utilized to determine the size of the drops to estimate a flow rate as described herein.

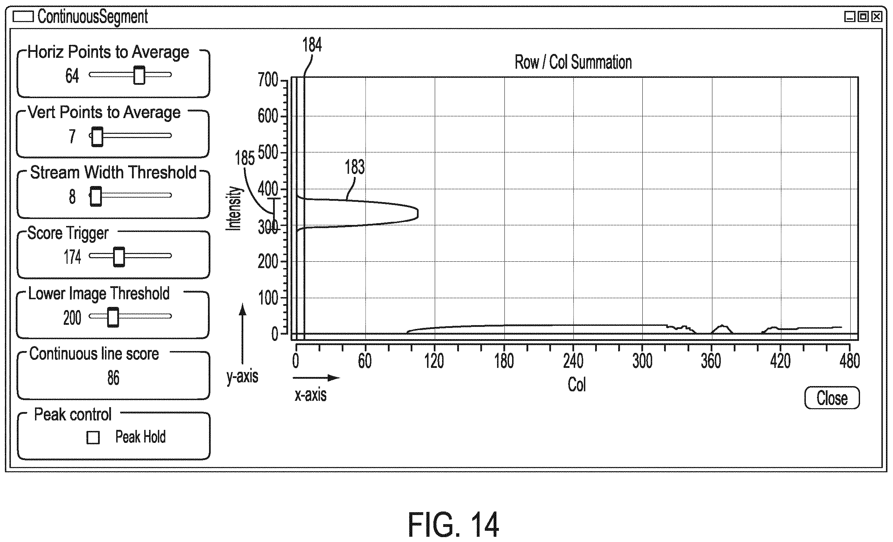

Fig. 14 is a graphic representation of some of the image processing that may be performed using Figs. 11-13 to determine if a free flow condition exists in accordance with an embodiment of the present disclosure. Referring to Figs. 14 and 13, all of the white pixels for each row are summed together, and are illustrated in Fig. 14 as results 183. The y-axis represents the row number, and the x-axis represents the summed number of white pixels for each respective row.

Referring now to only Fig. 14, as previously mentioned, the number of white pixels for each row is summed together and is illustrated as results 183, which are used to determine if or when a free flow condition exists. In some specific embodiments, the processor 90 of system 84 (see Fig. 8) determines that a free flow condition exists when a predetermined number of contiguous values of the summed rows of the results 183 exist above a threshold 184. For example, within the results 183, a range of a plurality of contiguous rows represented generally by 185 have a total value above the threshold 184. When greater than a predetermined number of contiguous summed rows are determined to exist within the results 183 above a predetermined threshold (e.g., threshold 184), a free flow condition is determined to exist by the processor 90 of Fig. 8. For example, as shown in Fig. 14, the range of the plurality of contiguous summed rows 185 are below the predetermined number of contiguous summed rows (i.e., the range 185 is not wide enough) and therefore a free flow condition is determined to not exist.

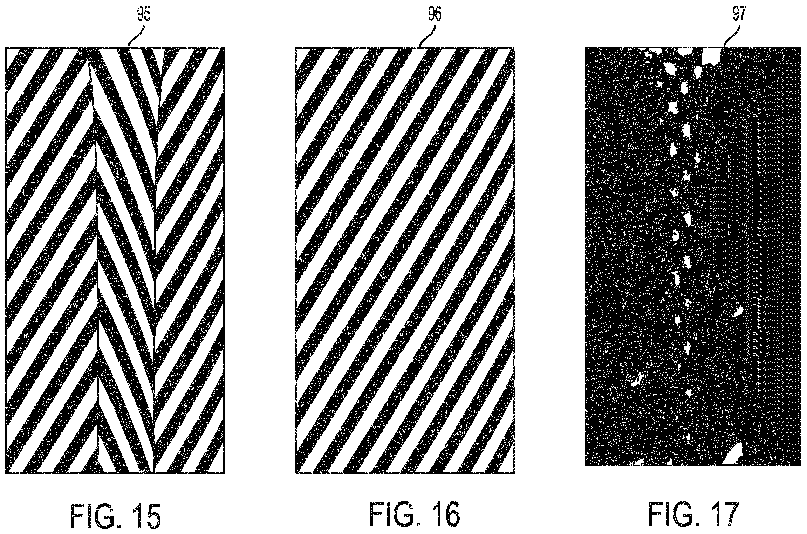

Fig. 15 shows an image 95 showing a stream as captured by the image sensor 63 of Fig 8 when a free flow condition exists. Fig. 16 shows a background image 96. Fig. 17 shows an image 97 formed by the absolute value of the difference between the image 96 of Fig. 16 and the image 95 from Fig. 15 when the absolute value is converted either to a white pixel (when the absolute value of the difference is above a threshold) or to a black pixel (when the absolute value of the difference is below the threshold). As shown in Fig. 17, high-contrast spots caused by the reverse orientation of the lines in the stream that run from top to bottom are detectable by the processor 90. The processor 90 of Fig. 8 can use the image 97 to determine if a free flow condition exists using the algorithm described above.

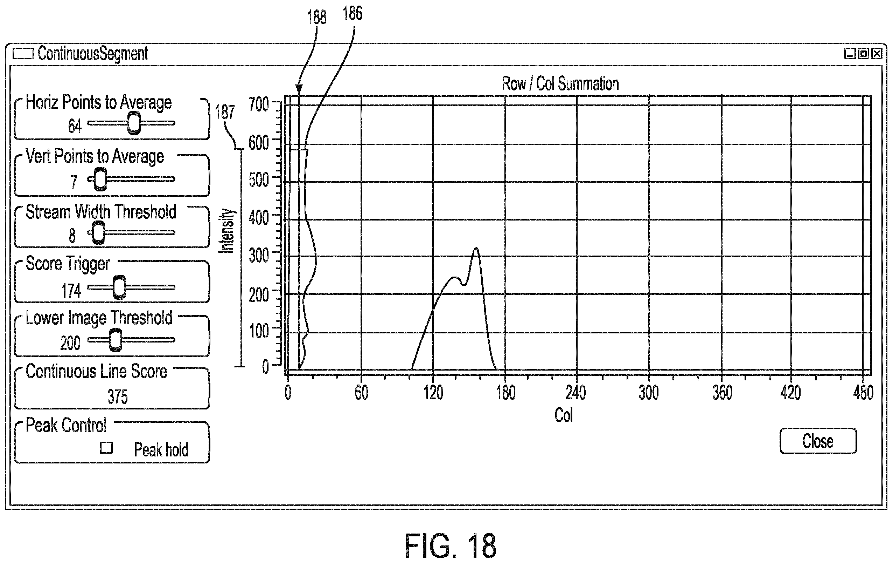

That is, as shown in Fig. 18, results 186 are shown as having a contiguous range 187 of the results 186 that are above a threshold 188. Because the contiguous range 187 of summed rows is greater than a predetermined threshold number of contiguous values above the threshold 188, a free flow condition is determined to exist by the processor 90 (see Fig. 8). That is, the contiguous range of the results 186 above the threshold 188 is greater than a predetermined threshold range of contiguous values; therefore, the processor 90 determines that a free flow condition exists when using the results 186 of Fig. 18.

In yet an additional embodiment of the present disclosure, the intensity, the intensity squared, or other function may be used to produce the results 183 of Fig. 14 and/or results 186 of Fig. 18. In yet an additional embodiment, one or more data smoothing functions may be used to smooth the results 183 and/or 186, such as a spline function, a cubic spline function, a B-spline function, a Bezier spline function, a polynomial interpolation, a moving average, or other data smoothing function.

For example, an image of the image sensor 63 of Fig. 8, e.g., image 95 of Fig. 15, may be subtracted from a background image, e.g., the image 96 of Fig. 16, to obtain intensity values. That is, a pixel of row x and column y of Fig. 15 may be subtracted from a pixel of row x and column y of the image 96 of Fig. 16 to create an intensity value at row x and column y; this may be repeated for all pixel locations to obtain all of the intensity values. The intensity values of each row may be summed together to obtain the results 183 and/or 186 (see Figs. 14 and 18, respectively), such that the processor 90 may determine that a free flow condition exists when the summed rows of the intensity values has a contiguous range of summed rows above a threshold. In some embodiments, the intensity values are converted to absolute values of the intensity values, and the summed rows of the absolute values of the intensity values are used to determine if a contiguous range of summed rows of the absolute values is above a threshold range of contiguous values. Additionally or alternatively, the intensity may be squared and then the processor 90 may sum the squared intensity rows and determine if a contiguous range of summed rows of the intensity squared values exists beyond a threshold range of contiguous values to determine if a free flow condition exists.

In some embodiments, a predetermined range of contiguous values above a threshold (e.g., min and max ranges) of the summed rows of intensity values or intensity squared values may be used by the processor 90 to determine if a drop of liquid is within the image. For example, each row of the rows of the intensity values (or the intensity squared values) may be summed together and a range of the summed values may be above a threshold number; if the range of contiguous values is between a minimum range and a maximum range, the processor 90 may determine that the range of contiguous values above a predetermined threshold is from a drop within the field of view of the image sensor 63 (see Fig. 8). In some embodiments of the present disclosure, the summed rows of intensity values or intensity squared values may be normalized, e.g., normalized to have a value between 0 and 1.

The following describes a smoothing function similar to the cubic spline (i.e., the cubic-spline-type function) that may be used on the summed rows, the summed rows of intensity values, or the summed rows of the intensity values squared prior to the determination by the processor 90 to determine if a free flow condition exits. In some specific embodiments, the cubic-spline-type function may be used to identify blocks, as described *infra,* which may facilitate the processor's 90 identification of free flow conditions.

The cubic-spline-type function is an analog to the cubic spline, but it smoothes a data set rather than faithfully mimics a given function. Having data sampled on the interval from [0,1] (e.g., the summation along a row of intensity squared or intensity that is normalized) the processor 90 (see Figs. 6 or 8) may find the best fit set of cubic functions on the intervals $[x_0,x_1],[x_1,x_2],K,[x_{N-1},x_N]$ with $x_0=0$ and $x_N=1$ where the total function is continuous with continuous derivatives and continuous curvature.

The standard cubic spline definition is illustrated in Equation (1) as follows:

$$\chi(x) = A_i(x)y_i + B_i(x)y_{i+1} + C_i(x)y_i'' + D_i(x)y_{i+1}'' \qquad x_i \le x \le x_{i+1} \tag{1}$$

with the functions $A_i$, $B_i$, $C_i$, $D_i$ defined as in the set of Equations (2):

$$A_i\left(x\right) = \frac{x_{i+1} - x}{x_{i+1} - x_i} = \frac{x_{i+1} - x}{\Delta_i}, \qquad B_i = \frac{x - x_i}{x_{i+1} - x_i} = \frac{x - x_i}{\Delta_i}$$

$$C_i\left(x\right) = \frac{\Delta_i^2}{6}\left(A_i^3\left(x\right) - A_i\left(x\right)\right), \qquad D_i = \frac{\Delta_i^2}{6}\left(B_i^3\left(x\right) - B_i\left(x\right)\right) \tag{2}.$$

The Equations (1) and (2) guaranty continuity and curvature continuity. The only values which can be freely chosen are $y_i$, $y_0''$ and $y_N''$. Please note that Equation (3) is chosen as follows:

$$y_0'' = y_1'' = 0 \tag{3},$$

i.e., the function is flat at 0 and 1. The remaining $y_i''$ must satisfy the following set of Equations (4):

$$\frac{y_1 - y_0}{\Delta_0} + \frac{y_1'' \Delta_0}{3} = \frac{y_2 - y_1}{\Delta_1} - \frac{y_1'' \Delta_1}{3} - \frac{y_2'' \Delta_1}{6}$$

$$\frac{y_2 - y_1}{\Delta_1} + \frac{y_1'' \Delta_1}{6} + \frac{y_2'' \Delta_1}{3} = \frac{y_3 - y_2}{\Delta_2} - \frac{y_2'' \Delta_2}{3} - \frac{y_3'' \Delta_2}{6}$$

$$\frac{y_3 - y_2}{\Delta_2} + \frac{y_2'' \Delta_2}{6} + \frac{y_3'' \Delta_2}{3} = \frac{y_4 - y_3}{\Delta_3} - \frac{y_3'' \Delta_3}{3} - \frac{y_4'' \Delta_3}{6}$$

$$\mathrm{M}$$

$$\frac{y_{N-2} - y_{N-3}}{\Delta_{N-3}} + \frac{y_{N-3}'' \Delta_{N-3}}{6} + \frac{y_{N-2}'' \Delta_{N-3}}{3} = \frac{y_{N-1} - y_{N-2}}{\Delta_{N-2}} - \frac{y_{N-2}'' \Delta_{N-2}}{3} - \frac{y_{N-1}'' \Delta_{N-2}}{6}$$

$$\frac{y_{N-1} - y_{N-2}}{\Delta_{N-2}} + \frac{y_{N-2}'' \Delta_{N-2}}{6} + \frac{y_{N-1}'' \Delta_{N-2}}{3} = \frac{y_N - y_{N-1}}{\Delta_{N-1}} - \frac{y_{N-1}'' \Delta_{N-1}}{3} \tag{4}.$$

The set of Equations (4) can be rewritten as the set of Equations (5) as follows:

$$\frac{\Delta_0 + \Delta_1}{3} y_1'' + \frac{\Delta_1}{6} y_2'' = \frac{y_0}{\Delta_0} - \left[\frac{1}{\Delta_0} + \frac{1}{\Delta_1}\right] y_1 + \frac{y_2}{\Delta_1}$$

$$\frac{\Delta_1}{6} y_1'' + \frac{\Delta_1 + \Delta_2}{3} y_2'' + \frac{\Delta_2}{6} y_3'' = \frac{y_1}{\Delta_1} - \left[\frac{1}{\Delta_1} + \frac{1}{\Delta_2}\right] y_2 + \frac{y_3}{\Delta_2}$$

$$\frac{\Delta_2}{6} y_2'' + \frac{\Delta_2 + \Delta_3}{3} y_3'' + \frac{\Delta_3}{6} y_4'' = \frac{y_2}{\Delta_2} - \left[\frac{1}{\Delta_2} + \frac{1}{\Delta_3}\right] y_3 + \frac{y_4}{\Delta_3}$$

$$\mathrm{M}$$

$$\frac{\Delta_{N-4}}{6} y_{N-3}'' + \frac{\Delta_{N-3} + \Delta_{N-2}}{3} y_{N-2}'' + \frac{\Delta_{N-2}}{6} y_{N-1}'' = \frac{y_{N-3}}{\Delta_{N-3}} - \left[\frac{1}{\Delta_{N-3}} + \frac{1}{\Delta_{N-2}}\right] y_{N-2} + \frac{y_{N-1}}{\Delta_{N-2}}$$

$$\frac{\Delta_{N-1}}{6} y_{N-2}'' + \frac{\Delta_{N-2} + \Delta_{N-1}}{3} y_{N-1}'' = \frac{y_{N-2}}{\Delta_{N-2}} - \left[\frac{1}{\Delta_{N-2}} + \frac{1}{\Delta_{N-1}}\right] y_{N-1} + \frac{y_N}{\Delta_{N-1}} \tag{5}.$$

In turn, this becomes the matrix Equation (6):

$$
\begin{bmatrix}
\frac{\Delta_0+\Delta_1}{3} & \frac{\Delta_1}{6} & 0 & 0 & 0 & 0 \\
\frac{\Delta_1}{6} & \frac{\Delta_1+\Delta_2}{3} & \frac{\Delta_2}{6} & L & 0 & 0 & 0 \\
0 & \frac{\Delta_2}{6} & \frac{\Delta_2+\Delta_3}{3} & 0 & 0 & 0 \\
& M & & O & & M \\
0 & 0 & 0 & \frac{\Delta_{N-4}+\Delta_{N-3}}{3} & \frac{\Delta_{N-3}}{6} & 0 \\
0 & 0 & 0 & L & \frac{\Delta_{N-3}}{6} & \frac{\Delta_{N-3}+\Delta_{N-2}}{3} & \frac{\Delta_{N-2}}{6} \\
0 & 0 & 0 & 0 & \frac{\Delta_{N-2}}{6} & \frac{\Delta_{N-2}+\Delta_{N-1}}{3}
\end{bmatrix}
\begin{Bmatrix}
y_1'' \\ y_2'' \\ y_3'' \\ M \\ y_{N-3}'' \\ y_{N-2}'' \\ y_{N-1}''
\end{Bmatrix}
$$

$$
=
\begin{bmatrix}
\frac{1}{\Delta_0} & -\frac{1}{\Delta_0}-\frac{1}{\Delta_1} & \frac{1}{\Delta_1} & 0 & 0 & 0 \\
0 & \frac{1}{\Delta_1} & -\frac{1}{\Delta_1}-\frac{1}{\Delta_2} & L & 0 & 0 & 0 \\
0 & 0 & \frac{1}{\Delta_2} & 0 & 0 & 0 \\
& M & & O & & M \\
0 & 0 & 0 & \frac{1}{\Delta_{N-3}} & 0 & 0 \\
0 & 0 & 0 & L & -\frac{1}{\Delta_{N-3}}-\frac{1}{\Delta_{N-2}} & \frac{1}{\Delta_{N-2}} & 0 \\
0 & 0 & 0 & \frac{1}{\Delta_{N-2}} & -\frac{1}{\Delta_{N-2}}-\frac{1}{\Delta_{N-1}} & \frac{1}{\Delta_{N-1}}
\end{bmatrix}
\begin{Bmatrix}
y_0 \\ y_1 \\ y_2 \\ y_3 \\ M \\ y_{N-3} \\ y_{N-2} \\ y_{N-1} \\ y_N
\end{Bmatrix}
\quad (6)
$$

The matrix Equation (6) may be rewritten as the set of Equations (7) as follows:

$$
\mathbf{F}\mathbf{y_{dd}} = \mathbf{G}\mathbf{y}
$$

$$
\mathbf{y_{dd}} = \mathbf{F}^{-1}\mathbf{G}\mathbf{y} = \mathbf{H}\mathbf{y} \quad (7).
$$

Choosing the values in the vector **y** using a least squares criterion on the collected data is shown in Equation (8) as follows:

$$
E = \sum \left[ \psi_k - A_{i_k}\left(\xi_k\right) y_{i_k} - B_{i_k}\left(\xi_k\right) y_{i_k+1} - C_{i_k}\left(\xi_k\right) y_{i_k}'' - D_{i_k}\left(\xi_k\right) y_{i_k}'' \right]^2 \quad (8).
$$

Equation (8) is the minimum deviation between the data and the spline, i.e., Equation (8) is an error function. The y values are chosen to minimize the error as defined in Equation (8). The vector of predicted values can be written as illustrated in Equation (9) as follows:

$$
\hat{\mathbf{y}} = \left(\mathbf{A}_{\{k\}} + \mathbf{B}_{\{k\}}\right)\mathbf{y} + \left(\mathbf{C}_{\{k\}} + \mathbf{D}_{\{k\}}\right)\mathbf{y_{dd}}
$$

$$
= \left(\mathbf{A}_{\{k\}} + \mathbf{B}_{\{k\}}\right)\mathbf{y} + \left(\mathbf{C}_{\{k\}} + \mathbf{D}_{\{k\}}\right)\mathbf{H}\mathbf{y}
$$

$$
= \left[ \mathbf{A}_{\{k\}} + \mathbf{B}_{\{k\}} + \mathbf{C}_{\{k\}}\mathbf{H} + \mathbf{D}_{\{k\}}\mathbf{H} \right]\mathbf{y}
$$

$$
= \mathbf{A}\mathbf{y} \quad (9).
$$

The elements of the matrix in brackets of Equation (9) depend upon the x-value corresponding to each data point (but this is a fixed matrix). Thus, the final equation can be determined using the pseudo-inverse. In turn, the pseudo-inverse only depends upon the x-locations of the data set and the locations where the breaks in the cubic spline are set. The implication of this is that once the geometry of the spline and the size of the image are selected, the best choice for **y** given a set of measured values $\mathbf{y_m}$ is illustrated in Equation (10) as follows:

$$\mathbf{y} = \left( \mathbf{A}^T \mathbf{A} \right)^{-1} \mathbf{A} \cdot \mathbf{y_m}$$

(10).

The cubic spline through the sum intensity-squared function of the image will then be given by Equation (11) as follows:

$$\mathbf{y_{cs}} = \mathbf{A} \cdot \mathbf{y}$$

(11).

Because the maximum values of the cubic spline are of interest, the derivative of the cubic spline is determined and utilized to determine the maximum values of the cubic spline. The cubic spline derivative is given by Equation (12) as follows:

$$\chi'\left(x_k\right) = A'_{i_k}\left(x_k\right)y_{i_k} + B'_{i_k}\left(x_k\right)y_{i_k+1} + C'_{i_k}\left(x_k\right)y''_{i_k} + D'_{i_k}\left(x_k\right)y''_{i_k+1}$$

$$= -\frac{y_{i_k}}{\Delta_{i_k}} + \frac{y_{i_k+1}}{\Delta_{i_k}} - \frac{\Delta_{i_k}y''_{i_k}}{6}\left(3A^2_{i_k}\left(x_k\right)-1\right) + \frac{\Delta_{i_k}y''_{i_k+1}}{6}\left(3B^2_{i_k}\left(x_k\right)-1\right)$$

(12).

Equation (12) can be written as Equation (13) as follows:

$$\mathbf{y'_{cs}} = \left( \mathbf{A'_{\{k\}}} + \mathbf{B'_{\{k\}}} \right)\mathbf{y} + \left( \mathbf{C'_{\{k\}}} + \mathbf{D'_{\{k\}}} \right)\mathbf{y_{dd}}$$

$$= \left[ \mathbf{A'_{\{k\}}} + \mathbf{B'_{\{k\}}} + \mathbf{C'_{\{k\}}}\mathbf{H} + \mathbf{D'_{\{k\}}}\mathbf{H} \right]\mathbf{y}$$

$$= \mathbf{A'}\mathbf{y}$$

(13).

Once the current values of **y** are found, the cubic spline, $\mathbf{y_{cs}}$, and its derivative, $\mathbf{y'_{cs}}$, can be calculated. The cubic spline data may include "blocks" of data that includes values above a predetermined threshold. A pipe block is formed by the liquid flowing out of the tube into the drip chamber 59 and a pool block is formed as the liquid collects at the gravity end of the drip chamber 59 (see Fig. 8).

The following algorithm may be applied to the cubic spline data: (1) determine the local maxima of the cubic spline data using the derivative information; (2) determine the block surrounding each local maxima by including all points where the cubic spline value is above a threshold value; (3) merge all blocks which intersect; (4) calculate information about the block of data including the center of mass (intensity), the second moment of the mass (intensity), the lower x-value of the block, the upper x-value of the block, the mean value of the original sum of intensity squared data in the block, the standard deviation of the original sum of intensity squared data in the block, and the mean intensity of a high-pass filtered image set in the block; and (5) interpret the collected data to obtain information about when drops occur and when the system is streaming.

The mean intensity of a high-pass filtered image set in the block is used to determine if the block created by each contiguous range of spline data is a result of a high frequency artifact (e.g., a drop) or a low frequency artifact. This will act as a second background filter which tends to remove artifacts such as condensation from the image. That is, all previous images in an image memory buffer (e.g., 30 previous frames, for example) are used to determine if the data is a result of high frequency movement between frames. If the block is a result of low frequency changes, the block is removed, or if it is a result of high frequency changes, the block is kept for further analysis. A finite impulse response filter or an infinite impulse response filter may be used.

Each block is plotted over its physical extent with the height equal to the mean value of the data within the block. If a block has a mean value of the high-pass filtered image less than the threshold, it is an indication that it has been around for several images and thus may be removed.

Free flow conditions may be determined by the processor 90 (see Figs. 6 or 8) to exist using the blocks when the pipe block extends nearly to the pool block, the pipe block and the pool block merge together, and/or the summed range of widths of the pool and pipe blocks (or all blocks) is greater than a predetermined threshold, e.g., the total extent of the blocks exceeds 380 pixels in width. The processor 90 may detect a drop when the transition of the pipe block from a larger width to a shorter width occurs as a result of a drop formation in the tube and as the drop leaves the pipe (i.e., tube) opening of the drip chamber 59. The processor 90 may detect this by looking at the ratio of the current pipe block width to the previous image's pipe block width, e.g., an image where the ratio is less than 0.9 as is also a local minima may be considered by the processor 90 to be an image formed immediately after a drop has formed.

Various filtering algorithms may be used to detect condensation or other low frequency artifacts, such as: if a block has a low mean value in the high-pass filtered image, then it may be condensation. This artifact can be removed from consideration. Additionally or alternatively, long blocks (e.g., greater than a predetermined threshold) with a low high-pass mean value are possibly streams because stream images tend to remain unchanging; the processor 90 may determine that long blocks greater than a predetermined threshold corresponds to a streaming condition. Additionally or alternatively, an algorithm may be used on the current image to detect free flow conditions.

The processor 90 may, in some specific embodiments, use the block data to count the drops to use the system 84 as a drop counter. The processor 90 may also use width changes in the pool block as a drop disturbs the water to determine if a bubble formed with the drop hits the pool. For example, the processor 90 may determine that blocks that form below the pool block are from bubbles that formed when the drop hit the water. The bubble may be filtered out by the processor 90 when determining if a predetermined value of total block ranges indicates that a free flow condition exists.

In some embodiments of the present disclosure, the depth of field of the system 84 may have a narrow depth of field to make the system 84 less sensitive to condensation and droplets on the chamber walls. In some embodiments, a near focus system may be used.

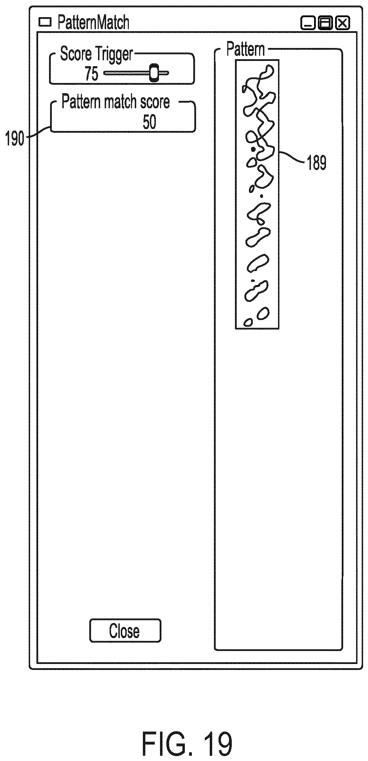

Referring now to Fig. 19, in another embodiment of the present disclosure, a template 189 is used to determine if a free flow condition exists. The template 189 is used by the processor 90 of Fig. 8 to determine a pattern match score 190 when performing a template match algorithm on an image, e.g., the image 94 of Fig. 13. For example, the template 189 may be compared to the image 94 to determine if a portion or all of the image 94 closely matches the template 189. As previously mentioned, the image 94 of Fig. 13 is a difference between a background image and an image captured by the image sensor 63 of Fig. 8 that has each pixel converted to either a black pixel if the difference value for that pixel is below a threshold value or a white pixel if the difference value for that pixel is above a threshold value. All pixels of the image 94 will be either a white pixel or a black pixel. If the pattern match score 190 is above a predetermined threshold, a free flow condition is determined to exist. The template matching method may utilize a template matching algorithm as found in the Open Source Computer Vision ("OpenCV") library. For example, the template 189 may be used with the matchTemplate() function call of the OpenCV library using the CV_TM_CCOEFF method or the method of CV_TM_CCOEFF_NORMED. The CV_TM_CCOEFF method uses the pattern matching algorithm illustrated in Equation (14) as follows:

$$R(x, y) = \sum_{x',y'} \left( T'(x', y') \cdot I'(x + x', y + y') \right) \qquad ,(14) ,$$

where:

$$T'(x', y') = T(x', y') - 1/(w \cdot h) \cdot \sum_{x'',y''} T(x'', y'')$$
$$I'(x + x', y + y') = I(x + x', y + y') - 1/(w \cdot h) \cdot \sum_{x'',y''} I(x + x'', y + y'')_,$$

The I denotes the image, the T denotes the template, and the R denotes the results. The summation is done over the template and/or the image patch, such that: $x' = 0...w\text{-}1$ and $y' = 0...h\text{-}1$.

The results R can be used to determine how much the template T is matched at a particular location within the image I as determined by the algorithm. The OpenCV template match method of CV_TM_CCOEFF_NORMED uses the pattern matching algorithm illustrated in Equation (15) as follows:

$$R(x, y) = \frac{\sum_{x',y'} (T'(x', y') \cdot I'(x + x', y + y'))}{\sqrt{\sum_{x',y'} T'(x', y')^2 \cdot \sum_{x',y'} I'(x + x', y + y')^2}} \quad (16).$$

In another embodiment of the present disclosure, the template matching algorithm uses a Fast Fourier Transform ("FFT"). In some embodiments, any of the methods of the matchTemplate() function of OpenCV may be used, e.g., CV_TM_SQDIFF, CV_TM_SQDIFF_NORMED, CV_TM_CCORR, and/or CV_TM_CCORR_NORMED.

The CV_TM_SQDIFF uses the pattern matching algorithm illustrated in Equation (17) as follows:

$$R(x, y) = \sum_{x',y'} (T(x', y') - I(x + x', y + y'))^2 \quad (17).$$

CV_TM_SQDIFF_NORMED uses the pattern matching algorithm illustrated in Equation (18) as follows:

$$R(x, y) = \frac{\sum_{x',y'} (T(x', y') - I(x + x', y + y'))^2}{\sqrt{\sum_{x',y'} T(x', y')^2 \cdot \sum_{x',y'} I(x + x', y + y')^2}} \quad (18).$$

CV_TM_CCORR uses the pattern matching algorithm illustrated in Equation (19) as follows:

$$R(x, y) = \sum_{x',y'} (T(x', y') \cdot I(x + x', y + y')) \quad (19).$$

CV_TM_CCORR_NORMED uses the pattern matching algorithm illustrated in Equation (20) as follows:

$$R(x, y) = \frac{\sum_{x',y'} (T(x', y') \cdot I'(x + x', y + y'))}{\sqrt{\sum_{x',y'} T(x', y')^2 \cdot \sum_{x',y'} I(x + x', y + y')^2}} \quad (20).$$

In yet another embodiment of the present disclosure, a template of a grayscale image of a free flow condition is compared to an image taken by the image sensor 63 of Fig. 8 to determine if a free flow condition exists. In some embodiments, the template matching function within the OpenCV library may be utilized.

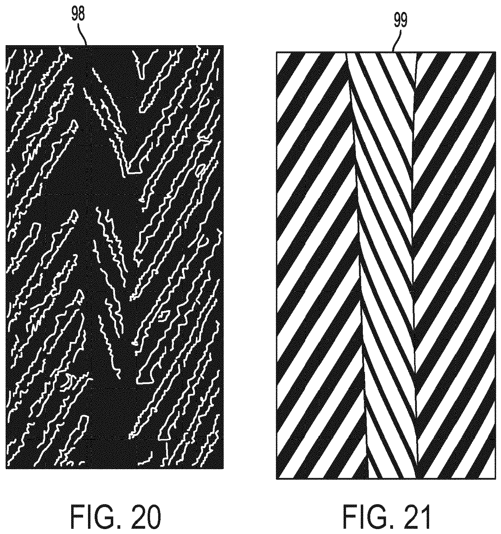

Refer now to Figs. 20 and 21; in yet an additional embodiment of the present disclosure, the algorithm to determine when a free flow condition exists, e.g., as executed by the processor 90 of Fig. 8, may utilize an algorithm to determine if a template pattern matches an array of pixels utilizing edge detection followed by line detection. As shown in Fig. 20, an image 98 is formed from an image 99 of Fig. 21, by using edge detected followed by line detection. The resulting lines may be utilized by the processor 90 to determine that a free flow condition exists. As shown in Fig. 20, the feature which shows up after this processing by the processor 90 are lines that have a different slope than the expected 45° slope of the background reference image. The lines having the angle of the background image may be filtered out of Fig. 20, in some embodiments. The lines may be detected as edges using a Canny algorithm as found in the OpenCV library. The Hough algorithm also found in the OpenCV library may be used to determine the slope of the lines.

One type of Hough transfer uses an algorithm described in Progressive Probabilistic Hough Transform by J. Matas, C. Galambos, and J. Kittler in 1998 ("Algorithm 1"). However, the following "Alternative Hough" transform may be utilized and is shown in pseudo code form in Table 1 ("Algorithm 2"). Algorithm 2 selects two pixels at random and calculates the Hough transform of the line passing through these two points. Algorithm 2 is shown in Table 1 as follows:

| Table 1 |
|---|
| Alternative Hough Transform Pseudocode |
| 1. If the image is empty, then exit.<br>2. Randomly select two pixels and update the accumulator<br>   a. Required Operations<br>      i. Two random numbers<br>      ii. One inverse tangent<br>3. Check if the new location is higher than the threshold I. If not, goto 1<br>   a. Operations<br>      i. One logical operation<br>4. Look along a corridor specified by the peak in the accumulator, and find the longest segment of pixels either continuous or exhibiting a gap not exceeding a given threshold.<br>5. Remove the pixels in the segment from the input image.<br>6. Unvote from the accumulator all the pixels from the line that have previously voted.<br>7. If the line segment is longer than the minimum length add it to the output list<br>8. Goto 1. |

If the line comprises a proportion, $p$, of the total points, then the likelihood that we will see a result in the representative $(r,\theta)$-bin is $p$ for Algorithm 1 and $p^2$ for Algorithm 2. Generally, in some embodiments, a proportion test has at least 5 positive results and 5 negative results. Assuming that it is more likely to see negative results than positive results, in some embodiment, the Algorithms 1 and 2 continue to search for lines until there are at least 5 positive results in a particular bin.

The probability of seeing a fifth positive result in Algorithm 1 after $N \geq 5$ tests is shown in Equation (21) as follows:

$$p_1\left(5 \text{ on } N\right) = p\left(4 \text{ in } N-1\right) \cdot p = \frac{\left(N-1\right)!}{4!\left(N-5\right)!} p^5 \left(1-p\right)^{N-5} \quad (21),$$

and the probability in Algorithm 2 is shown in Equation (22) as follows:

$$p_2\left(5 \text{ on } N\right) = p\left(4 \text{ in } N-1\right) \cdot p^2 = \frac{\left(N-1\right)!}{4!\left(N-5\right)!} p^{10} \left(1-p^2\right)^{N-5} \quad (22).$$

Table 2, shown below, shows the number of tries to have a 50% chance of seeing 5 successes, $p_{1,50}$ and $p_{2,50}$, as well as the number of tries to have a 90% chance of seeing 5 successes, $p_{1,90}$ and $p_{2,90}$.

| TABLE 2 | | | | | | |
|---|---|---|---|---|---|---|
| $p$ | $p_{1,50}$ | $p_{1,90}$ | $p_{2,50}$ | $p_{2,90}$ | $r_{50}$ | $r_{90}$ |
| **0.5** | 9 | 14 | 20 | 31 | 2.22 | 2.21 |
| **0.25** | 19 | 30 | 76 | 127 | 4 | 4.23 |
| 0.125 | 39 | 62 | 299 | 511 | 7.67 | 8.24 |
| **0.0625** | 76 | 127 | 1197 | 2046 | 15.75 | 16.11 |

Table 2 shows that the increase in the number of tries between Algorithm 1 and Algorithm 2 to see 5 positive results is approximately $^1/_p$. There should be 1 positive result in $^1/_p$ trials when the proportion is $p$.

Algorithm 2's computationally expensive operation is, in some embodiments, the arc tangent function, which may be about 40 floating point CPU operations. There are approximately $2N$ floating point operations in Algorithm 1's equivalent step. The Hough transform of a 640×480 pixel image with full resolution has $N$ equal to 2520, while the Hough transform of a 1080x1920 pixel image has $N$ equal to 7020. This implies that Algorithm 2 has a speed advantage over Algorithm 1 when *p* is greater than 0.008 for a 640x480 image and when *p* is greater than 0.003 for a 1080×1920 image.

In some embodiments, it is assumed that every bin in the Hough transform space is equally likely to be occupied in the presence of noise. This simplification speeds up the thresholding decision; however, in some embodiments, this assumption is not true. The primary effect of the simplification is to underestimate the probability that is seen in values greater than one in the Hough transform with a corresponding likelihood of falsely declaring that a line exists. For a particular combination of image size and Hough transform bin arrangement, the true probabilities can be pre-computed. This allows the false alarm rate to be minimized without a corresponding increase in computation. With additional restrictions on the type of imagery even more accurate estimates of the probability of seeing a value in a bin of the Hough transform is possible.

There are additional forms of the Hough transform which parameterizes different features. For example, there is a three-element parameterization of circles, (*x*,*y*,*r*), where x and y specify the center and r is the radius. Algorithm 2 can work using these parameterizations as well. For the circle example, Algorithm 2 would select three pixels at random and calculate the circle passing through them.

Algorithm 2 would have a similar speed advantage for features comprising a suitably large portion of the total pixels considered. It would also have a significant advantage in storage required, since the Hough transform could be stored in a sparse matrix, while the Algorithm 1's analog would require a full-size matrix.

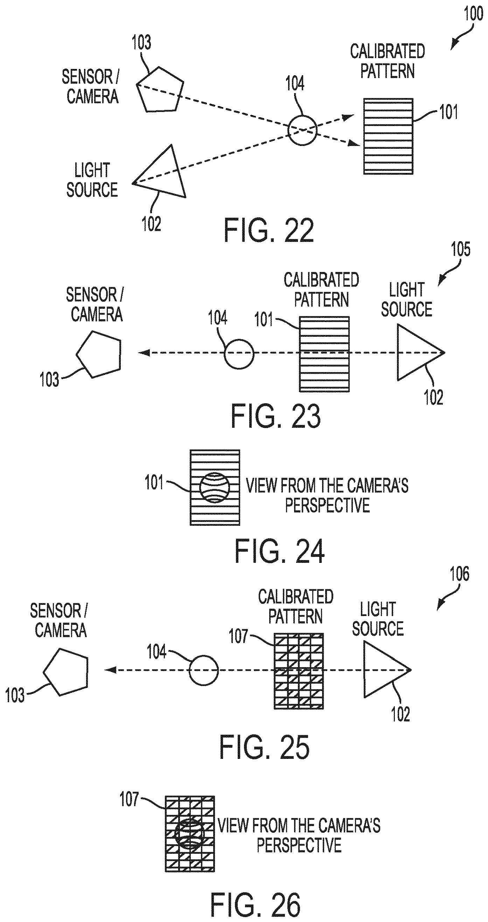

Referring now to Figs. 22-26, which illustrate various background patterns that may be used to detect a free flow condition or estimate the size of a drop of liquid. The image sensor 103 may be used with the background patterns of Figs. 22-26 and may be the image sensor 11 of Fig. 1, the image sensor 68 of Fig. 5, the image sensor 63 of Fig. 6, or the image sensor 63 of Fig. 8, each of which may be coupled to a respective processor for processing the images from the image sensor, such as the processor 15 of Fig. 1 or the processor 90 of Fig. 8.

Fig. 22 is a block diagram of an imaging system 100 for use with the drip chamber 104 (e.g., a drip chamber 4 of Fig. 1) having a background pattern 101 with stripes and a light source 102 shining on the stripes from an adjacent location to an image sensor 103 in accordance with an embodiment of the present disclosure. Any drops or free flow streams within the drip chamber 104 distorts the image taken by the image sensor 103. A processor coupled to the image sensor 103 (e.g., processor 15 of Fig. 1) can use the distortions of the background pattern 101 as captured by the image sensor 103 to estimate a flow rate and/or detect free flow conditions.

Fig. 23 is a block diagram of an imaging system 105 for use with the drip chamber 104 having a background pattern 101 with stripes and a light source 102 shining on the stripes from behind the background pattern 101 relative to an opposite end to an image sensor 103 in accordance with an embodiment of the present disclosure. Fig. 24 shows an image from the image sensor 103 of Fig. 23 when a drop distorts the background pattern 101 of Fig. 23 in accordance with an embodiment of the present disclosure. Note that as shown in Fig. 24, the background pattern's 101 stripes are distorted by a drop (or will be distorted by a free flow stream) in the drip chamber 104 as captured in images by the image sensor 103. This distortion may be used to estimate the drop size, to calculate the flow rate through a drip chamber, or to determine if a free flow condition exists within the drip chamber.

Fig. 25 shows a block diagram of an imaging system for use with a flow meter having a background pattern with a checkerboard pattern and a light source shining on the stripes from behind the background pattern relative to an opposite end to an image sensor in accordance with an embodiment of the present disclosure. Fig. 26 shows an image from the image sensor of Fig. 25 when a drop distorts the background pattern 107 of Figs 25-26 in accordance with an embodiment of the present disclosure. In yet another embodiment of the present disclosure, a background pattern having a plurality of random dots and/or circles may be utilized by an imaging system disclosed herein.

Referring to Figs. 22-26, the "lensing" of a drop (i.e., the distortion of the background pattern from the view of an image sensor) may be used to measure the radius of the drop. The radius of the drop corresponds to how much and what effect the drop has on any light passing through it. By measuring the change to the calibration grid (i.e., the background pattern) as seen through the drop, the radius, and hence the volume of the drop, can be calculated. For example, the magnification of a test grid of known size as seen through the drop could be measured optically and the radius inferred from this measurement. In some embodiments of the present disclosure, the relationship between the radius and the drop may be calculated and/or may be determined using a lookup table that has been generated empirically.

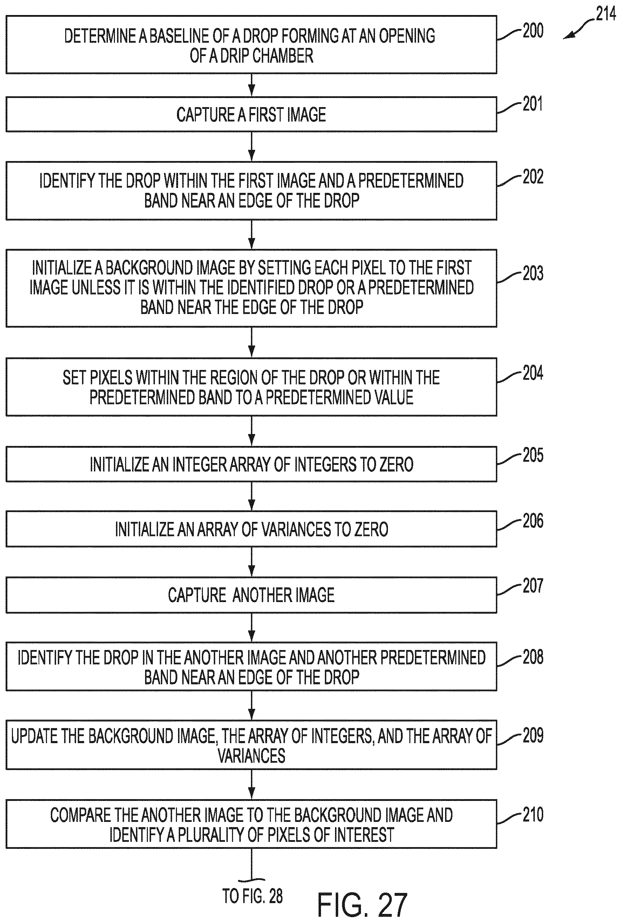

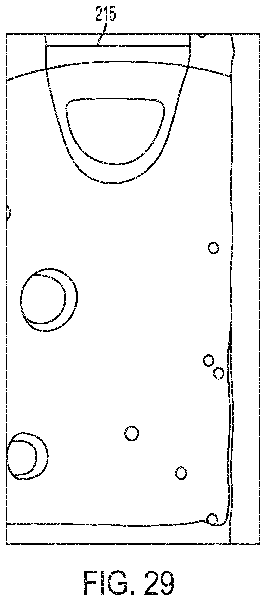

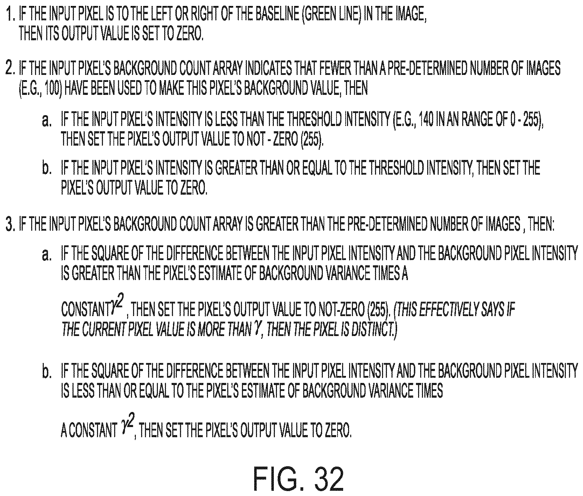

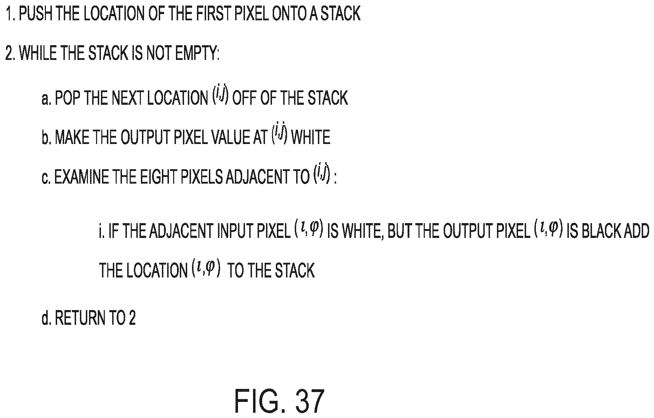

Figs. 27-28 show a flow chart diagram illustrating a method for estimating a volume of a drop within a drip chamber in accordance with an embodiment of the present disclosure. That is, Figs. 27-28 illustrate a method 214. Method 214 will be also described with reference to Figs. 29-37. Figs. 29-31 and 33-36 illustrate images used or generated by a flow meter to estimate a volume of a drop within a drip chamber in accordance with an embodiment of the present disclosure. Figs. 32 and 37 illustrate pseudo code that may be used by the method 214 of Figs. 27-28.

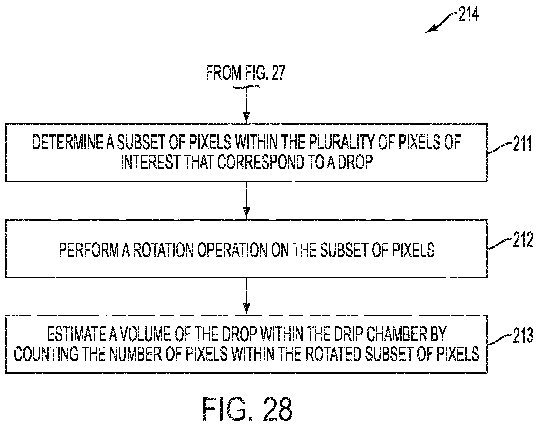

The method 214 of Figs. 27 and 28 may be implemented by the flow meter 7 of Fig. 1, the flow meter 67 of Fig. 5, the imaging system 78 of Fig. 6, the imaging system 84 of Fig. 8, or other flow meter of an imaging system disclosed herein (each with or without a background pattern and/or with or without active illumination).

The method 214 includes acts 200-213. Act 200 determines a baseline of a drop forming at an opening of a drip chamber. Act 201 captures a first image. The first image may be captured using a uniform backlight. In some embodiments, the first image may be captured using a background pattern and/or an exposure algorithm as described herein. Acts 200 and 201 may be performed simultaneously. Fig. 29 shows an image with the baseline 215 overlaid. The baseline 215 may be a predetermined group of pixels or may be generated using fiducial markers disposed on the opening of the drip chamber and/or on a background pattern (not shown in Fig. 29). The first image is used by the method 214 to initialize a background image, $\mu_{i,j}$, a variance array, $s_{i,j}$, and an integer array, $I_{i,j}$. The background image may have i by j pixels, while the variance array and the integer array may be 2-D arrays that also have a size of i by j.

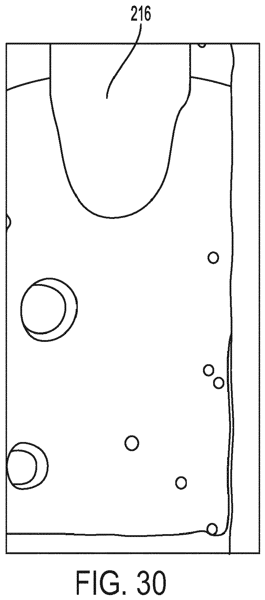

Act 202 identifies the drop within the first image and a predetermined band near an edge of the drop (e.g., the band may be a predetermined number of pixels beyond the edge of the drop). Act 203 initializes a background image by setting each pixel to the same value as the first image (for that respective location) unless it is within the identified drop or a predetermined band near the edge of the drop. Act 204 sets pixels within the region of the drop or within the predetermined band to a predetermined value. Fig. 30 shows an example background image created after initialization. In the exemplary image of Fig. 30, the area of the drop and of a band beyond the edge of the drop, designated generally as 216, is set to a predetermined value, e.g., 140.

For example, when the method creates the first background image, every pixel in the background image that is part of the drop or a band outside of an edge of the drop is set to a default threshold value, e.g. 140 out of an intensity range of 0-255.

Act 205 initializes the integers of the array of integers to zeros. Act 206 initializes the values within the array of variances to zeros. The integer array is the same size as the image. The integer array counts how often each pixel of the background image has been updated with new information and is initialized to all zeros. The array of variances (e.g., an array of the data type "double") is also the same size as the background image and contains an estimate of the variance of the intensity of each pixel within the background image.

Act 207 captures another image and act 208 identifies the drop in the another image and another predetermined band near an edge of the drop. Act 209 updates the background image, the array of integers, and the array of variances.

As additional images are captured, the background image may be updated. For example, when an image is collected by the system, the background algorithm evaluates every pixel. If a pixel is considered part of the drop or its guard band, then its value in the background image is not altered.

If a pixel is not considered part of the drop or its guard band: (1) if the pixel's corresponding integer in the integer array is zero, the pixel's value in the background image is set equal to the pixel's value in the input image; or (2) if the pixel's count is greater than 0, then the background image value for that pixel is updated using a low pass filter. In some embodiments, any style of filter may be used, such as a high pass filter, a bandpass filter, etc. One low pass filter that may be used is illustrated in Equation (23) as follows:

$$P_{\text{background},i,j} = P_{\text{background},i,j}\left(1-\alpha_{\text{background}}\right) + \alpha_{\text{background}}P_{\text{input},i,j} \quad (23).$$

In addition, the variance array may be updated using Equations (24) as follows:

$$\sigma^2_{\text{temp}} = \left(P_{\text{background},i,j} - P_{\text{input},i,j}\right)^2$$

$$\sigma^2_{\text{background},i,j} = \sigma^2_{\text{background},i,j}\left(1-\beta_{\text{background}}\right) + \beta_{\text{background}}\sigma^2_{\text{temp}} \quad (24).$$

Note that the filter used for both operations is an exponential filter; however, in additional embodiments, other suitable filters may be used, such as other low-pass filters. The variance estimate can be performed in any known way or using a stand in for the estimate, e.g., using standard deviation.

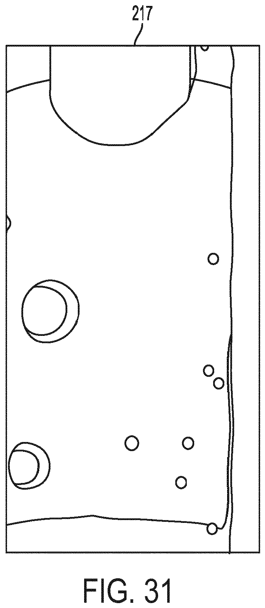

The new estimates of each pixel's background intensity (mean value), the number of images used to update each pixel's mean and variance, and each pixel's variance (e.g., an approximation to the true variance and/or a value that is proportional to the variance) are used to update the arrays. That is, each additional image captured may be used to update the background image, the array of integers, and the array of variances. After several images have been processed, the background image may appear as Fig. 31. Note that this image still has a region (the uniformly medium gray area, designated generally as 217) where the pixels have never changed from the initial threshold value. This region has been considered part of the drop or its guard band in every image.

Act 210 compares the another image (e.g., current or most recent image) to the background image and identifies a plurality of pixels of interest. Act 211 determines a subset of pixels within the plurality of pixels of interest that corresponds to a drop.

The comparison of act 210 compares the another image pixel-by-pixel to the background image. Out of this comparison comes an array the same size as the image where every pixel has a value of zero or not zero (255).

Act 210 may be implemented by the pseudo code shown in Fig. 32. That is, the determination of this threshold value is made in accordance with the following: If the input pixel is to the left or right of the baseline in the image, then its output value is set to zero (Line 1); if the input pixel's background count array indicates that fewer than a pre-determined number of images (e.g., 100) have been used to make this pixel's background value (Line 2), then: if the input pixel's intensity is less than the threshold intensity (e.g., 140 in a range of 0-255), then set the pixel's output value to not-zero (255) (Line 2a); or if the input pixel's intensity is greater than or equal to the threshold intensity, then set the pixel's output value to zero (Line 2b); and if the input pixel's background count array is greater than the pre-determined number of images (Line 3), then: if the square of the difference between the input pixel intensity and the background pixel intensity is greater than the pixel's estimate of background variance times a 2 constant $\gamma^2$, then set the pixel's output value to not-zero (255) (Line 3a) (that is, if the difference between current pixel value and the background image is more than $\gamma$, then the pixel is distinct); or if the square of the difference between the input pixel intensity and the background pixel intensity is less than or equal to the pixel's estimate of background variance times a constant $\gamma^2$, then set the pixel's output value to zero (see Line 3b). Line 3 captures portions of the image that are altered by the presence of a drop, but which are made a higher intensity.

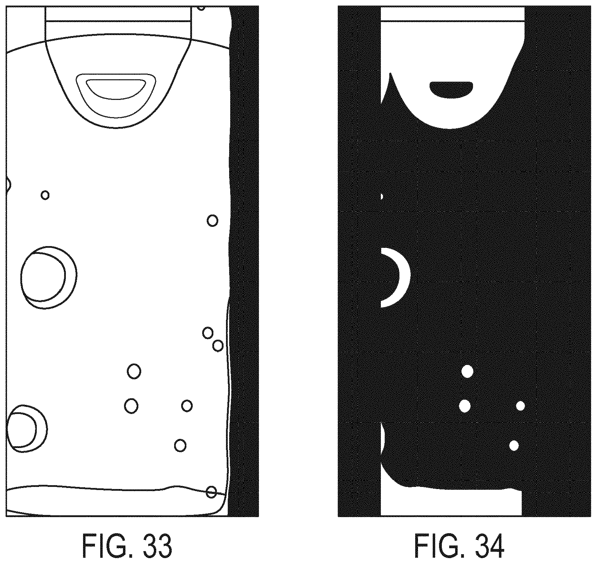

When act 210 is implemented as an algorithm, the algorithm is initialized, the input and output of this thresholding algorithm will look like the images in Figs. 33 and 34, respectively. Because the number of images used in estimating the background image is initially small, the only criterion applied are shown as lines (1) and (2) above because there have not been enough images used for the integer array to have a value beyond the threshold for certain respective pixels. This may result in many low-intensity regions being identified as distinct, including poorly illuminated edges and condensation on the chamber walls.

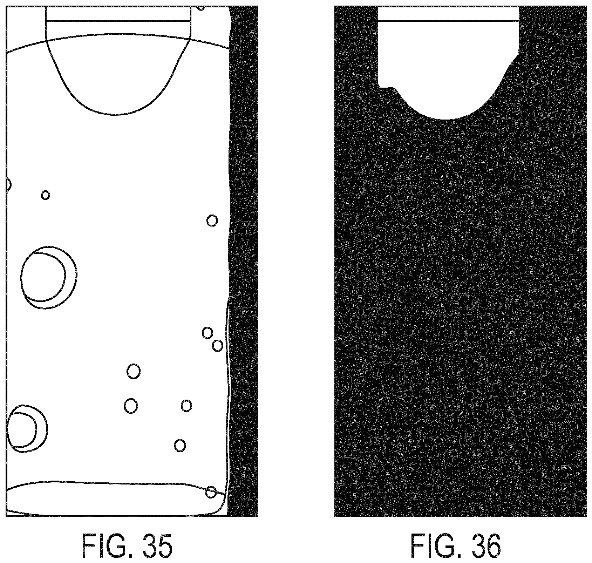

After enough images have been gathered such that most (or all) of the pixels of the background image have been generated with a sufficient number of pixels, lines (3), (3a), and (3b) of Fig. 32 are utilized. After thresholding, the background is largely black with an occasional noisy pixel exceeding the variance threshold, as shown in Figs. 35 and 36 (which show an image captured by the camera and the results of the comparison algorithm described above, respectively).

As previously mentioned, after act 210, act 211 determines which of a subset of pixels within the plurality of pixels of interest corresponds to a drop. Act 211 may be implemented by the pseudo code shown in Fig. 37. That is, the threshold image is passed to an algorithm which finds the connected component representing the drop as illustrated by the pseudo code of Fig. 37.

The binary image after processing the pseucode of Fig. 32 is evaluated to find the binary component which occupies the space given by the drop. The algorithm is passed the location of a pixel on the baseline which is white (or it is passed the center pixel of the longest stretch of contiguous white pixels on the line).

Once the algorithm has an initial white pixel, it performs the algorithm illustrated by the pseudo code shown in Fig. 37. The pseudo code determines locations that include white pixels that have a path to the baseline (i.e., a white pixel path). Line 1 pushes the location of the first pixel onto a stack. Line 2 performs a while loop while the stack is not empty. The while loop includes lines (2a)-(2d). Line 2a pops the next location $(i,j)$ off of the stack. Line 2b makes the output pixel value at $(i,j)$ white. Line 2c examines the eight pixels adjacent to $(i,j)$. Line (2ci) is an "if statement" and if the adjacent input pixel $(\iota,\varphi)$ is white, but the output pixel $(\iota,\varphi)$ is black, line 2c adds the location $(\iota,\varphi)$ to the stack. Line 2d return to line 2 to continue the while loop (if the stack remains empty).

This algorithm will set to white all output pixel locations which can be connected to the input pixel's location by a continuous path of white input pixels. The left boundary of the drop is found by stepping through each row of pixels from the left edge until the algorithm hits a white pixel. The right boundary is found by stepping from the right edge of the image until it hits a white pixel. The first row where it is possible to step from the left edge to the right edge without hitting a white pixel is where the drop is considered to end.

The pseudo code shown in Fig. 37 is a one-pass version of a connected-component labeling algorithm. However, other connected-component labeling algorithms or other suitable algorithms may be used to determine which pixels correspond to the drop.

Act 212 of Fig. 28 performs a rotation operation on the subset of pixels. Act 213 estimates a volume of the drop within the drip chamber by counting the number of pixels within the rotated subset of pixels. The total number of pixels within the 3-D version of the drop is counted; and because each pixel corresponds to a distance, the number of pixels may be used to estimate the volume of the drop.

**Imaging System Optics**

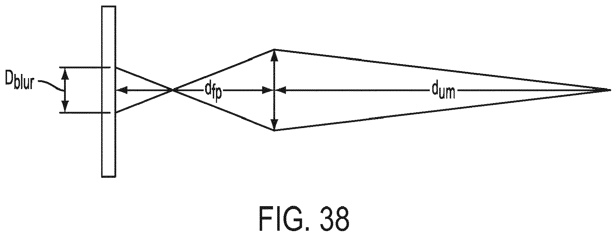

Figs. 38-42 facilitate the following description of the optics of an imaging system disclosed herein. For example, an image sensor disclosed herein may be an image sensor cube manufactured by OmniVision of 4275 Burton Drive, Santa Clara, California 95054; and, for example, the image sensor cube may be one manufactured for phone image sensor applications. In some embodiments of the present disclosure, an image sensor disclosed herein may use a fixed focus and have a depth of field ("DOF") from 15 centimeters to infinity.

The image sensor may have the blur circle of a point imaged in the range of the image sensor entirely contained within the area of a single pixel. The focal length of the image sensor lens may be 1.15 millimeters, the F# may be 3.0, and the aperture of the lens of the image sensor may be 0.3833 millimeter. A first order approximation of the optical system of one or more of the image sensors may be made using matrix equations, where every ray, r, is represented as the vector described in Equation (25) as follows:

$$r = \left\{ \begin{matrix} h \\ \theta \end{matrix} \right\} \quad (25).$$

In Equation (25) above, h is the height of the ray at the entrance to the image sensor, and $\theta$ is the angle of the ray. Referring to Fig. 38, when imaging a hypothetical point at a distance $d_{im}$ from the lens of one of the image sensors (which has focal length f) and the lens is a distance $d_{fp}$ from the focal plane, the corresponding matrix, $M_{cam}$, describing the image sensor is described by Equation (26) as follows:

$$M_{cam} = \begin{bmatrix} 1 & d_{fp} \\ 0 & 1 \end{bmatrix} \cdot \begin{bmatrix} 1 & 0 \\ -\frac{1}{f} & 1 \end{bmatrix} \cdot \begin{bmatrix} 1 & d_{im} \\ 0 & 1 \end{bmatrix} \quad (26).$$

To find the place on the focal plane, fp, where the ray strikes, a matrix multiplication as described in Equation (27) as follows may be used:

$$\left\{ \begin{matrix} h_{fp} \\ \theta_{fp} \end{matrix} \right\} = M_{cam} \cdot \left\{ \begin{matrix} h_{im} \\ \theta_{im} \end{matrix} \right\} \quad (27).$$

As illustrated in Fig. 38, the diameter of the blur circle, $D_{blur}$, is shown as approximately the distance between the two points illustrated in Fig. 38. This distance is found by tracing rays from the point $d_{im}$ away from the lens on the optical axis to the edges of the lens and then to the focal plane. These rays are given by the vectors shown in (28) as follows:

$$\left\{ \begin{matrix} 0 \\ \left( \pm \tan^{-1} \frac{D_{lens}}{2 * d_{im}} \right) \end{matrix} \right\} \quad (28).$$

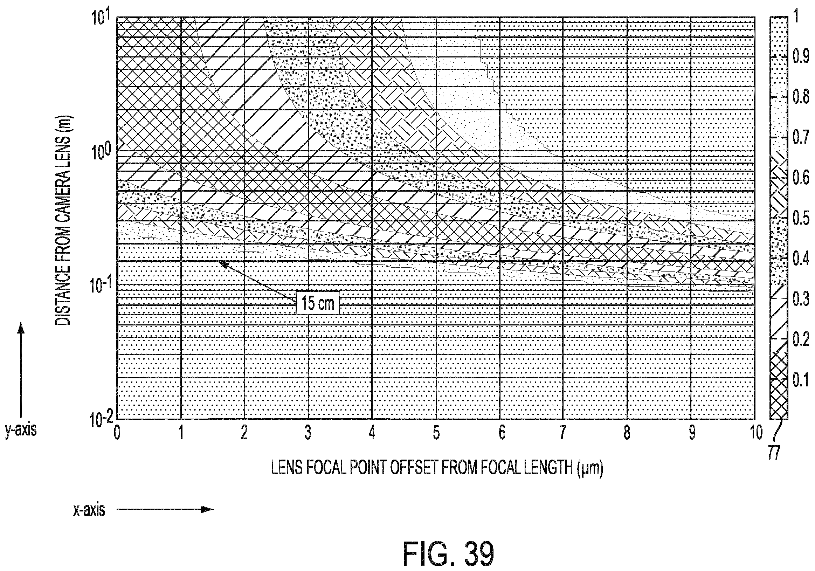

As shown in Fig. 39, the blur circle, $D_{blur}$, is calculated and shown for a variety of lens-to-focal plane separations and lens-to-image separations. A contour map 77 is also shown in Fig. 39. The x-axis shows the distance in microns between the focal plane and a point located a focal length away from the lens of an image sensor. The y-axis shows the distance in meters between the lens and the point being imaged. The values creating the contour map 77 is the blur size divided by the pixel size; therefore anything about 1 or less is sufficient for imaging. As shown in Fig. 39, the focal plane is located a focal length and an additional 5 micrometers away from the lens.

The image sensor may utilize a second lens. For example, an image sensor may utilize a second lens to create a relatively larger depth of field and a relatively larger field of view. The depth of field utilizing two lenses can be calculated using the same analysis as above, but with the optical matrix modified to accommodate for the second lens and the additional distances, which is shown in Equation (29) as follows:

$$M_{sys} = \begin{bmatrix} 1 & d_{fp} \\ 0 & 1 \end{bmatrix} \cdot \begin{bmatrix} 1 & 0 \\ -\dfrac{1}{f_{cam}} & 1 \end{bmatrix} \cdot \begin{bmatrix} 1 & d_{lens} \\ 0 & 1 \end{bmatrix} \cdot \begin{bmatrix} 1 & 0 \\ -\dfrac{1}{f_{lens}} & 1 \end{bmatrix} \cdot \begin{bmatrix} 1 & d_{im} \\ 0 & 1 \end{bmatrix} \quad (29).$$

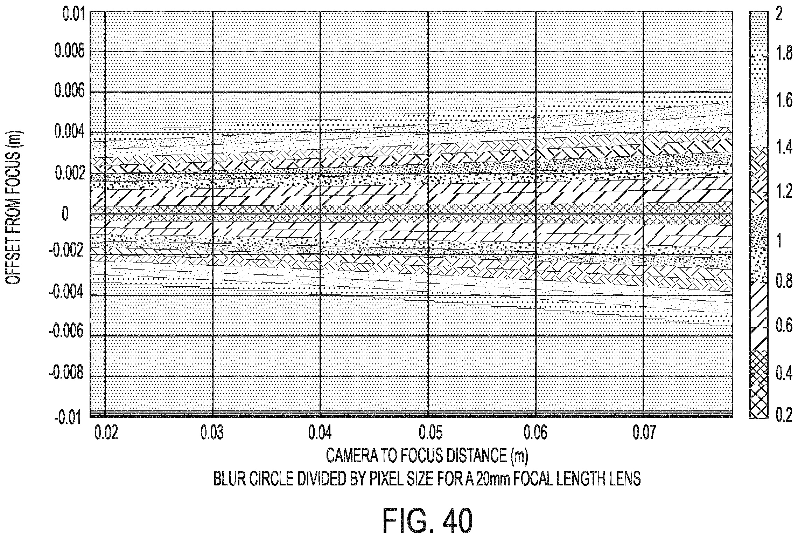

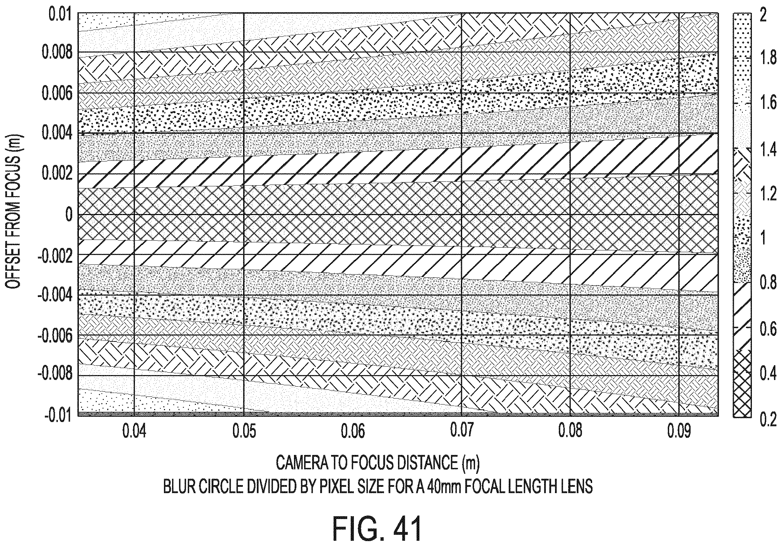

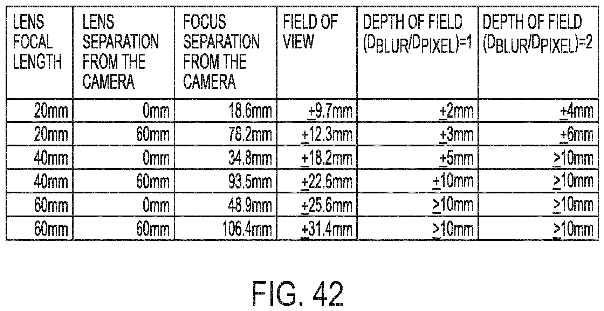

Figs. 40 and 41 illustrate the field changes with the separation between the lens and the image sensor and the corresponding change in the focus of the image sensor. Figs. 40 and 41 show the blur circle divided by the pixel size. Fig. 40 shows the blur circle divided by pixel size when a 20 millimeter focal length lens is used. Fig. 41 shows the blur circle divided by pixel size when a 40 millimeter focal length lens is used. The corresponding fields of views about the optical axis for the corners of the two configurations of Figs. 40 and 41 are shown in the table in Fig. 42.

As shown in Fig. 42, in some embodiments, the image sensor may utilize a 40mm to 60mm focal length lens; this configuration may include placing an image sensor about 2 inches from the focus. In other embodiments of the present disclosure, other configurations may be used including those not shown in Fig. 42.

For example, the following analysis shows how the depth of field can be set for an image sensor: using a lens of focal length, f, a distance, z, from the focal plane, and a distance, d, from a point in space; a matrix of the system is shown in Equation (30) as follows:

$$M = \begin{bmatrix} 1 & z \\ 0 & 1 \end{bmatrix} \cdot \begin{bmatrix} 1 & 0 \\ -\dfrac{1}{f} & 1 \end{bmatrix} \cdot \begin{bmatrix} 1 & d \\ 0 & 1 \end{bmatrix} \quad (30).$$

Equation (30) reduces to Equation (31) as follows:

$$M = \begin{bmatrix} 1 & z \\ 0 & 1 \end{bmatrix} \cdot \begin{bmatrix} 1 & d \\ -\dfrac{1}{f} & 1-\dfrac{d}{f} \end{bmatrix} \quad (31).$$

Equation (31) reduces to Equation (32) as follows:

$$M = \begin{bmatrix} 1-\dfrac{z}{f} & d+z-\dfrac{dz}{f} \\ -\dfrac{1}{f} & 1-\dfrac{d}{f} \end{bmatrix} \quad (32).$$

Considering the on-axis points, all of the heights will be zero. The point on the focal plane where different rays will strike is given by Equation (33) as follows:

$$\left( d+z-\dfrac{dz}{f} \right)\theta \quad (33).$$

As shown above in (33), $\theta$ is the angle of the ray. The point in perfect focus is given by the lens maker's equation given in Equation (34) as follows:

$$\frac{1}{f} = \frac{1}{z} + \frac{1}{d} \quad (34).$$

Equation (34) may be rearranged to derive Equation (35) as follows:

$$d = \frac{1}{\frac{1}{f} - \frac{1}{z}} = \frac{fz}{z - f} \qquad (35).$$

Inserting d from Equation (35) into Equation (33) to show the striking point results in Equation (36) as follows:

$$\left[ \frac{fz}{z-f} + z - \frac{\frac{fz}{z-f}z}{f} \right] \theta = \frac{f^2 z + fz^2 - f^2 z - fz^2}{f(z-f)} \theta = 0 \qquad (36).$$

All rays leaving this point strike the focal plane at the optical axis. As shown in Equation (37), the situation when the image sensor is shifted by a distance $\delta$ from the focus is described as follows:

$$\left[ \frac{fz}{z-f} + \delta + z - \frac{\left[\frac{fz}{z-f} + \delta\right]z}{f} \right] \theta = \frac{f^2 z + fz\delta - f^2\delta + fz^2 - f^2 z - fz^2 - \delta z^2 + f\delta z}{f(z-f)} \theta$$

$$= \frac{fz - f^2 - z^2 + fz}{f(z-f)} \delta\theta = -\frac{(z-f)^2}{f(z-f)} \delta\theta$$

$$= \frac{f-z}{f} \delta\theta \qquad (37).$$

Equation (37) shows that by properly positioning the lens of the image sensor with respect to the focal plane, we can change the depth of field. Additionally, the spot size depends upon the magnitude of the angle θ. This angle depends linearly on the aperture of the vision system created by the image sensor.

Additionally or alternatively, in accordance with some embodiments of the present disclosure, an image sensor may be implemented by adjusting for various parameters, including: the distance to the focus as it affects compactness, alignment, and sensitivity of the vision system to the environment; the field of view of the system; and the lens-focal plane separation as it affects the tolerances on alignment of the system and the sensitivity of the system to the environment.

**Embodiments of the flow meter with or without valves connected thereto**

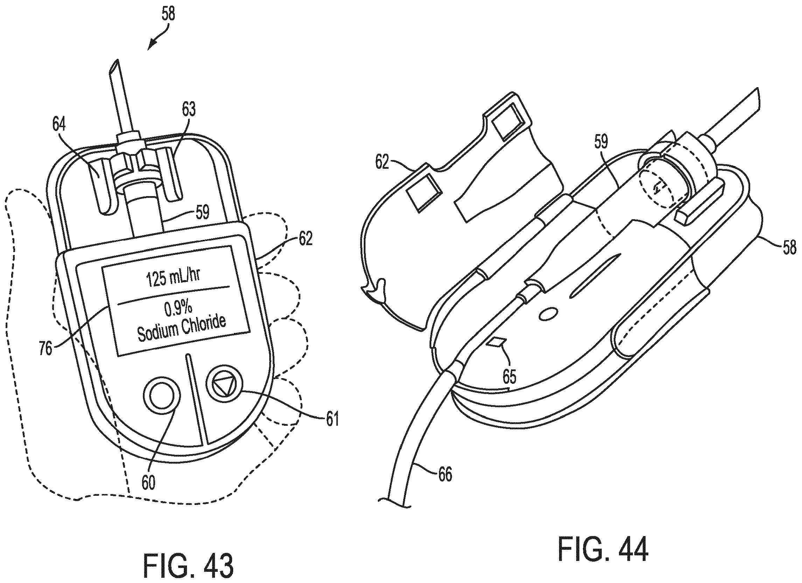

Referring to the drawings, Figs. 43 and 44 show a flow meter 58 coupled to a drip chamber 59. As described *infra,* the flow meter 58 may optionally include a free flow detector component 12 (see Fig. 1) in accordance with an embodiment of the present disclosure. Additionally, alternatively, or optionally, the flow meter 58 may include a flow rate estimator component 13 (see Fig. 1) in accordance with some embodiments of the present disclosure. Fig. 43 shows the flow meter 58 with a shut door 62, and Fig. 44 shows the flow meter 58 with an open door 62. The flow meter 58 may be the flow meter 7 of Fig. 1 with a valve 6 or with no valve. The flow meter 58 includes a start button 60 and a stop button 61. Additionally or optionally, the flow meter 58 may include a backup valve to stop fluid from flowing therethrough or may signal another valve to stop the fluid from flowing in response to error conditions.

The flow meter 58 optionally includes image sensors 63 and 64 that can estimate fluid flow and/or detect free flow conditions. Although the flow meter 58 includes two image sensors (e.g., 63 and 64), only one of the image sensors 63 and 64 may be used in some embodiments. The image sensors 63 and 64 can image a drop while being formed within the drip chamber 59 and estimate its size. The size of the drop may be used to estimate fluid flow through the drip chamber 59. For example, in some embodiments of the present disclosure, the image sensors 63 and 64 use an edge detection algorithm to estimate the outline of the size of a drop formed within the drip chamber 59; a processor therein (see processor 15 of Fig. 1, processor 75 of Fig. 5, or processor 90 of Figs. 6 or 8) may assume the outline is uniform from every angle of the drop and can estimate the drop's size from the outline. In the exemplary embodiment shown in Figs. 43 and 44, the two image sensors 63 and 64 may average together the two outlines to estimate the drop's size. For example, the algorithm may average the measured outlines of the two image sensor 63 and 64 to determine the size of the drop. The image sensors 63 and 64 may use a reference background pattern to facilitate the recognition of the size of the drop as described herein.

In another embodiment of the present disclosure, the image sensors 63 and 64 image the fluid to determine if a free flow condition exists. The image sensors 63 and 64 may use a background pattern to determine if the fluid is freely flowing (i.e., drops are not forming and the fluid streams through the drip chamber 59). As previously mentioned, although the flow meter 58 includes two image sensors (e.g., 63 and 64), only one of the image sensors 64 and 64 may be used in some embodiments to determine if a free flow condition exists and/or to estimate the flow of fluid through the drip chamber.

Additionally or alternatively, in some embodiments of the present disclosure, another image sensor 65 monitors the fluid tube 66 to detect the presence of one or more bubbles within the fluid tube. In alternative embodiments, other bubble detectors may be used in place of the image sensor 65. In yet additional embodiments, no bubble detection is used in the flow meter 58.

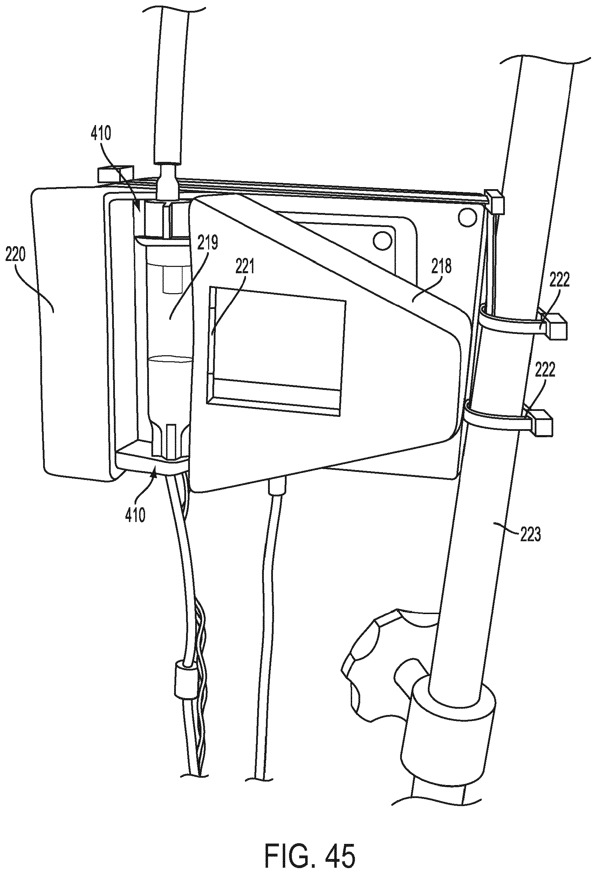

Referring now to the drawings, Fig. 45 shows a flow meter 218 coupled to a drip chamber 219 in accordance with an embodiment of the present disclosure. The drip chamber 219 is secured to the flow meter 218 via couplers 410. A backlight 220 shines light through the drip chamber toward the image sensor 221 (shown in outlined form).

The flow meter 218 may electronically transmit a flow rate to a monitoring client 8 (see Fig. 1). Additionally or alternatively, in some optional embodiments, the flow meter 218 may include a display that displays a flow rate (e.g., a touch screen, LED display, and the like). The flow meter 218 may be coupled to a pole 223 via clamps 222.

In some embodiments, the flow meter 218 may be coupled to an actuator which is coupled to a valve (not shown in Fig. 45) to form a closed-loop system (e.g., control component 14 of Fig. 1, such as a PID, bang-bang, neural network, or fuzzy logic control system) to regulate the flow of fluid through the drip chamber 219.

The flow meter 218 may use any flow algorithm described herein and may include any imaging system described herein. Additionally or alternatively, the flow meter 218 may include a free flow detector component (e.g., the free flow detector component 12 of Fig. 1).

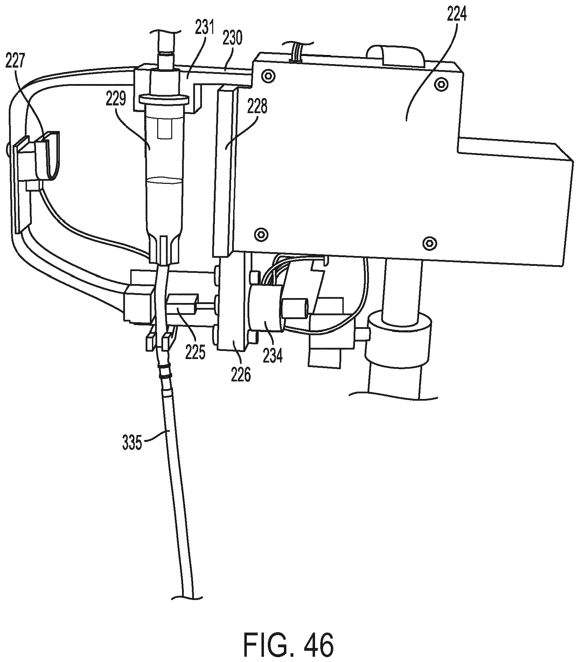

Fig. 46 shows a flow meter 224 and a pinch valve 225 coupled to the body 226 of the flow meter 224 to control the flow of fluid to a patient in accordance with an embodiment of the present disclosure. The flow meter 224 includes an image sensor 227 and a backlight 228.

The image sensor 227 images a drip chamber 229 and can receive illumination from the backlight 228. The flow meter 224 includes a support member 230 coupled to a coupler 231 that couples the drip chamber 229 to the flow meter 224.

The flow meter 224 may implement any flow rate estimator described herein (e.g., the flow rate estimator component 13 of Fig. 1) and/or a free flow detector disclosed herein (e.g., the free flow detector component 12 of Fig. 1). The flow meter 224 may use the pinch valve 225 in a close-loop fashion to control the flow of fluid to a patient (e.g., using a control component 14 as shown in Fig. 1).

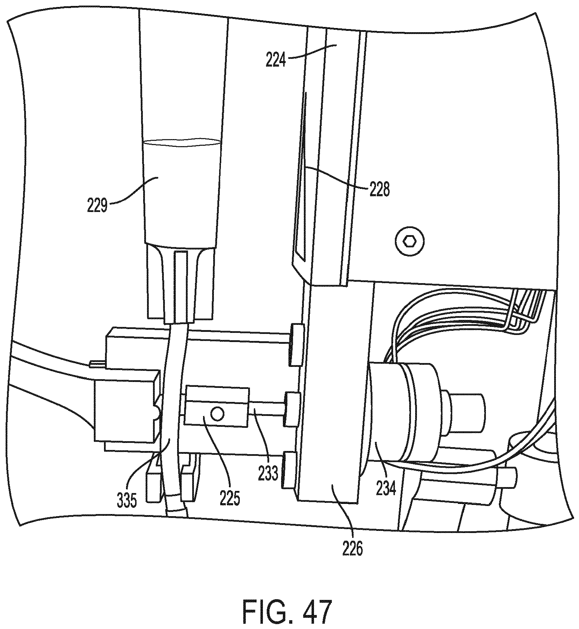

The pinch valve 225, as is more easily seen in Fig. 47, is coupled to a shaft 233 which is coupled to an actuator 234. The actuator 234 may be a solenoid or any actuator that can move the pinch valve 225 toward a tube 335.

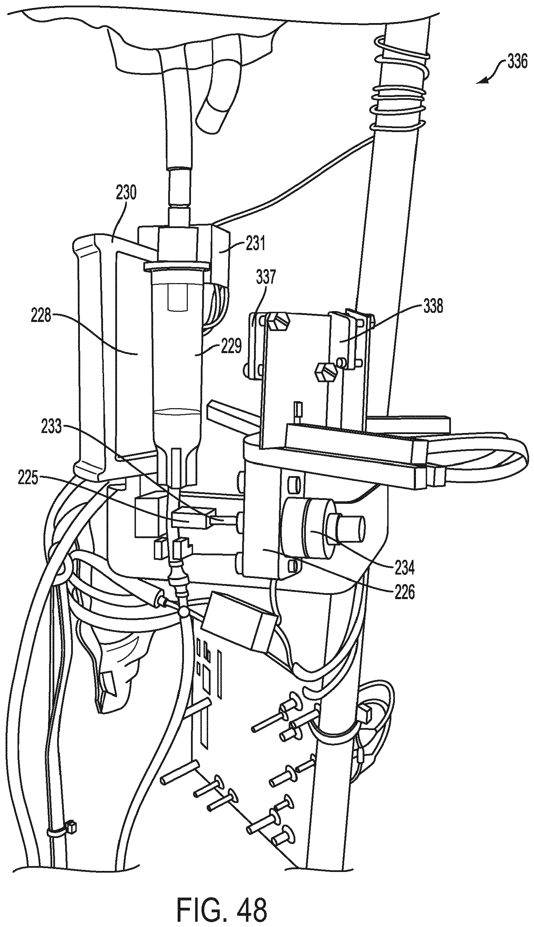

Fig. 48 shows a flow meter 336 and a pinch valve 225 in accordance with an embodiment of the present disclosure. The flow meter includes two image sensors 337 and 338. The flow meter 336 may use the pinch valve 225 in a closed-loop feedback configuration. The flow meter 336 may implement a volume estimation algorithm described herein using both image sensors 337 and 338 to estimate the flow of fluid through the drip chamber 229. For example, the flow meter 336 may average the two volumes together for use in the feedback loop.

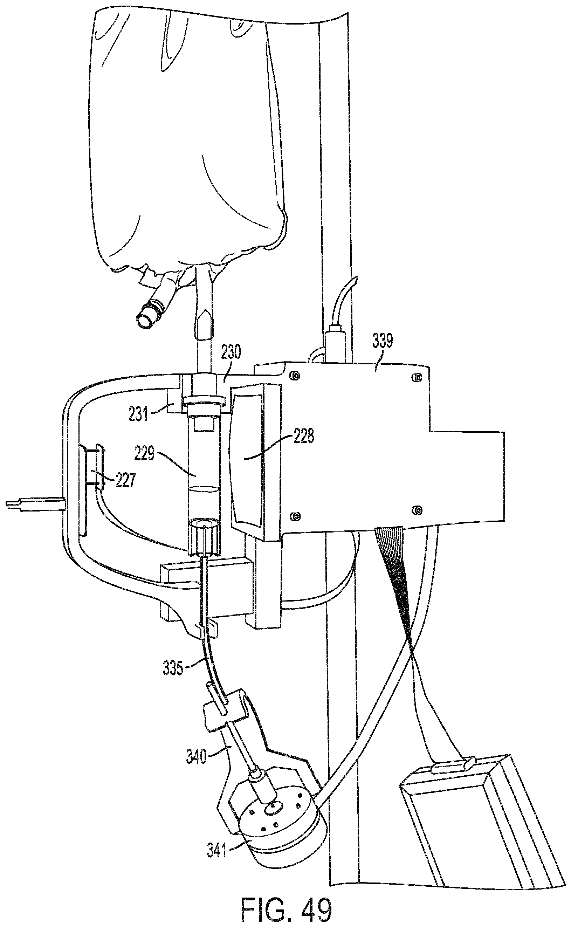

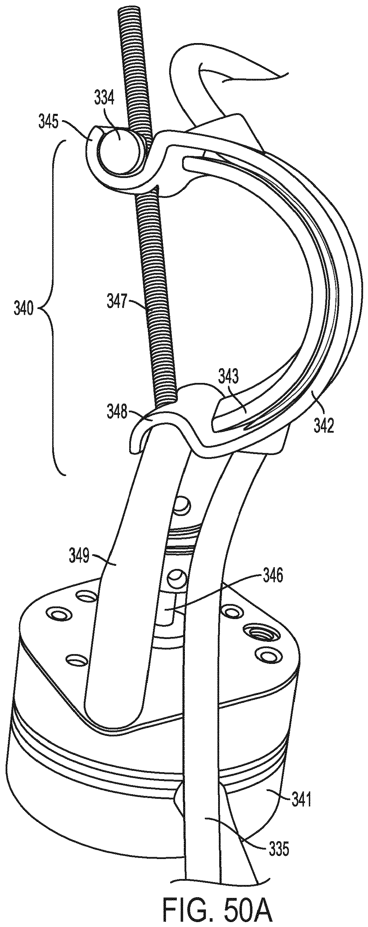

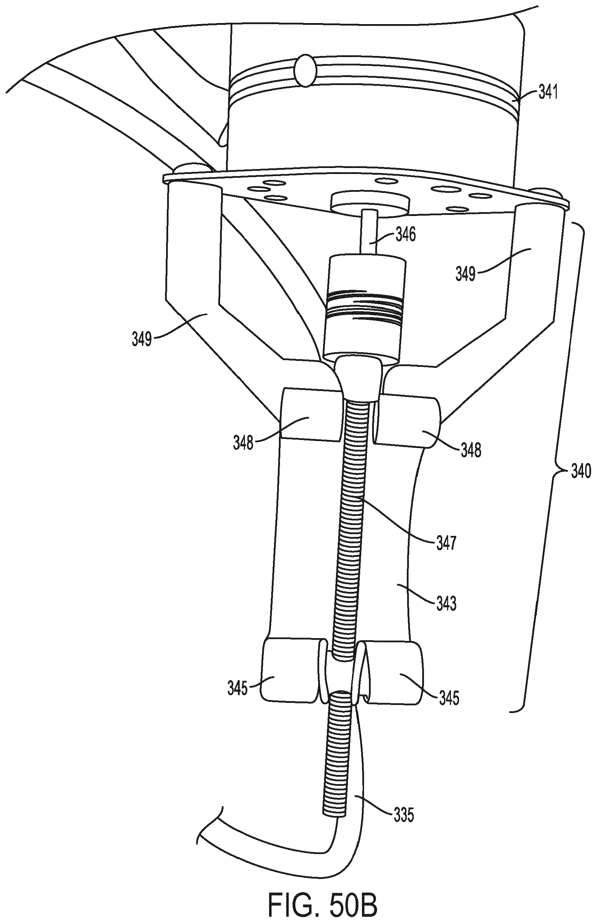

Fig. 49 shows a flow meter 339 and a valve 340 coupled to an actuator 341 to control the flow of fluid into a patient in accordance with an embodiment of the present disclosure. The flow meter 339 of Fig. 49 is similar to the flow meter 224 of Fig. 46; however, the flow meter 339 of Fig. 49 includes a valve 340 that has curved, elongated support members 342 and 343 (see Figs. 50A-50B).

The flow meter 339 includes an image sensor 227 and a backlight 228. The image sensor 227 images a drip chamber 229 and can receive illumination from the backlight 228. The flow meter 339 includes a support member 230 coupled to a coupler 231 that couples the drip chamber 229 to the flow meter 339.

The flow meter 339 can implement any flow rate estimator described herein (e.g., the flow rate estimator component 13 of Fig. 1) and/or a free flow detector disclosed herein (e.g., the free flow detector component 12 of Fig. 1). The flow meter 339 may use the valve 340 in a close-loop fashion to control the flow of fluid into a patient (e.g., using the control component 14 of Fig. 1).

The flow meter 339 may actuate the actuator 341 to actuate the valve 340, which thereby regulates the fluid flowing through the IV tube 335 in a feedback (i.e., closed-loop) configuration using any control algorithm.

Referring now to Figs. 50A-50B, which shows close-up views of the valve 340 of Fig. 49 in accordance with an embodiment of the present disclosure. The valve 340 includes an inner curved, elongated support member 343 and an outer curved, elongated support member 342. The tube 335 is positioned between the support members 342 and 343.

The inner support member 343 includes a barrel nut 344. The outer support member 342 is coupled to the barrel nut 344 via hooks 345. In some embodiments, the barrel nut 344 is not coupled to the valve 340 and the inner support member 342 includes a hole for the threaded rod or screw 347 to slide through. The outer support member 342 also has hooks 348 to secure it to a frame 349 of the actuator 341. The actuator 341 includes a shaft 346 coupled to a screw 347. As the actuator 341 rotates the shaft 346, the screw 347 can rotate to push the barrel nut 334 toward the actuator 341. That is, the hooks 345 and the barrel nut 334 moves toward the hooks 348 and the frame 349 because the inner and outer support members 342 and 343 are flexible.

As the support members 342 and 343 are compressed, the tube 335 becomes compressed because it is positioned between the support members 342 and 343. Compression of the tube 335 restricts the flow of fluid through the tube 335. The valve 340 compresses a length of the tube 335 that is substantially greater than the diameter of the tube 335.

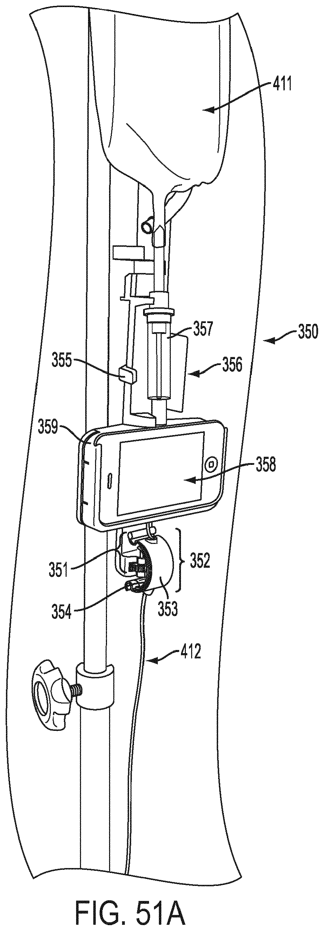

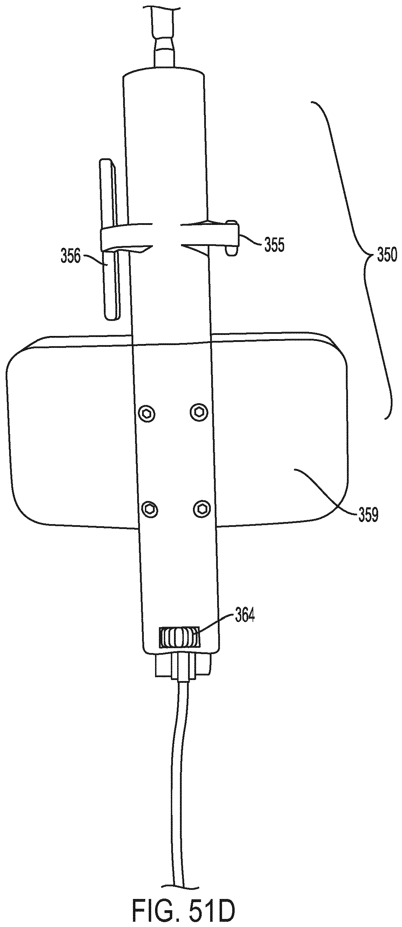

Figs. 51A-51D show several views of a flow meter 350 with a monitoring client 358, a valve 352, a drip chamber 357, an IV bag 411, and a fluid tube 412 in accordance with an embodiment of the present disclosure. The flow meter 350 includes a receiving portion 351 to receive the valve 352. The valve 352 includes two curved, elongated support members 353 and 354.

The flow meter 350 includes an image sensor 355 and a backlight 356 that can monitor drops formed within the drip chamber 357. The flow meter 350 may use the image sensor 355 to implement a flow rate estimator algorithm described herein (e.g., the flow rate estimator component 13 of Fig. 1) and/or to implement a free flow detector disclosed herein (e.g., the free flow detector component 12 of Fig. 1).

The flow meter 350 includes a base 359 that can form a dock to receive the monitoring client 358. The monitoring client 358 may be a smart phone, or other electronic computing device (e.g., an Android-based device, an Iphone, a tablet, a PDA, and the like).

The monitoring client 358 may contain software therein to implement a free flow detector, a flow rate estimator, a control component, an exposure component, etc. (e.g., the free flow detector component 12, the flow rate estimator component 13, the control component 14, the exposure component 29 of Fig. 1) and may contain one or more transceivers (e.g., the transceiver 9). Additionally or alternatively, a base 359 of the flow meter 350 may implement these items.

For example, the flow meter 350 may implement a free flow detector, a flow rate estimator, a control component, an exposure component, etc. using internal software, hardware, electronics, and the like. The flow meter 350 may implement a closed-loop feedback system to regulate the fluid flowing to a patient by varying the fluid flowing through the valve 352.

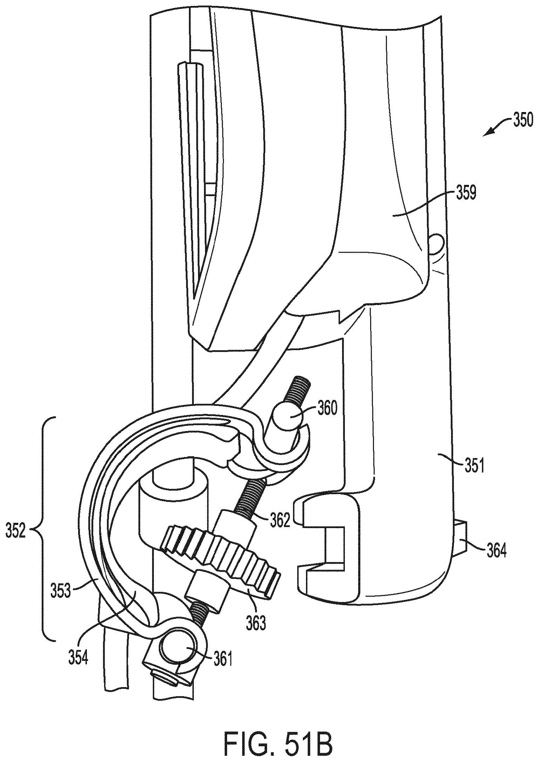

As is easily seen in Fig. 51B, the valve 352 includes an inner support member 354 and an outer support member 353. The inner support member 354 is coupled to a barrel nut 360 and to a barrel 361. In some embodiments, the barrel nut 360 is not coupled to the inner support member 354 and the inner support member 354 includes a hole for the threaded shaft 362 to slide through.

A threaded shaft 362 (e.g., a screw) spins freely within a bearing located within the barrel 361 and engages a threaded nut within the barrel nut 360 to push or pull the barrel nut 360 relative to the barrel 361 by rotation of the knob 363 (e.g., the actuator is a lead screw having a knob to actuate the lead screw.). The knob 363 may be manually rotated.

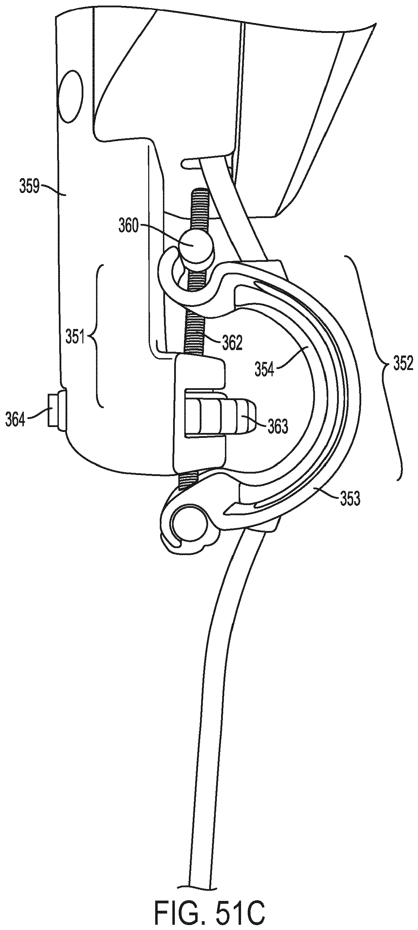

Additionally or alternatively, the valve 352 may be snapped into the receiving portion 351 which includes a rotating member 364 that engages the knob 363 within the receiving portion 351 (see Fig. 51C). The rotating member 364 engages the rotating knob 363 to actuate the valve 352. The rotating member 364 may be coupled to an electric motor which rotates the rotating member 364. The electric motor (not explicitly shown) may be controlled by the flow meter 350 in a closed-loop configuration to achieve a target flow rate of fluid flowing into a patient.

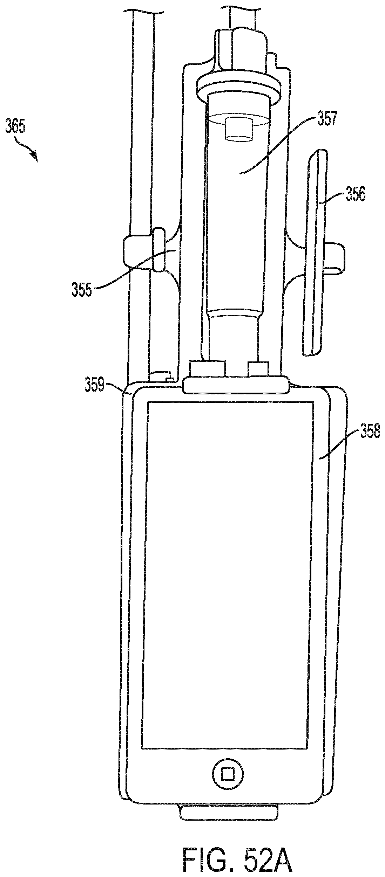

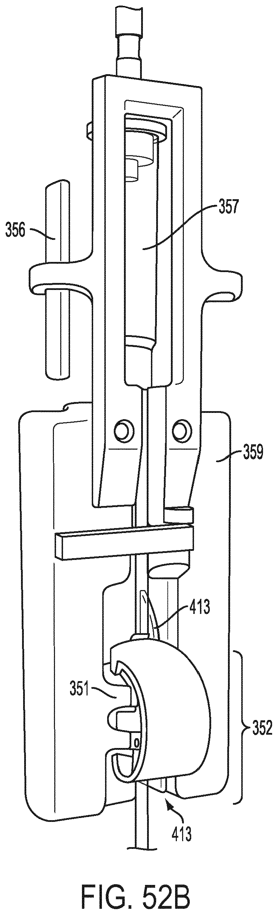

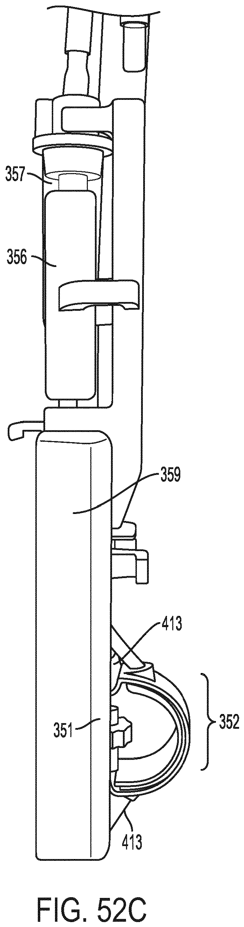

Figs. 52A-52D show several views of another flow meter 365 with a valve 352, a drip chamber 357, and a fluid tube trench 413 having a receiving portion 351 to receive a valve 352 in accordance with an embodiment of the present disclosure. The flow meter 365 of Figs. 52A-52D is similar to the flow meter 350 of Figs. 51A-51D; however, the base 359 holds the monitoring client 358 in an "upright" position. Additionally, the receiving portion 351 is on an opposite side of the base 359 from the monitoring client 358 (see Figs. 52B and 52C).

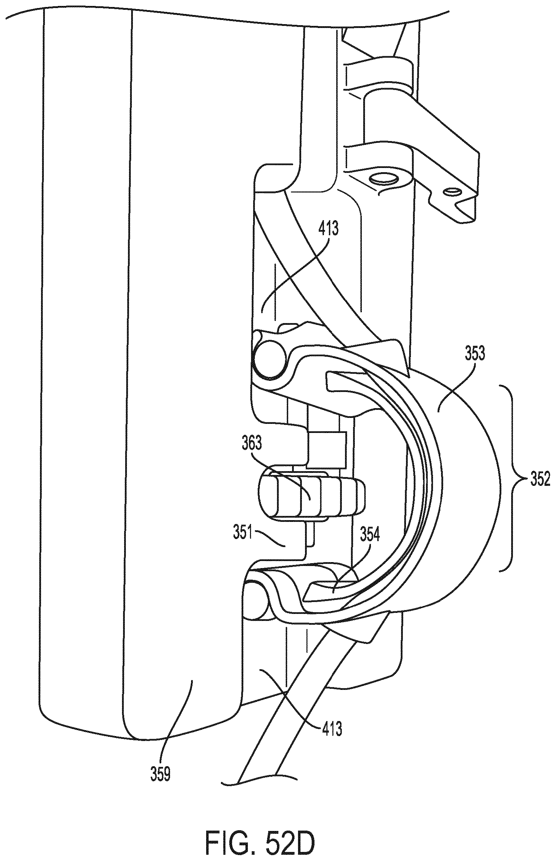

Fig. 52D shows a close-up view of the valve 352 engaging the receiving portion 351. The knob 363 engages a rotating member that is internal to the base 359 (not shown in Fig. 52D) that is coupled to a motor (also not shown in Fig. 52D).

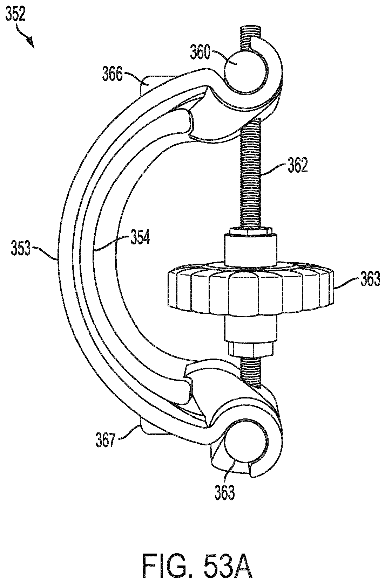

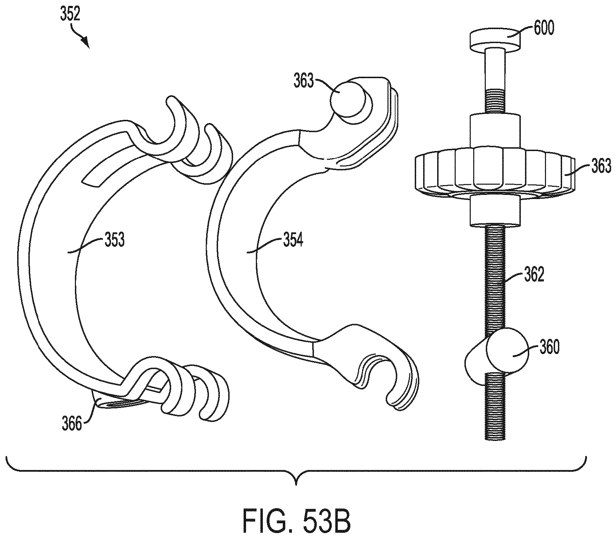

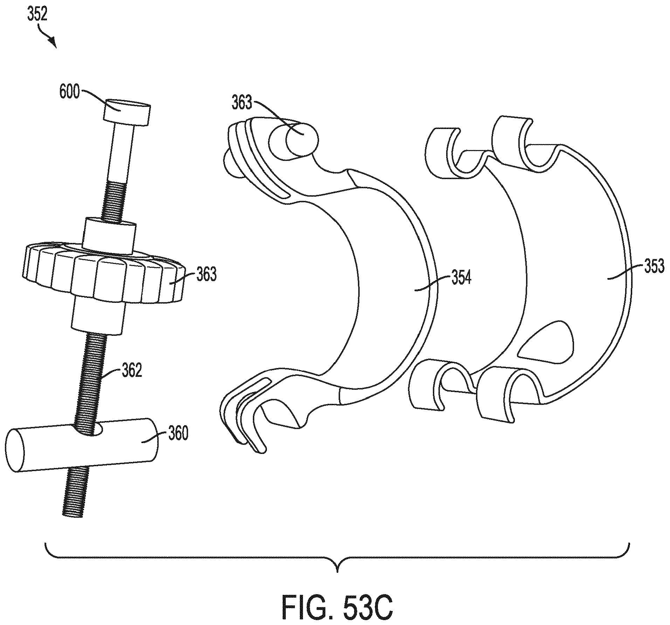

Fig. 53A shows another view of the valve 352 of Figs. 51A-51D and 52A-52D, and Figs. 53B-53C show two exploded views of the valve of Fig. 53A in accordance with an embodiment of the present disclosure.

As shown in Figs. 53A-53C, the valve 352 includes an inner support member 354 and outer support member 353. A tube may be inserted through holes 366 and 367 to position the tube between the support members 354 and 353.

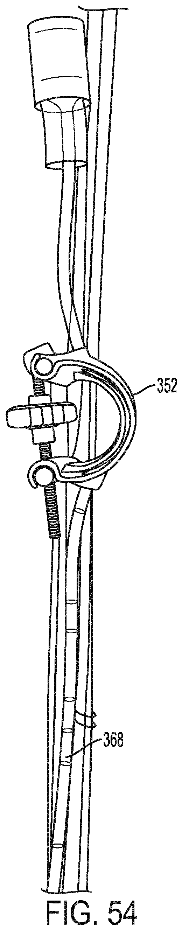

The knob 363 may be turned to turn the screw 362. Rotation of the screw 362 causes the barrel nut 360 to move toward the partial barrel 363 to compress a tube positioned between the support members 353 and 354. The partial barrel 363 includes two sides, however, there is a space to hold the end 600 (e.g., the cap) of the screw 362 securely within the space (e.g., a complementary space). Fig. 54 shows the valve 352 in manual use and coupled to a tube 368.

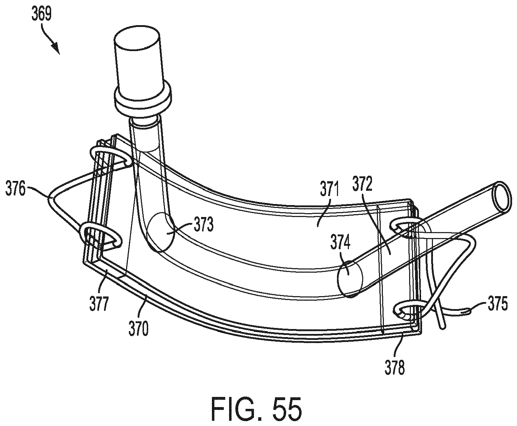

Fig. 55 shows a valve 369 that includes two flexible members 370 and 371 in accordance with an embodiment of the present disclosure. The flexible members 370 and 371 may be two flexible sheets. The flexible member 371 may include holes 373 and 374 for a tube 372 to be positioned between the flexible members 370 and 371.

The flexible members 370 and 371 are coupled together via two connector members 377 and 378. The connector members 377 and 378 are coupled to coupling members 376 and 375, respectively.

Actuation of the valve 369 may be by a linear actuator that pulls the coupling members 377, 378 toward each other or away from each other. The linear actuator (not explicitly shown) may be a screw-type actuator, a piston actuator, or other actuator. In some embodiments, one of the coupling members 375 and 376 may be coupled to a stationary support while the actuator is coupled to the other one of the coupling members 375 and 376 and another stationary support for pulling the coupling members 375 and 376 together or apart.

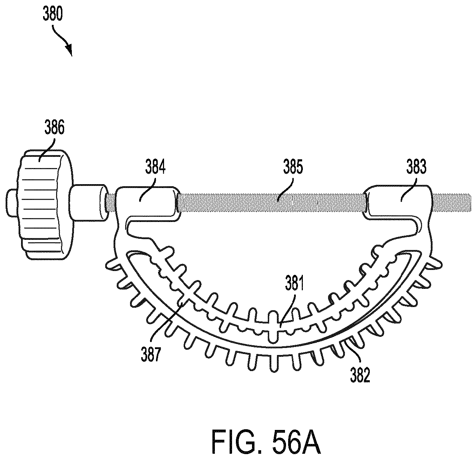

Figs. 56A-56C show several views of a valve 380 having two curved, elongated support members 381 and 382 with one of the elongated support members 381 having a plurality of ridges 387 adapted to engage a tube positioned between the support members 381 and 382, in accordance with an embodiment of the present disclosure.

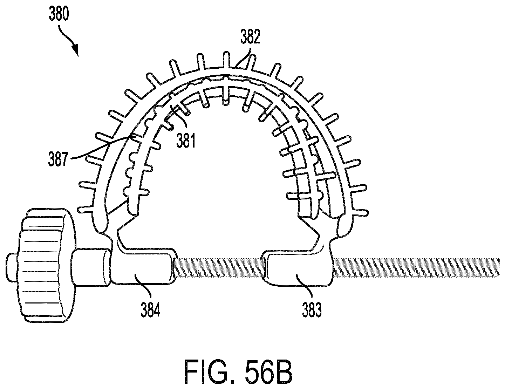

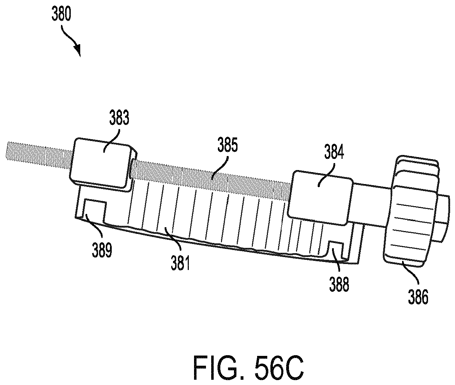

The valve 380 has both support members 381 and 382 coupled to a coupling member 383 at a first end and a second coupling member 384 at another end. That is, the coupling member 384 surrounds a screw 385 and the coupling member 383 includes internal threads for pulling the coupling member 383 toward or away from a knob 386 when the screw 385 is rotated with rotation of the knob 386. Fig. 56B shows the valve 380 when actuated to close fluid flowing through a tube coupled between the support members 381 and 382. Fig. 56C shows the support member 381 having two holes 388 and 389 to receive a tube. Also note that the support members 381 and 382 hold a tube off center from an axis of the screw 385, which is easily seen in Fig. 56C. Holding the tube off-center from the screw's 385 axis facilitates free movement of the tube.

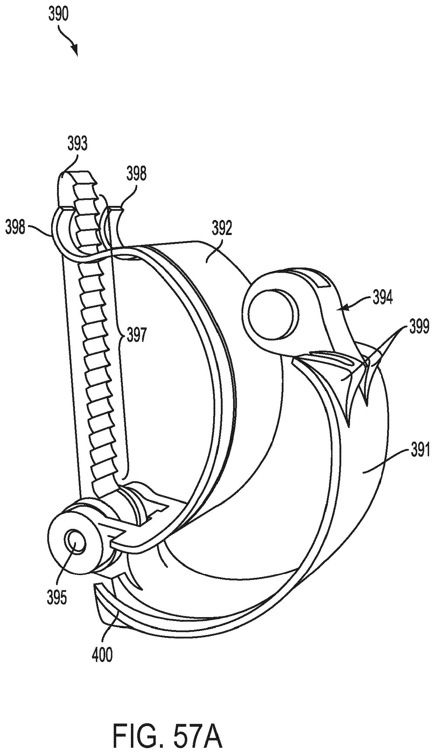

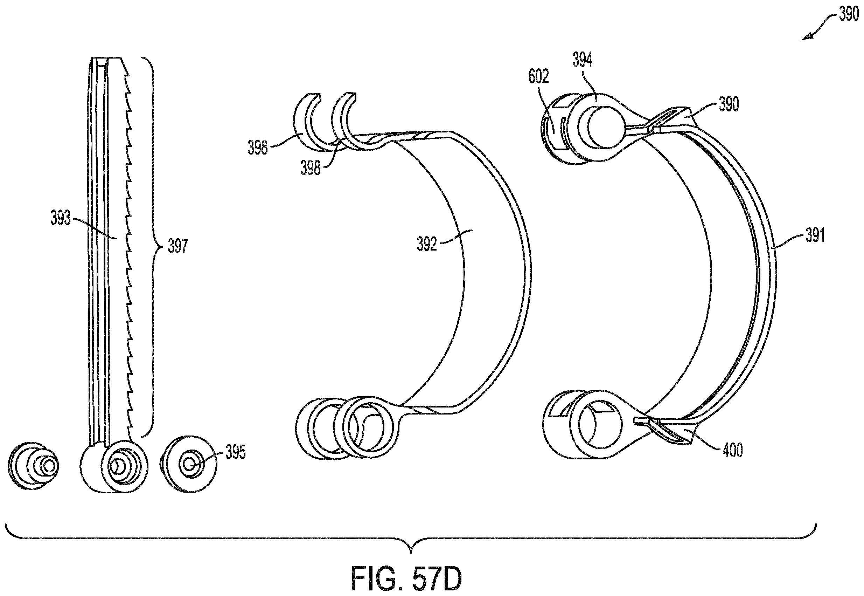

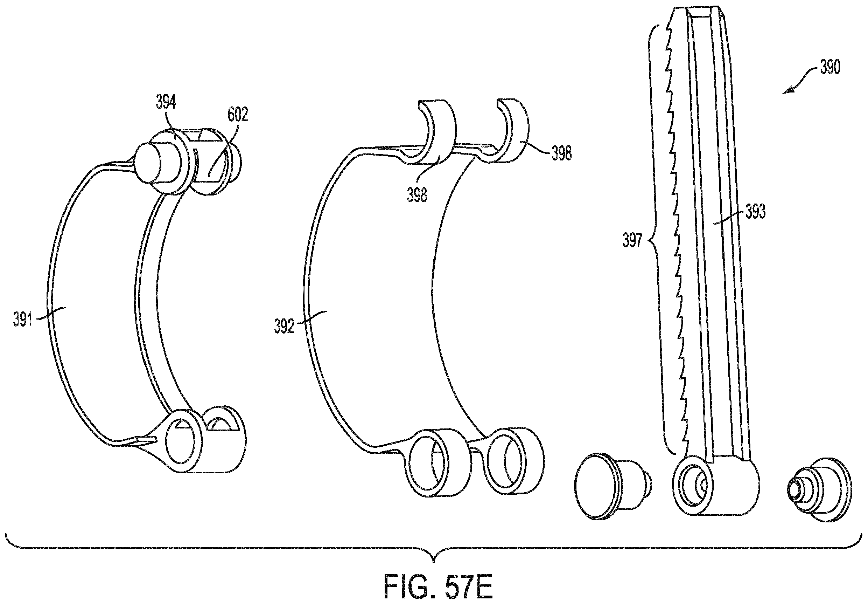

Figs. 57A-57C show several views of a valve 390 having a ratchet 394 that engages a connecting member 393 of the valve 390 in accordance with an embodiment of the present disclosure and Figs. 57D-57E show two exploded views of the valve 390 of Figs. 57A-57C. The ratchet 394 engages the connecting member 393 by interacting with a gear rack 397 disposed thereon. A finger 602 (see Figs. 57D and 57E) interacts with a gear rack 397 to provide the ratcheting action. That is, the finger 602 may hold the gear rack 397 against an engaging finger on a side opposite of the retaining finger 602. The valve 390 includes a support member 391 having an end coupled to the ratchet 394 and another end pivotally coupled to a hinge 395. The valve 390 also includes a support member 392 having hooks 398 that can couple to the body of the ratchet 394.

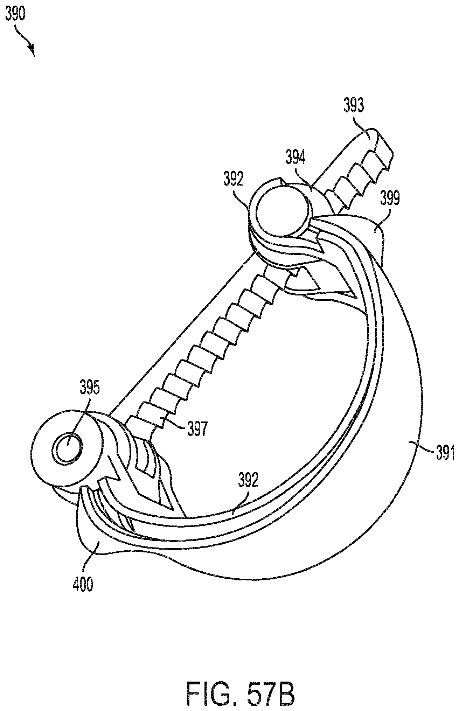

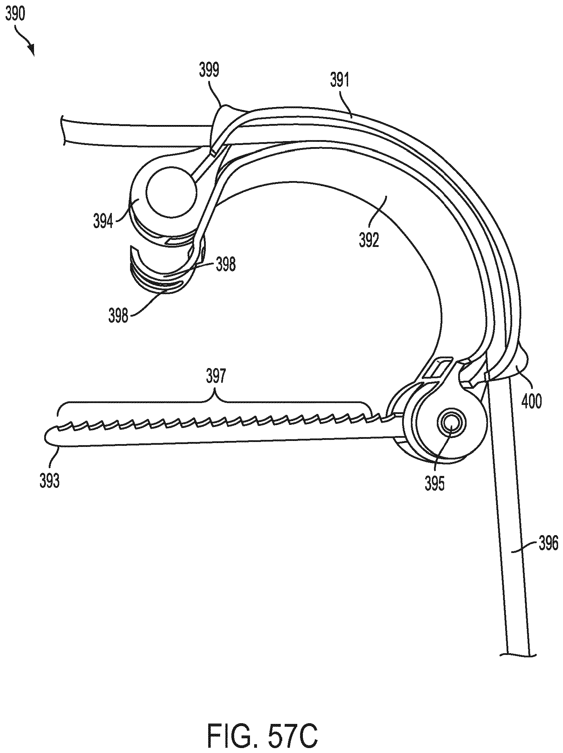

As shown in Fig. 57C, a tube 396 can be positioned between the support members 391 and 392, the hooks 398 can then be fastened to the body of the ratchet 394, and the connecting member 393 can be inserted into the ratchet 394 (as shown in Fig. 57B). As shown in Fig. 57C, the tube 396 is positioned against the support member 391 via openings 399 and 400.

The ratchet 394 engages the gear rack 397 such that the ratchet 394 can be manually moved toward the hinge 395 for course fluid flow adjustments. Thereafter, a knob (not shown) may be coupled to the ratchet 394 to make fine adjustments to the distance between the ratchet 394 and the hinge 395. Additionally or alternatively, the ratchet 394 may include a release button (not shown) to release the ratchet from the connecting member 393.

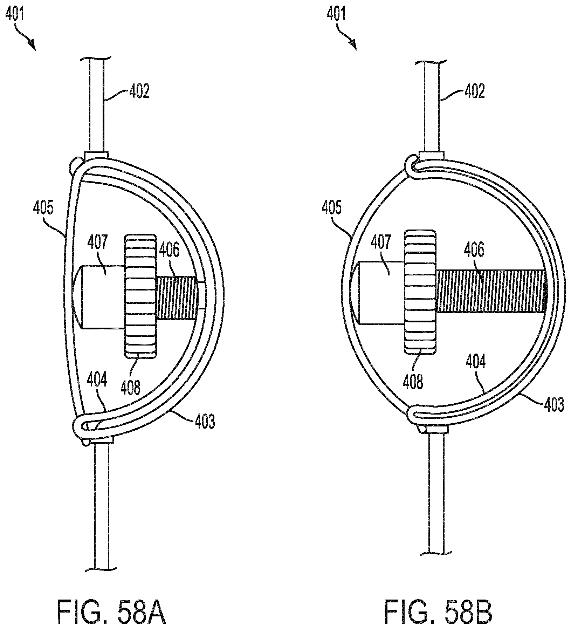

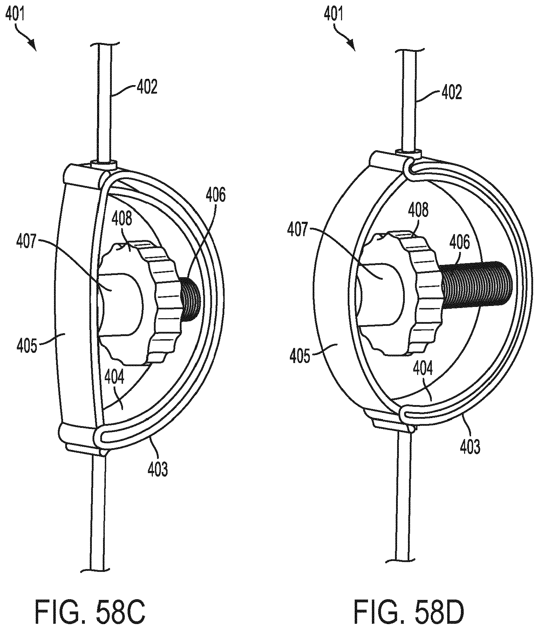

Figs. 58A-58D show several views of a valve 401 having two elongated support members 403 and 404, a connecting member 405, and a screw-type actuator 407 in accordance with another embodiment of the present disclosure.

The support members 403 and 404 may be permanently molded together at their ends with the ends of the connecting member 405. A tube 402 may be positioned between the support members 403 and 404.

As the knob 408 is turned, the screw-type actuator 407 expands or contracts because of engagement with a threaded rod 406. Fig. 58A shows the valve in an open position while Fig. 58B shows the valve in a closed position. Note that the tube 402 is squeezed along a substantial length of the tube 402. Figs. 58C-58D show the valve 401 in the open position and the closed position, respectively, from a perspective view.

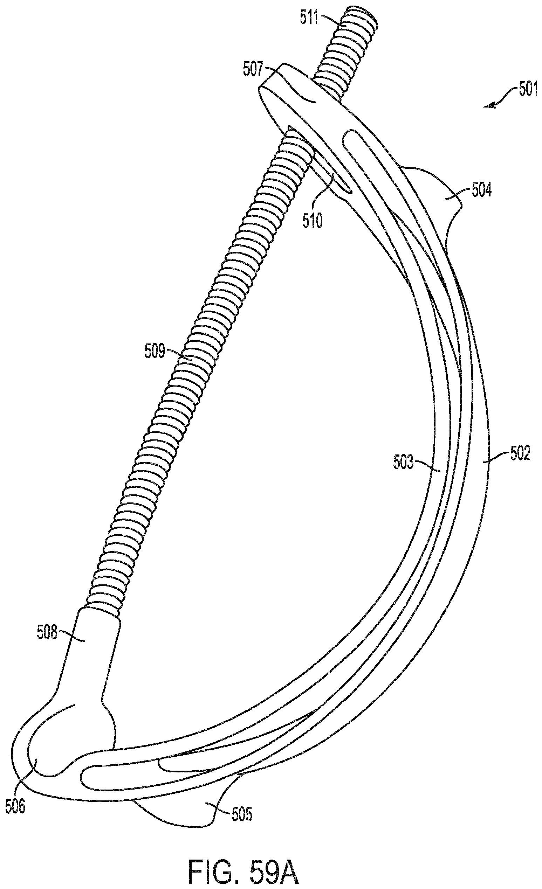

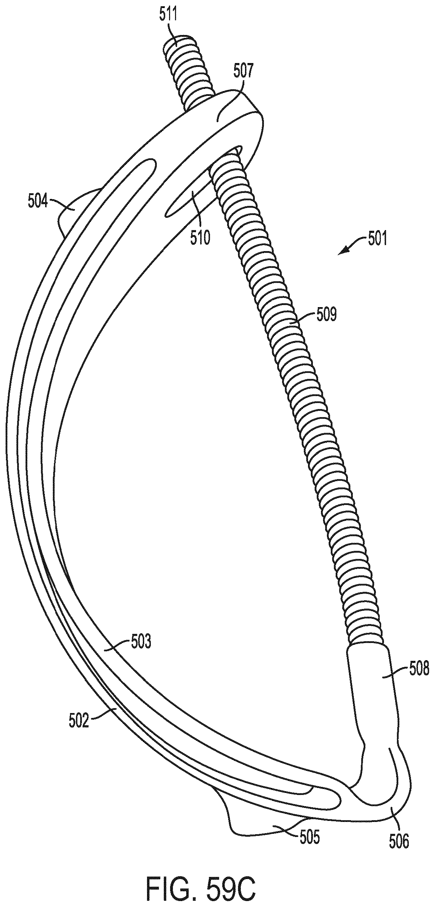

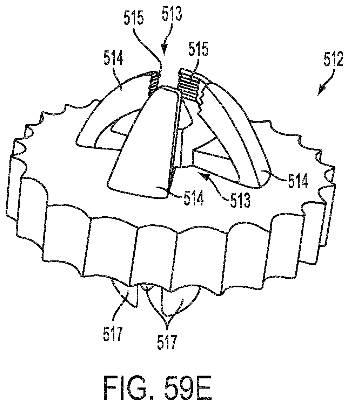

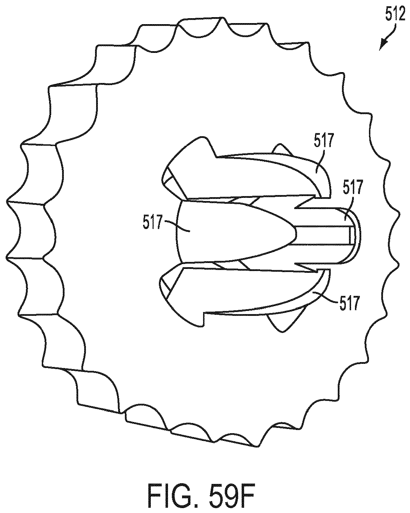

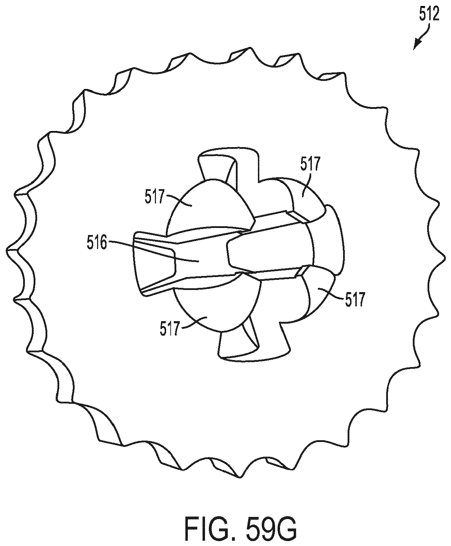

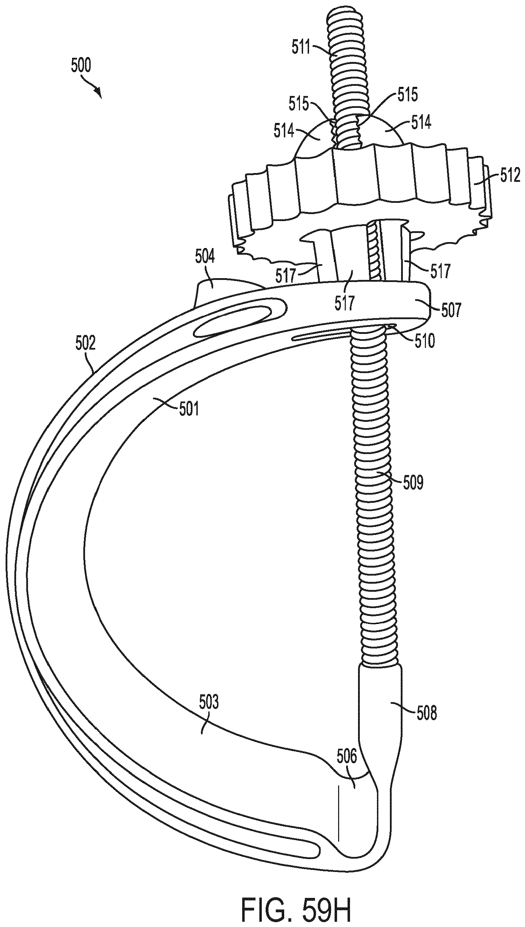

Figs. 59A-59C show several views of a body 501 of a valve 500 (see Fig. 59H for the assembled valve 500) in accordance with an embodiment of the present disclosure. The body 501 includes a first curved, elongated support member 502 and a second curved, elongated support member 503. The first support member 502 includes raised holes 504, 505 to hold a tube between the support members 502 and 503.

The body 501 also includes a first connector 506 that is coupled to the support members 503, 504 at an end, and a second connector 507 that is coupled to the other ends of the support members 503, 504.

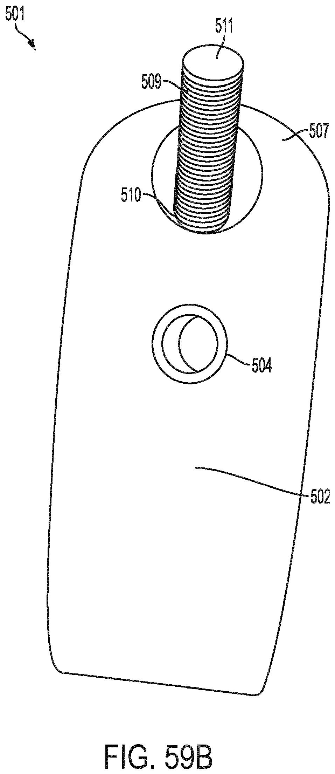

The first connector 506 is coupled to an end of the support members 503, 504 and to a first end 508 of a connecting member 509. The second connector 507 includes a hole 510 for positioning the second end 511 of the connector member 509 therethrough (as is easily seen in Fig. 59B).

When a tube is positioned between the support members 502, 503, movement of the second connector 507 toward the first connector 506 compresses the tube disposed between the support members 502, 503. As the second connector 507 moves towards the first connector, the hole 510 of the second connector 507 allows the second end 511 of the connector member 509 to freely slide therein.

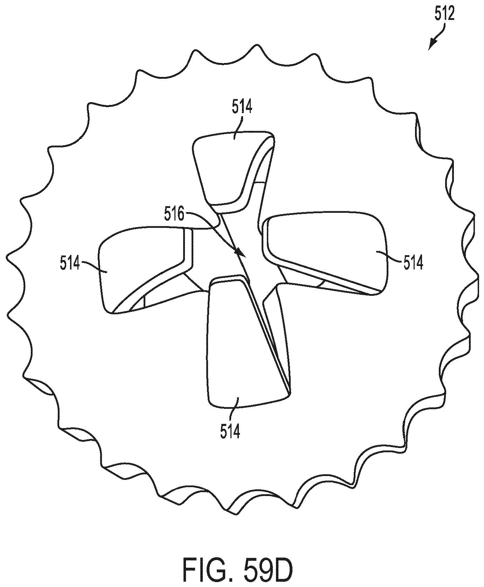

Figs. 59D-59G show several views of a knob 512 for use with the body 501 shown in Figs. 59A-59C in accordance with an embodiment of the present disclosure. The knob 512 includes a ratchet 513 defined by four fingers 514. Each of the fingers 514 includes a threaded surface 515 to engage a threaded connecting member 509. The fingers 514 are arched toward a hole 516 at the center of the knob 512. The knob 512 also includes fingers 517 that engage the second connector 507 (see Fig. 59H). In some embodiments, the body 501 includes a recess 510 to receive the fingers 517 on the second connector 508.

Fig. 59H shows an assembly valve 500 that includes the body 501 shown in Figs. 59A-59C coupled to the knob 512 of Figs. 59D-59G in accordance with an embodiment of the present disclosure. The knob 512 is slid onto the threads of the connecting member 509. The fingers 514 engage the threads of the connecting member 509 and ratchet onto the connecting member 509. That is, the knob 512 is freely moveable towards the first end 508 of the connecting member 509 along the threads of the connecting member 509, but cannot be moved away from the first end 508 of the connecting member 509 without rotating the knob 512. That is, the knob 512 may be place onto the connecting member 509 to provide a coarse adjustment of the valve 500 by coarsely moving the connectors 507, 508 toward each other to close the valve 500. Because the threaded surfaces 515 of the four fingers 514 engage the threads of the connecting member 509, rotation of the knob 512 either reduces or increases fluid flow within a tube. Each of the fingers 514 includes a threaded surface 515 to engage the threads of the connecting member 509 such that rotation of the knob 512 moves the second connector 507 toward or away from the first connector 506 to thereby control the flow of fluid of a tube positioned between the support members 502, 503.

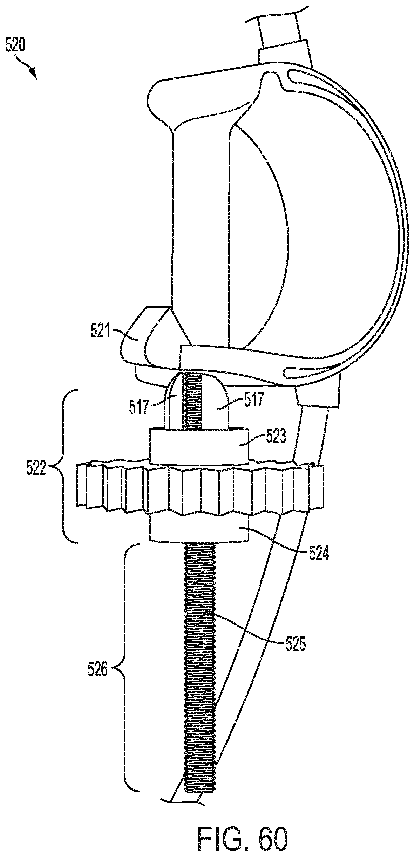

Fig. 60 shows a valve 520 having a guiding protrusion 521 in accordance with an embodiment of the present disclosure. The valve 520 is similar to the valve 500 of Fig. 59H, but includes the guiding protrusion 521 and a knob 522 having first and second collars 523, 524. The knob 522 also includes internal threads (not shown) to engage threads 525 of a connecting rod 526. In some embodiments, the internal threads may be ratcheting and in other embodiments, the internal threads may be fixed without providing a ratcheting action.

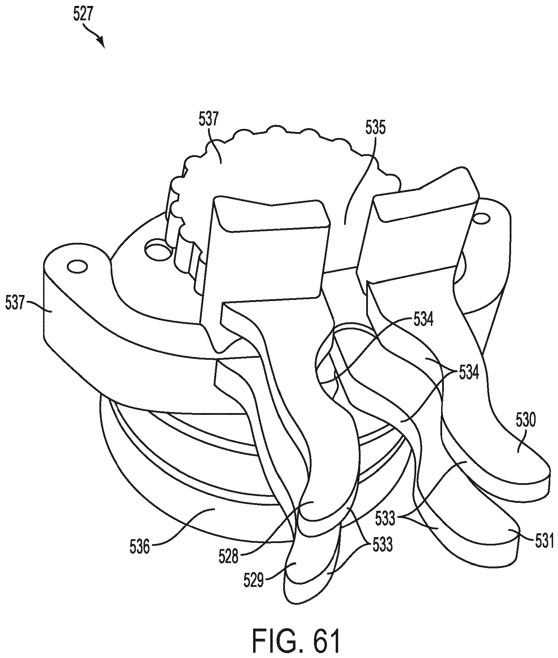

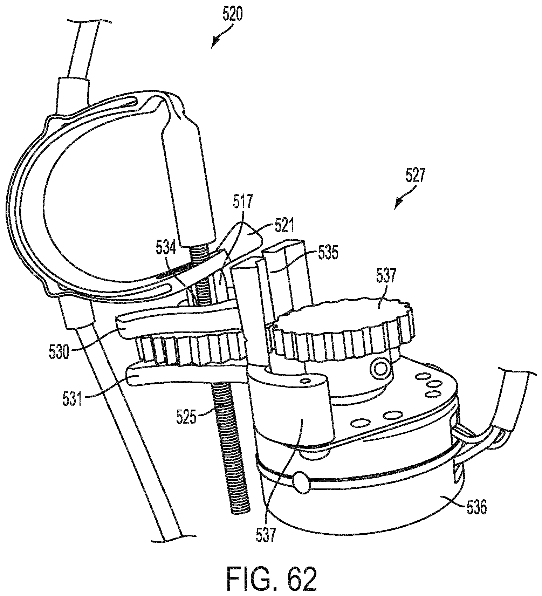

Fig. 61 shows a motor 536 and valve-securing structure 537 for coupling to the valve 520 of Fig. 60 in accordance with an embodiment of the present disclosure. The valve-securing structure 537 includes securing fingers 528, 529, 530, 531 each having a curved portion 533 for snapping onto collars 523, 524 of a knob 522 (see Fig. 62) into respective collar-guiding portions 534.

Referring now to Figs. 60, 61, and 62 once the collars 523, 524 are sufficiently secured, the knob 522 is free to rotate. That is, the collar 523 may be secured between the securing fingers 528 and 530 within their respective collar-guiding portion 534 allowing the knob 522 to rotate. Likewise, the collar 524 may be secured between the securing fingers 529 and 531 within their respective collar-guiding portion 534 allowing the knob 522 to rotate.

When the valve 520 is secured to the valve-securing structure 537, rotation of the wheel 537 (caused by the motor 536) rotates the knob 522 of the valve 520. As the valve 520 flexes, the protrusion 521 freely moves within the protrusion guide 535 or adjacent to the protrusion guide 535. Fig. 62 shows the valve of Fig. 60 secured to the motor 536 via the valve-securing structure 537.

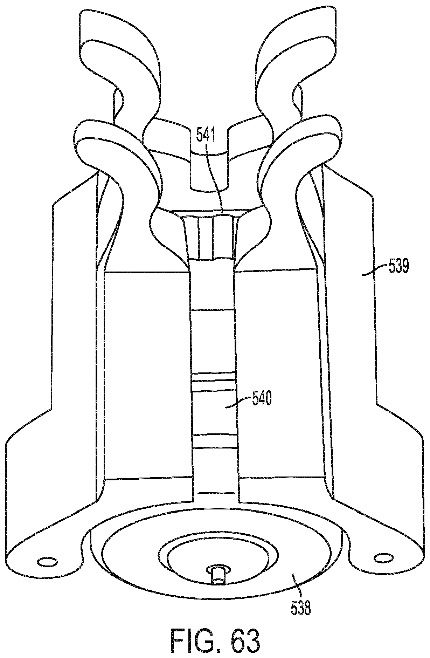

Fig. 63 shows another motor 538 and valve-securing structure 539 for coupling to the valve of Fig. 60 in accordance with an embodiment of the present disclosure. The valve-securing structure 539 includes a protrusion guide 540 adjacent to the motor 538. The motor 538 is coupled to the wheel 541 to engage the knob 522 (see Fig. 60).

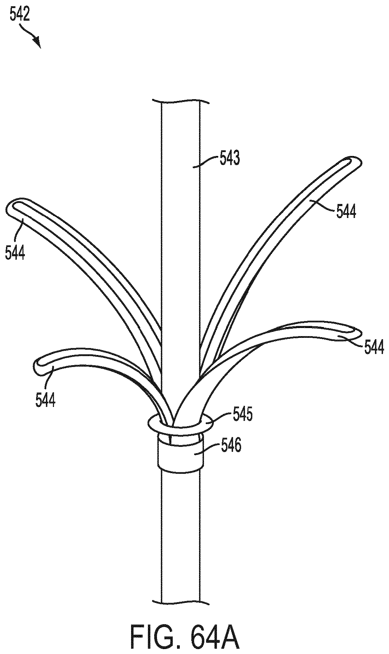

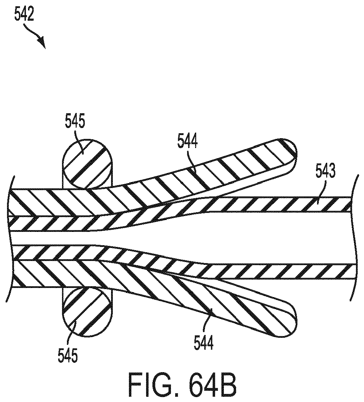

Fig. 64A shows a valve 542 having a slidable collar 545 and several compressing fingers 544 for regulating fluid flow through a fluid line 543 in accordance with an embodiment of the present disclosure. The base 546 is connected to all of the fingers 544. As the slidable collar 545 is moved over the compressing fingers 544, the compressing fingers 544 compress the tube 543 to impede fluid flow therewithin. Fig. 64B shows a cross-sectional view of the valve of Fig. 64A.

Fig. 64A shows a valve 542 having a slidable collar 545 and several fingers 544 for regulating fluid flow through a fluid line 543 in accordance with an embodiment of the present disclosure. The fingers 544 are coupled to a base 546 such that the base 546 and fingers 544 surround the tube 543. The collar 545 is slidable away from the base 546 such that the fingers 544 compress the tube 543 which thereby reduces an internal volume of the tube 543. The reduction of the internal volume of the tube 543 reduces the fluid flow through the tube. An actuator (not shown) may be coupled to the collar 545 to control the position of the collar 545 (e.g., a linear actuator may be coupled to the collar 545 and to the base 546). Fig. 64B shows a cross-sectional view of the valve 542 of Fig. 64A. Note that the fingers 544 may be shaped away from the tube near an opposite end of the base

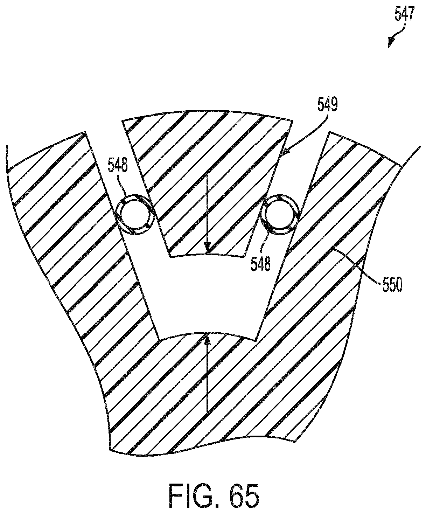

Fig. 65 shows a valve 547 having two curved surfaces 549 and 550 for positioning a fluid tube 548 therebetween to regulate fluid flow through the fluid tube 548 in accordance with an embodiment of the present disclosure. As the surface are compressed together, the tube 548 is compressed therebetween. The two curved surfaces 549 and 550 may be compressed together using an actuator. The tube 548 may be wrapped several times around the surface 549.

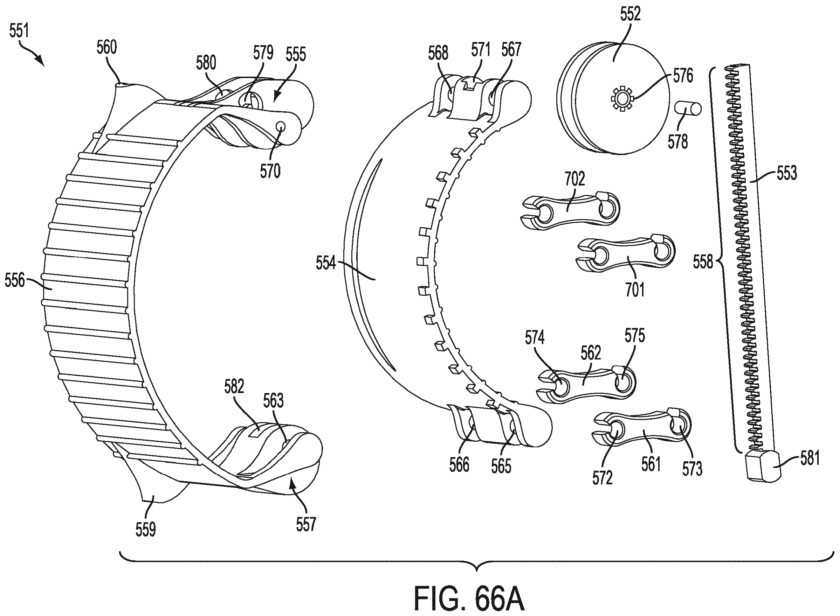

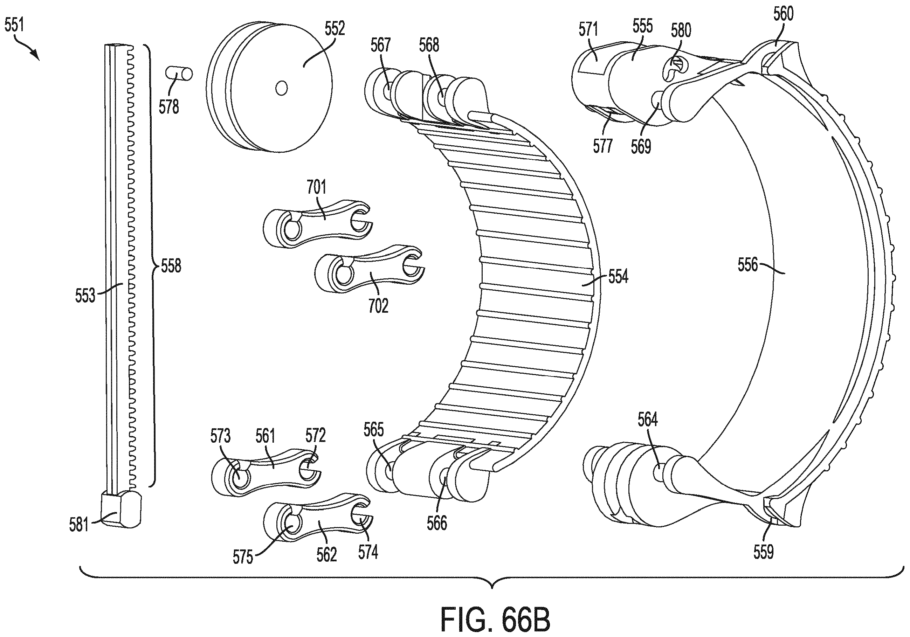

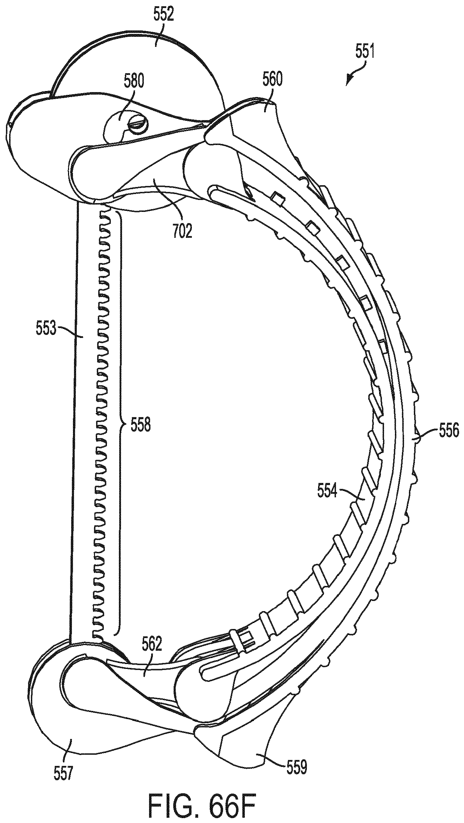

Figs. 66A-66G show several views of a valve 551 having a knob 552 to move a connecting member 553 which is locked into position after movement of the knob 552 in accordance with an embodiment of the present disclosure.

The valve 551 includes an inner curved, elongated support member 554 and an outer curved, elongated support member 556. A knob 552 is pivotally coupled to the outer support member 556 via a pin 578. A connecting member 553 engages teeth 576 of the knob 552.

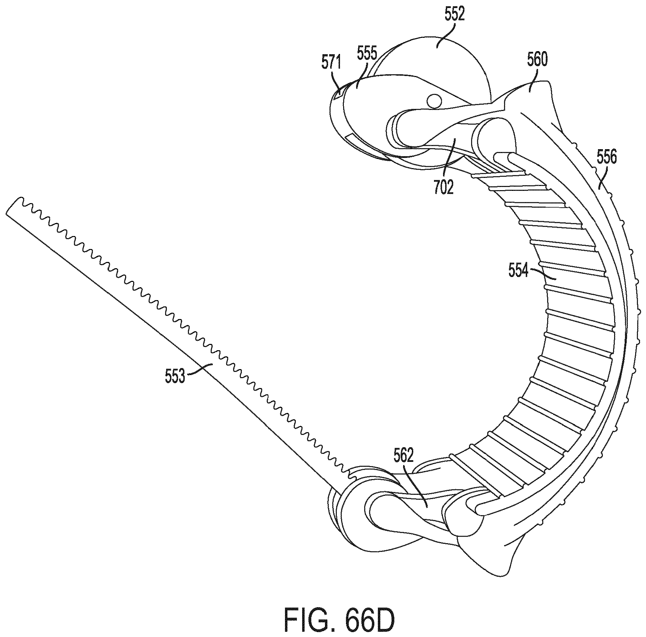

The connecting member 553 may be inserted into a hole of an end 555 of the support member 556 such that rotation of the knob 552 frictionally locks an engaging finger 700 (see Fig. 66D) into the gear rack 558 of the connecting member 553. The engaging finger 700 may engage the teeth 576 to lock the knob 552 to thereby prevent rotation of the knob 552 unless sufficient torque overcomes the locking action of the engaging finger 700. A retaining finger 577 is positioned on the other side of the hole 571 presses the connecting member 552 against the teeth 576 of the knob 552.

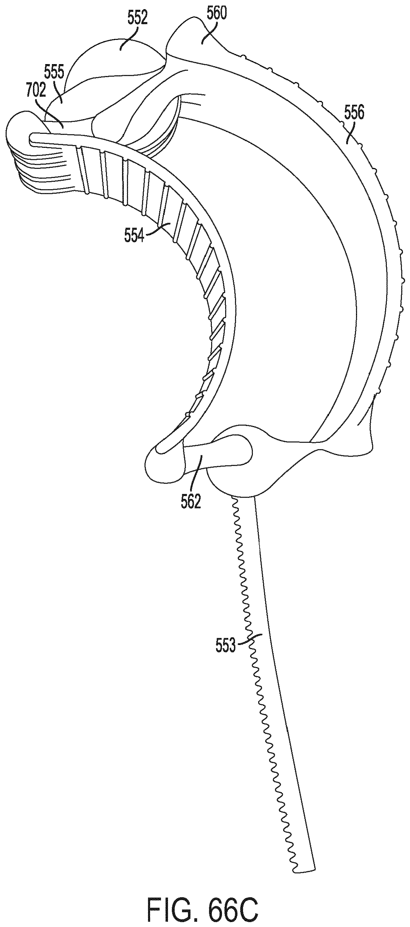

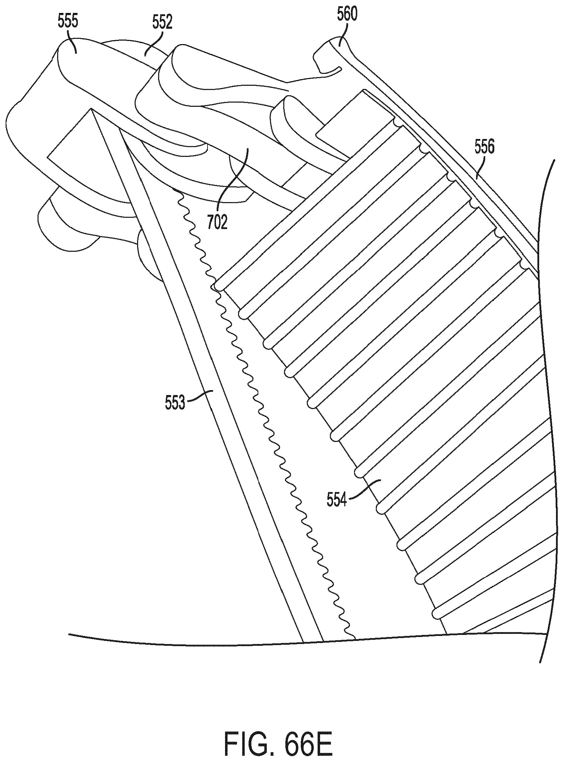

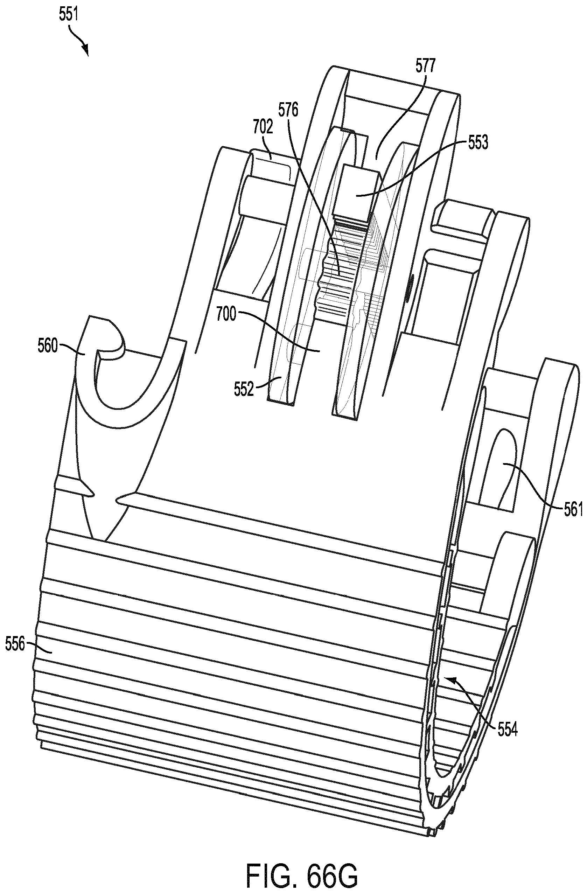

The inner support member 554 can pivot out away from the outer support member 556 such that a tube can be loaded via raised portions 559 and 560 (see Fig. 66C) . The inner support member 554 pivots away from the outer support member 556 via digbones 561, 562, 701, and 702 as shown in Fig. 66C. Thereafter, the inner support member 554 pivots back towards the support member 556 as shown in Fig. 66D. The connecting member 553 is then inserted into an end 555 of the outer support member 556 (a close up of the insertion is shown in Fig. 66E) that includes the engaging finger 700 that locks onto the teeth 576 of the knob 552 which temporarily immobilizes the connecting member 553 (see fig. 66G). The other end 581 of the connecting member 553 is locked into a hole 582 of an end 557 of the support member 556. The connecting member 553 may be pivotally connected to the end 557. The knob 552 includes teeth 576 to move the connecting member 553 in or out of the end 555. However, when the knob 552 is not moved, the engaging finger 700 locks the movement of the knob 552 unless a predetermined amount of torque clicks the finger 700 to the next tooth of the teeth 576 of the inner portion of the knob 552.

As previously mentioned, the support member 554 can swing away from the outer support member 556 as is shown in Fig. 66C, which is facilitated by the dog bone linkers 561, 562, 701, and 702. The dog bone linker 561 includes a pivot hole 572 that couples to a pivot 563, and a pivot hole 573 that couples to a pivot 565. The dog bone linker 562 includes a pivot hole 575 that couples to a pivot 566 and a pivot hole 574 that coupled to a pivot 566. The dog bone linker 701 couples to pivots 567 and 570, and the dog bone linker 702 couples to pivots 568 and 569 so that the end of the support member 556 also swings away from the inner support member 554.

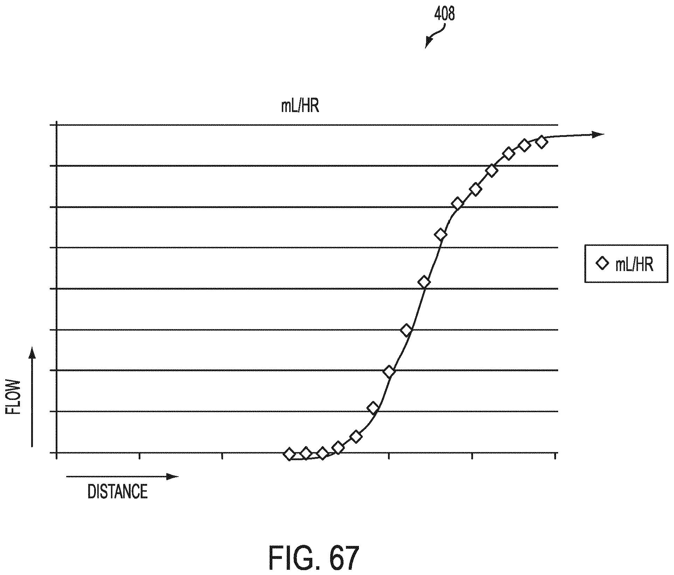

Fig. 67 shows a graphic 408 that illustrates actuation vs. flow rates for a valve in accordance with an embodiment of the present disclosure. The graphic 408 shows the operation of a valve having elongated support members, such as, for example, the valve 340 of Figs. 49 and 50A-50B, the valve 352 of Figs. 51A-54, the valve 369 of Fig. 55, the valve 380 of Figs. 56A-56C, the valve 380 of Figs. 57A-57E, the valve 401 of Figs. 58A-58D, the valve 500 of Fig. 59H, the valve 520 of Figs. 60-60, the valve 542 of Figs. 64A-64B, the valve 547 of Fig. 65, and/or the valve 551 of Figs. 66A-66G . The x-axis of the graphic 408 shows the displacement between the ends of the support members of the valve, and the y-axis shows the flow rate (e.g., caused by gravity and/or a pressure source). The response of the valve is a nonlinear function, such as an S-curve, a sigmoid curve, a Gompertz curve, or a generalized logistic function. These functions may be adjusted to match the valve and/or the valve may be adjusted to match one of the curves or functions.

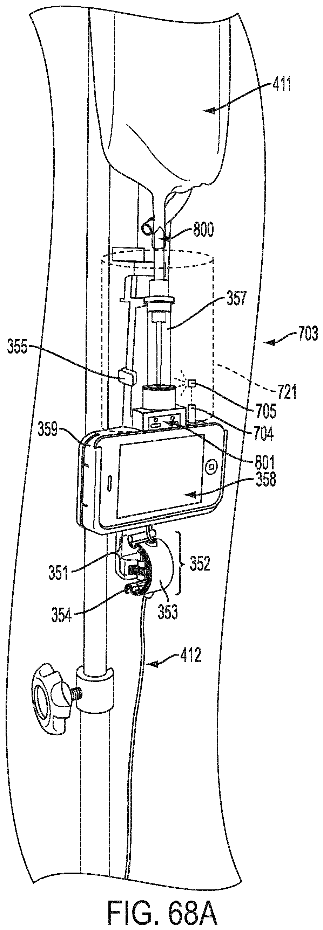

Fig. 68A shows a flow meter 703 that uses binary optics 705 in accordance with an embodiment of the present disclosure. The flow meter 703 includes a camera 355 that captures one or more images to estimate a flow rate of fluid through a drip chamber 357 using any sufficient method, e.g., the methods disclosed herein. The flow meter 703 includes a laser 704 that directs a laser beam onto a binary optic assembly 705. The binary optics assembly 705 thereafter redirects and reforms the laser beam through the drip chamber 357 and onto the image sensor 355 such that the image sensor 355 see pattern, e.g., the array of lines 85 shown in Fig. 8 which may form stripes as shown in the background pattern 89 of Fig. 10. The binary optics assembly 705 may form the stripes by using a plurality of ovals.

The image sensor 355 may include a filter to filter out all frequencies except for the frequency of the laser 704. For example, the image sensor 355 may include an optical band-pass filter that has a center frequency equal to (or about equal to) the optical frequency (or center frequency of the optical frequency) of the laser 704.

The monitoring client 358 may be electrically coupled to the laser 801 to modulate the laser 801. For example, the monitoring client 358 may turn on the laser 801 only when predetermined pixels are being exposed and may turn off the laser 801 when other pixels besides the predetermined pixels are being exposed.

The flow meter 703 optionally includes a first electrode 800 and a second electrode 801. The monitoring client 358 may be electrically coupled to the first and second electrodes 800, 801 to measure a capacitance defined therebetween. In streaming conditions, the capacitance changes because the relative permittivity is different for air and water. The monitoring client 358 may monitor the changes that results from a streaming condition with the drip chamber 357 by monitoring the capacitance between the first and second electrodes 800, 801 and correlate increases and/or decreases of the capacitance beyond a threshold as corresponding to either a streaming condition and/or a non-streaming condition. For example, if the capacitance between the first and second electrodes 800, 801 is higher than a threshold, a processer within the monitoring client 358 may determine that the drip chamber 357 is undergoing a streaming condition.

In an alternative embodiment, the first and second electrodes 800, 801 are loop antennas. The monitoring client 358 uses a transceiver to monitor the magnetic coupling between the loop antennas 800, 801. For example, the transceiver may transmit a coded message from one loop antenna of the antennas 800, 801, to another one of the loop antennas 800, 801 and then determine if the coded message was successfully received. If so, then a received signal strength indication ("RSSI") measurement may be made from the transceiver. The RSSI may be used to monitor the magnetic coupling between the antennas 800, 801. If the magnetic coupling is above a threshold, then the monitoring client 358 may determine that a streaming condition exists within the drip chamber 357. In some embodiments a change of magnetic coupling or a change of capacitive coupling may be determined to be an indication that a streaming condition has occurred.

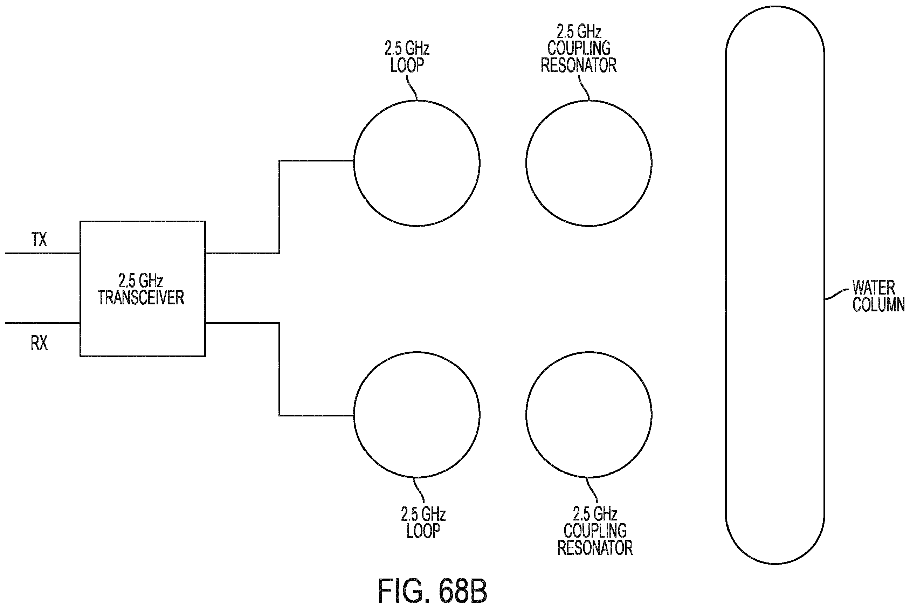

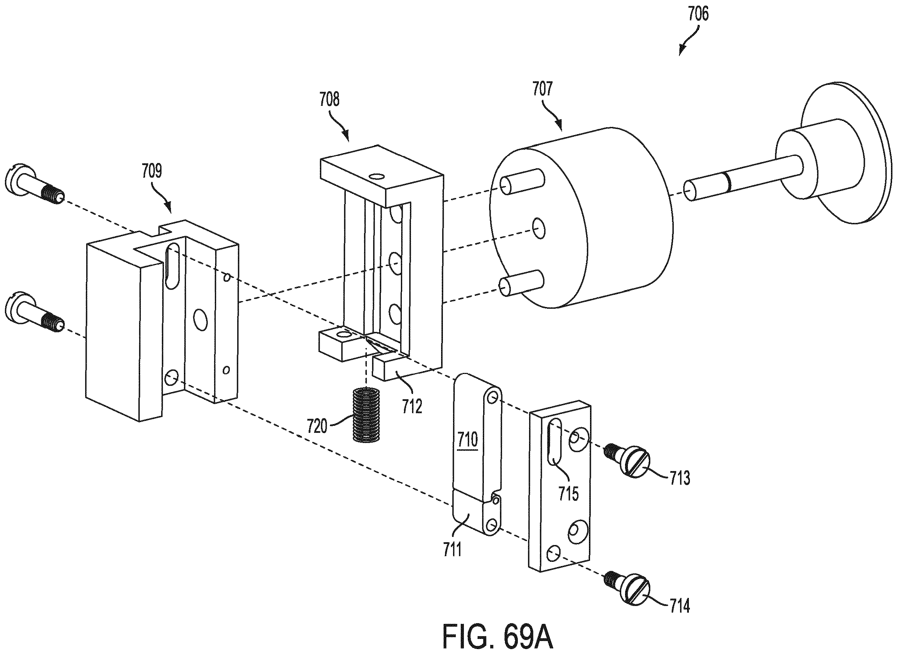

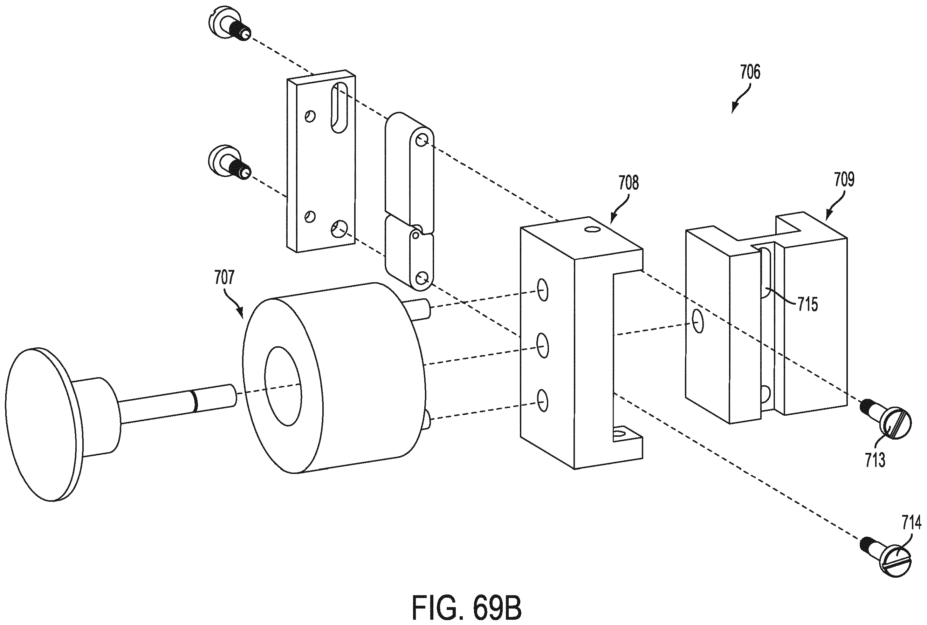

The flow meter 703 may also include a safety valve 706. Figs. 69A-69F show several views of the safety valve 706 that may be used with a flow meter, such as the flow meter 703 of Fig. 68, in accordance with an embodiment of the present disclosure.

Figs. 69A-69B show exploded views of the safety valve 706. The safety valve 706 includes a solenoid 707, an interface structure 708, a tube housing 709, a spring 720, a faceplate 712, a first axle 713, a second axle 714, a first occluding arm 710, and a second occluding arm 711. The faceplate 712 includes a hole 715 and the tube housing 708 also includes a hole 819. The holes 715, 819 allow the axle 713 to slide within the holes 715, 819.

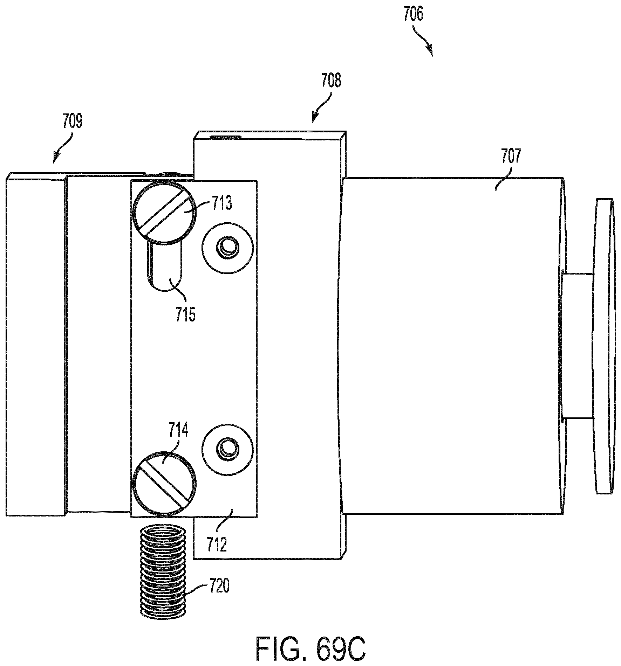

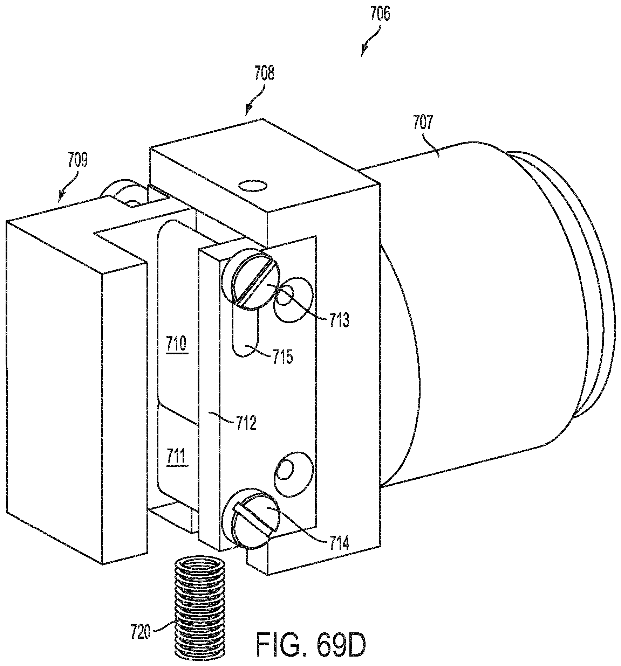

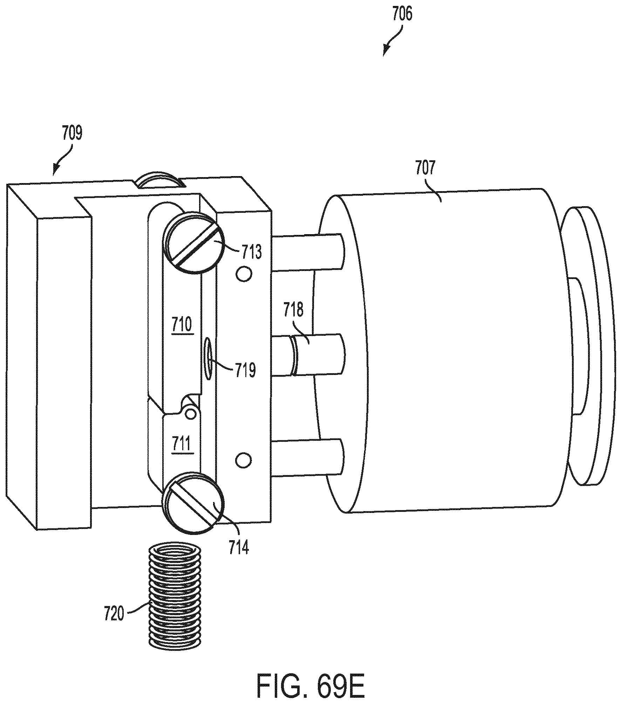

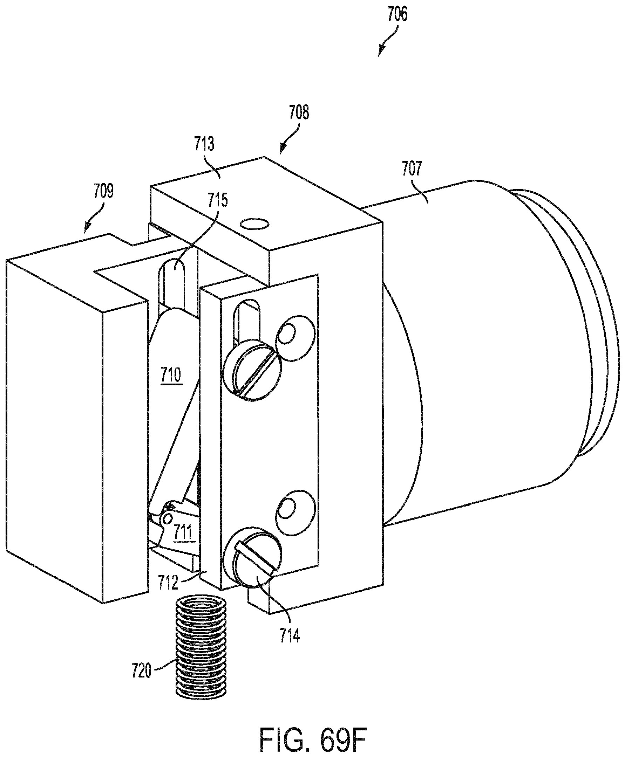

As shown in Fig. 69C, a tube 820 may be placed within the tube housing 709. Placing the tube 820 places the tube 820 next to the first and second occluding arms 710, 711, which are easily seen in Fig. 69D. A spring 720 keeps the first and second occluding arms 710, 711 retraced when in the retracted state (as shown in Fig. 69D), but stores energy such that a predetermined amount movement of the first and second occluding arms 710, 711 towards the tube 810 cause the spring to discharge its stored mechanical energy to cause the first and second occluding arms 710, 711 to extend out and occlude the tube 820. The spring 720 may be a compression spring 720 may pull 713 and 714 towards each other. The first and second occluding arms 710, 711 are pivotally connected together. As is easily seen in Fig. 69E, a shaft 718 of a solenoid 707 can actuate through a hole 719 in the tube to push on the spring 720 which causes the spring 720 to release its energy and occlude the tube 820 (see Fig. 69F for the case when the where the first and second occluding arms 710, 711 are in the occluding position).

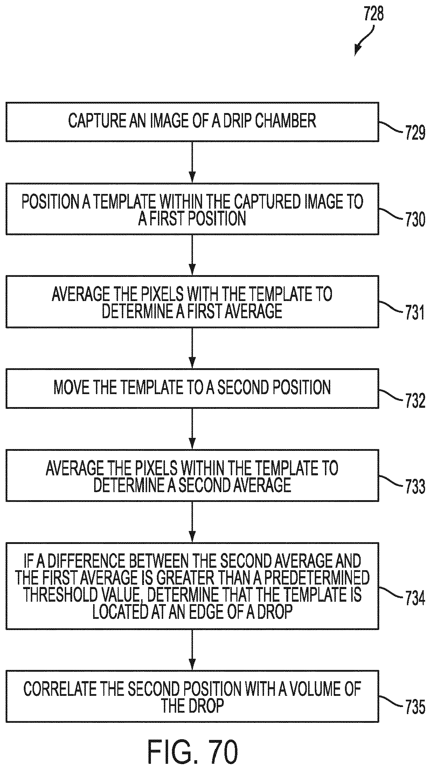

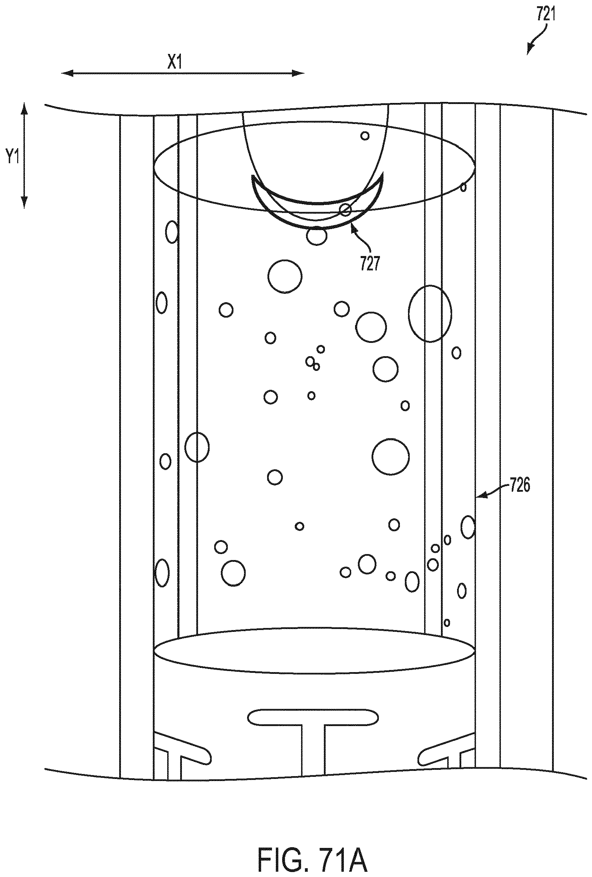

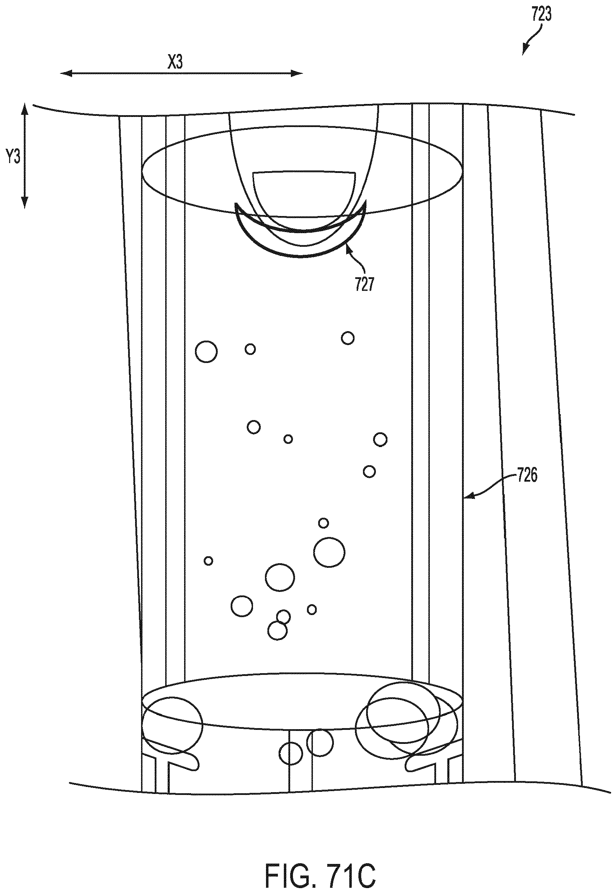

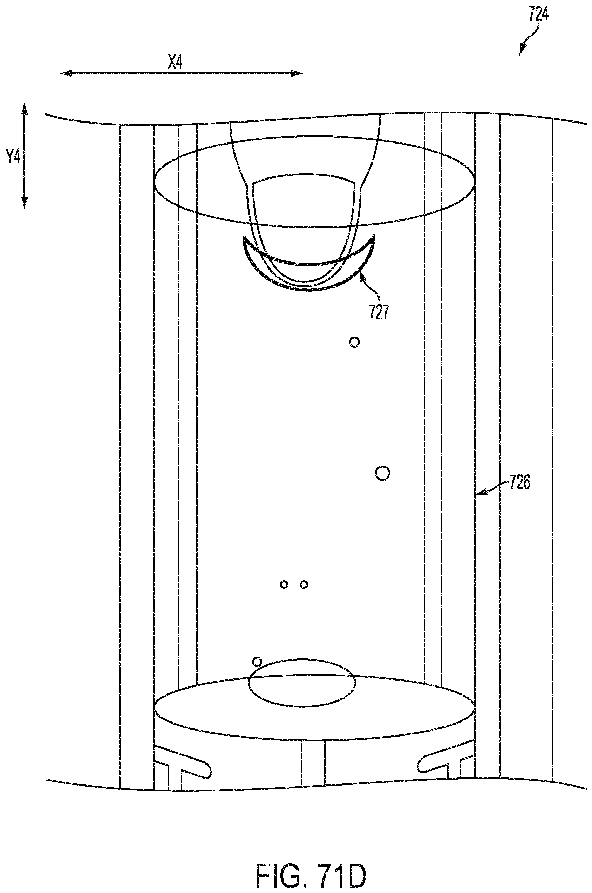

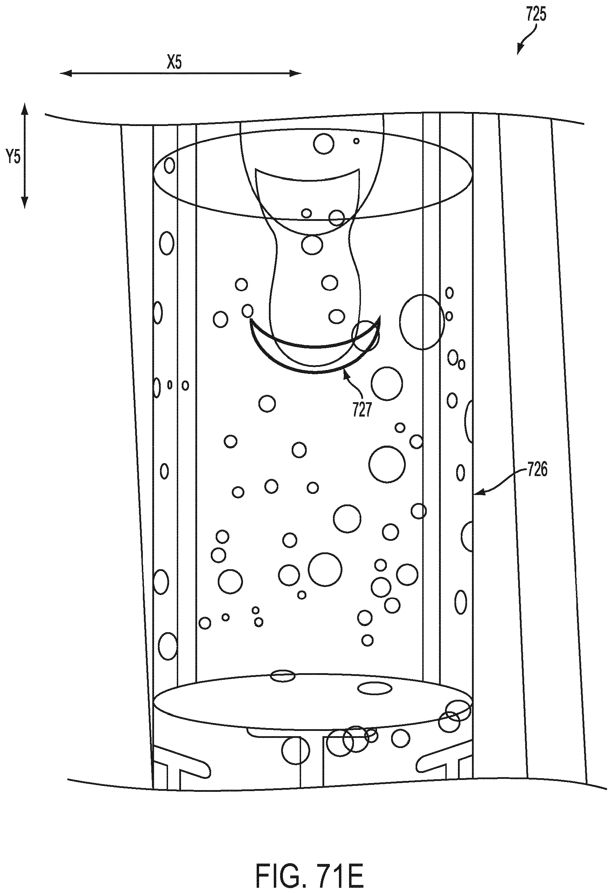

Fig. 70 shows a flow chart diagram illustrating a method 728 of estimating drop growth and/or flow within a drip chamber in accordance with an embodiment of the present disclosure. The method 728 includes acts 729-735. Figs. 71A-71B show images take by a flow meter with a template overlaid therein to illustrate the method of Fig. 70.

Act 729 captures an image of a drip chamber. The image capture may be the image 721 of Fig. 71A. Act 730 positions a template within the capture image to a first position. For example, as shown in Fig. 71A, a template 727 may be positioned within a predetermined position. Act 731 averages all of the pixels within the template 727. Act 732 moves the template to a second position. For example, the template 727 in Fig. 71A may move the template in the Y direction (e.g., down as seen in Fig. 71A).

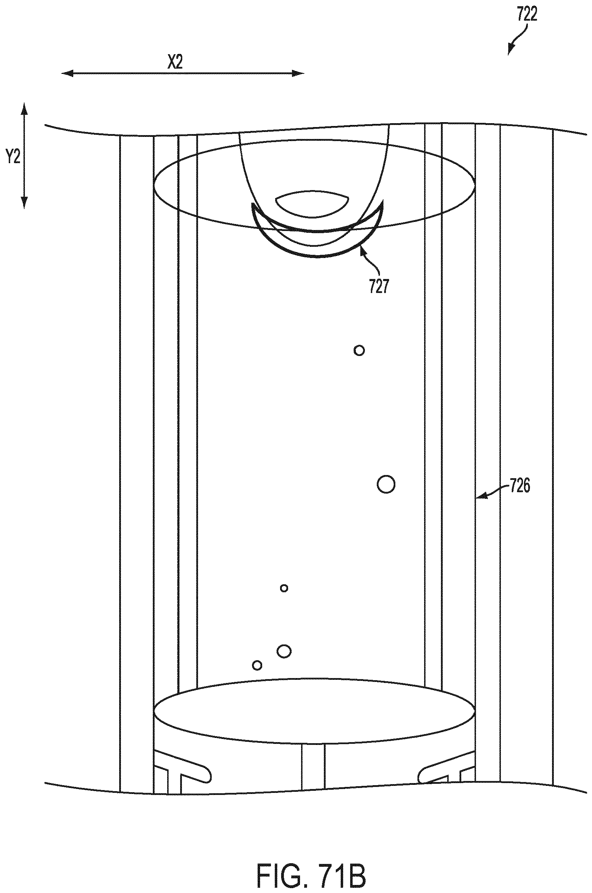

In act 733, the pixels within the template are used to determine a second average. In act 734, if a difference between the second average and the first average is greater than a predetermined threshold value, determine that the template is located at an edge of a drop. For example, referring to Fig. 71A, the template may be slowly lowered down in the Y direction, until the template 727 transitions from the edge of a drop to a portion of the image that doesn't contain the drop, in which case the average value of the pixels will transition abruptly to a dark average to a lighter average. When this transition occurs, the Y position of the template 727 is considered to be at the edge of the drop (e.g., $Y_1$ of Fig. 71A). In act 735, the second position of the drop is correlated with a volume of the drop. For example, the $Y_1$ value may be associated with a volume of a drop in a lookup table. In some embodiments of the present disclosure, multiple movements of the template 727 are needed to until the edge of the drop is detected. For example, the template 727 may be moved in the y-direction one pixel at a time (or several pixels at a time) and several template 727 movements may be needed such that the edge of the drop is detected. By monitoring the edge of the drop, the growth of the drop may be controlled by the flow meter to achieve a target flow rate (e.g., the rate of the transition between Y1 of Fig. 71A to Y2 of Fig. 71B may be controlled by a PID control loop within a flow meter). Fig. 71B shows a location, $Y_2$, that corresponds to a growth in the drop relative to the location, $Y_1$, of Fig. 71A.

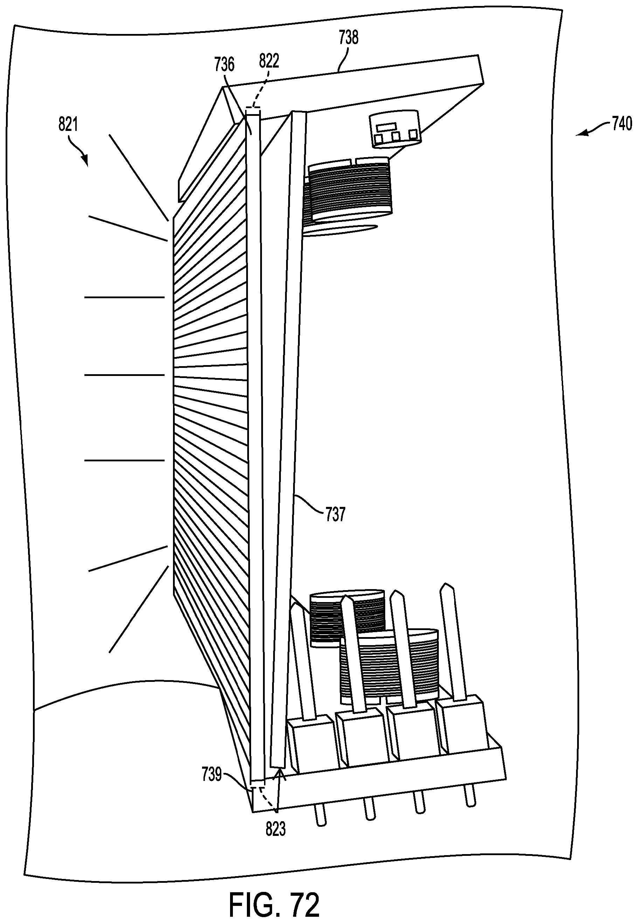

Fig. 72 shows a modulateable backlight assembly 740 in accordance with an embodiment of the present disclosure. The assembly 740 may be the backlight 18 of Fig. 1 or may be used as a backlight for any sufficient flow meter disclosed herein. The assembly 740 includes a first circuit board 738, a second circuit board 739, a first backlight diffuser 736, and a second backlight diffuser 737.

The first circuit board 738 includes embedded light sources 822 that extend along the interface between the first backlight diffuser 736 and the first circuit board 738. The embedded light sources 822 shine light into the first backlight diffuser 736 which is directed outwards as indicated by 821. The light 821 may be directed towards an image sensor. The first backlight diffuser 736 only diffuses light with no "pattern" formed when viewed by an image sensor.

The second circuit board 739 includes embedded lights 823 which are shined into the second backlight diffuser 737. The second backlight diffuser 737 creates a pattern of stripes that show up in light 821 when viewed by an image sensor. Therefore, a monitoring client (e.g., the monitoring client 358 of Fig. 51A) and/or a flow meter (e.g., the flow meter 7 of Fig. 1) can select between a striped background pattern (by activating the embedded lights 823) and a non-striped background pattern (by activating the embedded lights 822).

For example, referring now to Figs. 1 and 72, the flow meter 7 may use the backlight assembly 740 in some specific embodiments; The flow meter 7 may use a non-striped backlight pattern (by activating the embedded LEDs 822 without activating the embedded LEDs 823) to monitor the growth of drops and may switch to a striped background pattern (by activating the embedded LEDs 823 without activating the embedded LEDs 822) to detect streaming conditions.

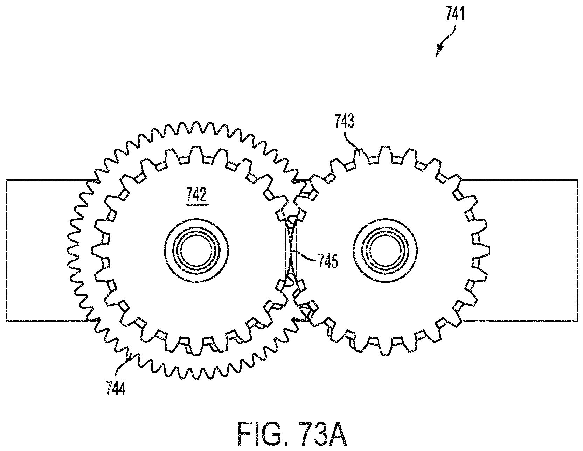

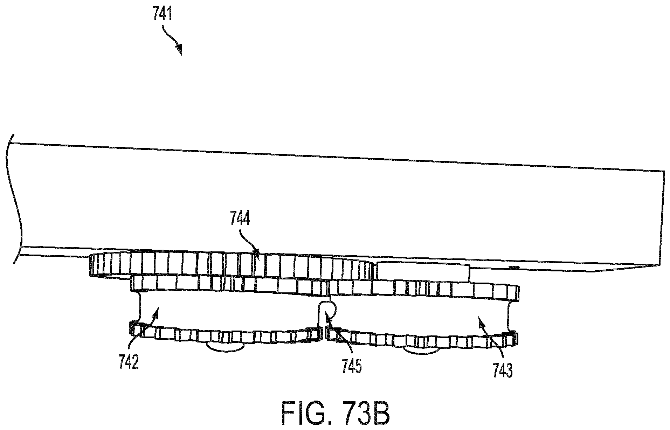

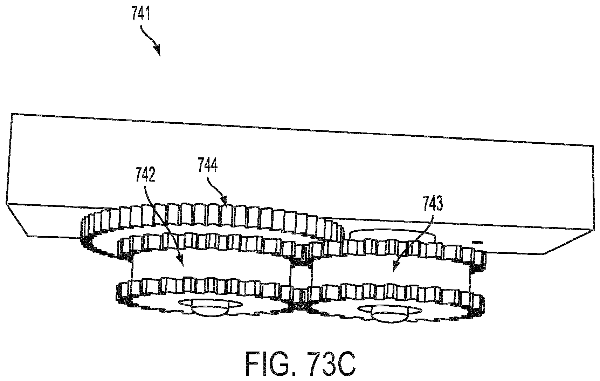

Figs. 73A-73C show several views of a tube-restoring apparatus 741 in accordance with an embodiment of the present disclosure. The apparatus 741 includes a drive gear 744 that is coupled to a first restoring gear 742. The first restoring gear 742 is mechanically coupled to a second restoring gear 743. A tube may be placed between the first and second restoring gears 742, 743. Portions of the first and second restoring gears 742, 743 define a space 745 in which a tube may be positioned. Rotation of the first and second restoring gears 742, 743 closes the distance between the space 745 when the tube is positioned between the first and second restoring gears 742, 743. The transition from a non-restoring position to a restoring position is shown in Fig. 73B to Fig. 73C. For example, a tube may be positioned such that an occlude presses against the tube from the bottom up (as shown in Fig. 73B). If the tube becomes distorted overtime, a motor connected to the driving gear 744 rotates the gears 743 and 744, to press against the walls of the tube (as shown in Fig. 73C) to restore the tube to an approximate cross-section by compressing on the wall portions of the tube that are expanded beyond a center axis of the tube such that the tube is distorted into an oval shape, for example.

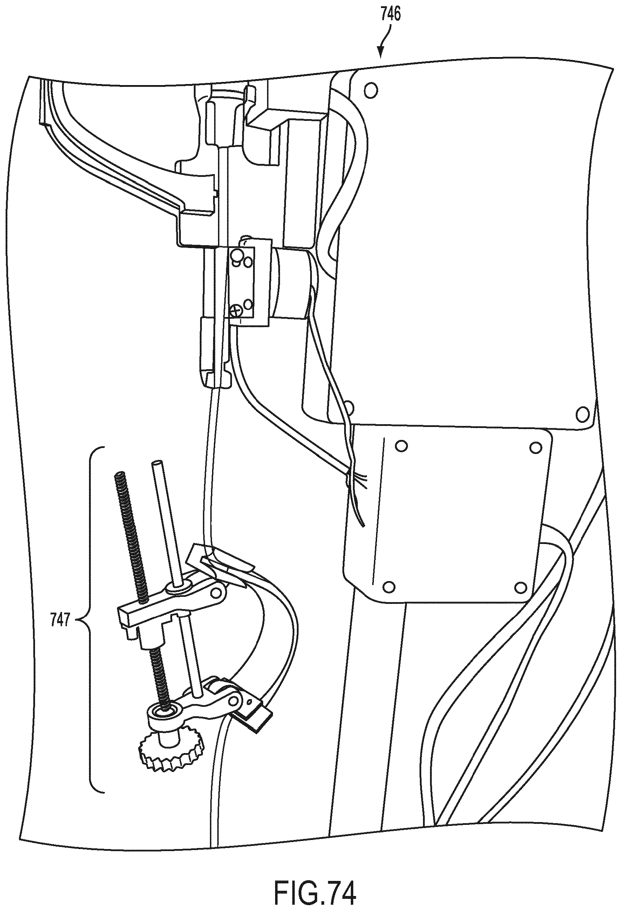

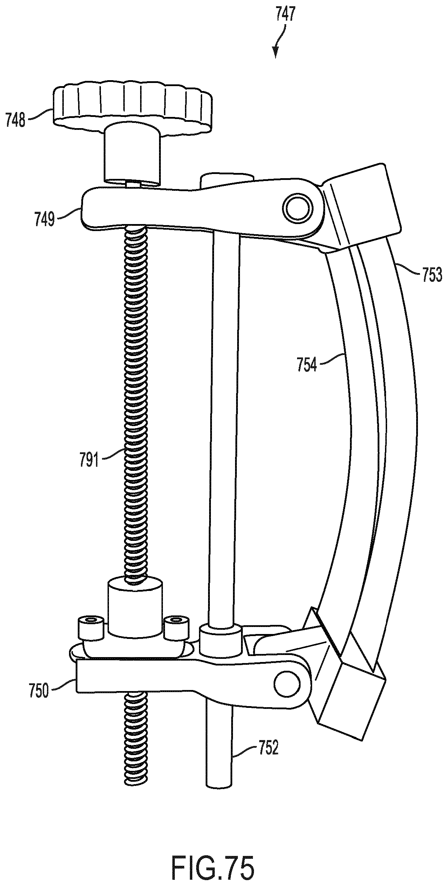

Fig. 74 shows a system for regulating fluid flow 746 using a valve 747 having two flexible strips 753 and 754 (see Fig. 75); And Fig. 75 shows the valve 746 of Fig. 74 in accordance with an embodiment of the present disclosure. Optionally, a motor may be attached to the valve 746 for control by a flow meter in one embodiment.

As shown in Fig. 75, the valve 74 includes two flexible strips 753, 754 in which a tube may be disposed therebetween, a guiding shaft 752, to guidable members 749, 750, a screw 751, and a knob 748.

When the knob 748 is turned, the screw 751 rotates. Rotation of the screw 751 pulls the distal guiding member 750 toward the proximal guiding member 749 (because the distal guiding member 750 includes internal threads and the screw 751 spins freely within the proximal guiding member 749). The guide 751 guides the movement of the distal guiding member 750. The guide 751 is coupled to the proximal guiding member 749.

FigA. 76 shows a valve 755 that utilizes a fluid-based bladder 758 in accordance with an embodiment of the present disclosure. The valve 755 includes two clamshells 756, 757, a bladder 758, and a piston 759. The piston 759 may be any fluid source. The bladder 758 may be placed within a cavity 764 and a tube may be placed across the bladder 758 and positioned within the throughways 760 and 761. Thereafter, the clamshell 757 may be placed over the bladder 758 such that the cavity 765 is placed over the bladder 758. The two clamshells 756, 757 may then be ultrasonically welded together, temporarily compressed together, and/or sufficiently held together. Thereafter, an actuator (e.g., an actuator controlled by a flow meter disclosed herein) may be actuated to move fluid in and out of the bladder 758 via the piston 759.

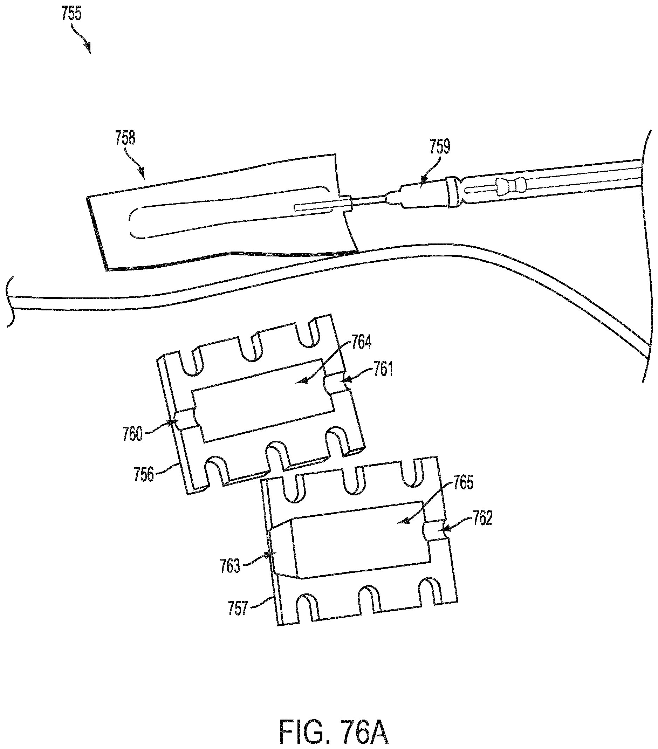

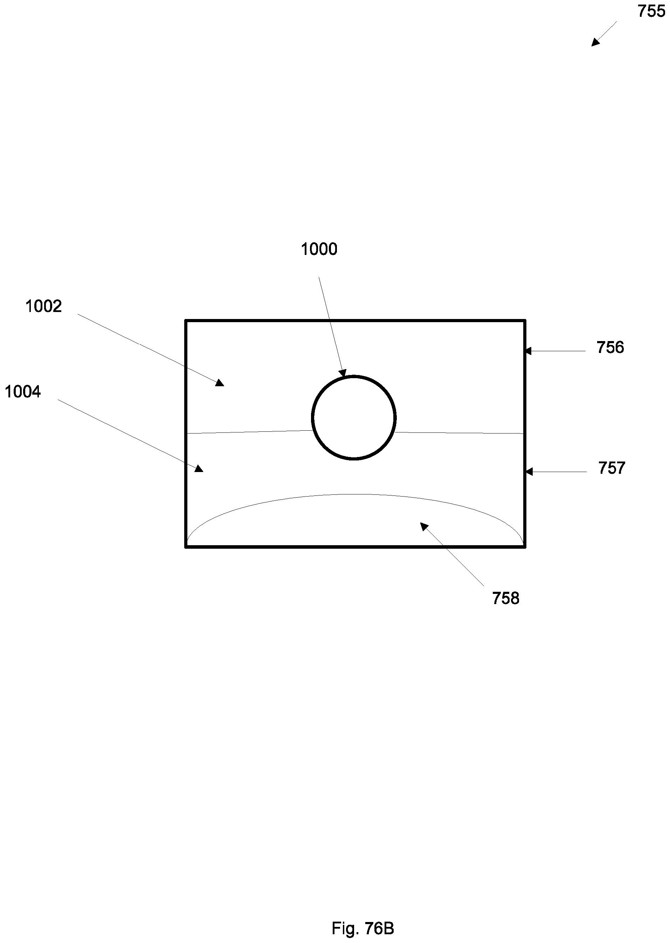

Fig. 76B shows a cross-sectional view of the assembled valve 755 of Fig. 76A with two elastomeric fillers 1002, 1004 in accordance with an embodiment of the present disclosure. The elastomeric fillers 1002, 1004 help hold the tube 1000 into position and help restore the tube 1000 when the bladder 758 is deflated.

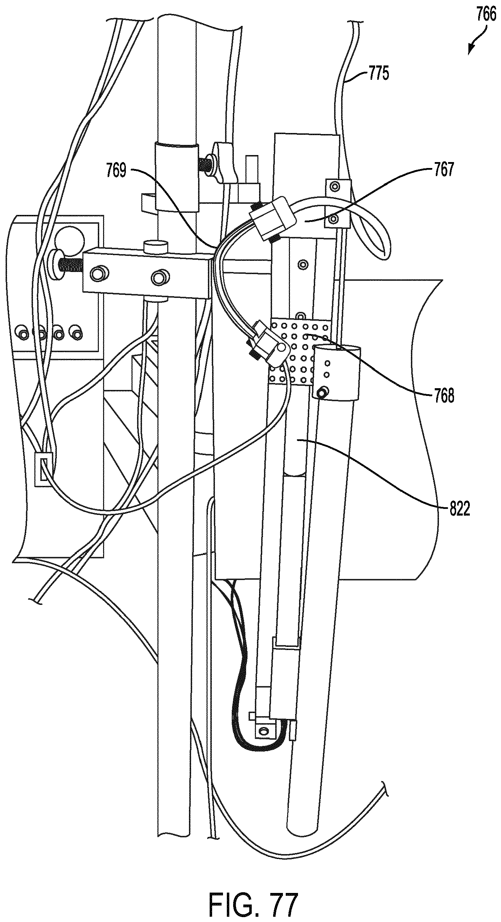

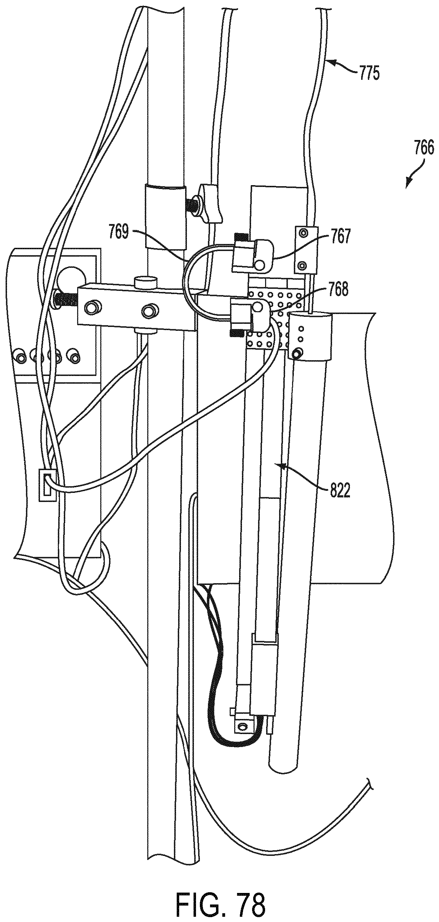

Fig. 77 shows a system 766 for regulating fluid flow using a valve 769 having two flexible strips 771, 772 actuateable by a linear actuator 822 in accordance with an embodiment of the present disclosure. Fig. 78 shows the linear actuator 822 actuating the valve 769 to impeded fluid flow through a tube 775. The valve 769 is coupled to two couplers 767 and 768. The proximal coupler 768 moves with the linear actuator 822 while the distal coupler 767 is fixed relative to a non-moving end of the linear actuator 822.

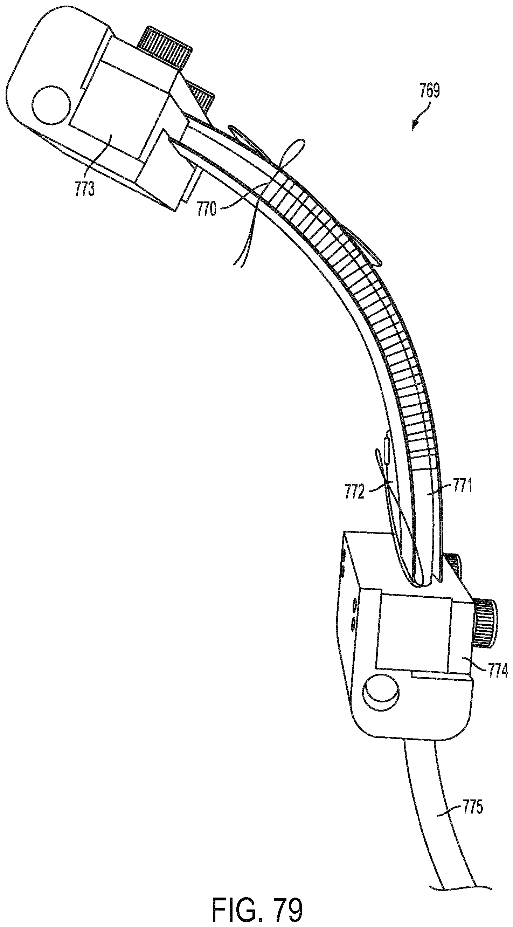

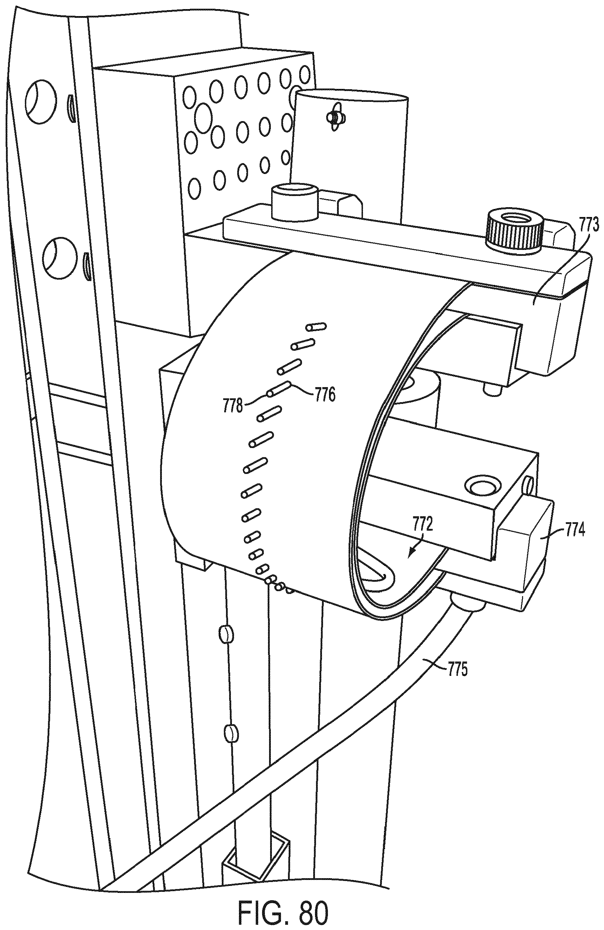

Fig. 79 shows a close-up of the valve 769 of Figs. 77-78. The valve 769 includes two strips 771, 772 (which may be metallic strips) in which the tube 775 may be disposed. The two strips 771, 772 of the valve 769 may be coupled to a first end structure 773 and a second end structure 774. The first end structure 773 may be coupled to the distal coupler 767 and the second end structure 774 may be coupled to the proximal coupler proximal coupler 768 (see Figs. 77-78). A string 770 or membrane may be wrapped around the tube 775 such that, when the strips 771, 772 are straightened out, the string 770 presses against the side walls of the tube 775 to help round the tube 775. The membrane may be a flexible by not stretchable material (or minimally stretchable material). Fig. 80 shows a close-up of the valve as actuated in Fig. 78. Note that holes 776 and 778 that the string 770 is threaded through. The string 770 (which may metallic) is spiraled around the tube 775 such that when the valve 769 opens, the string 770 restores the tube 775.

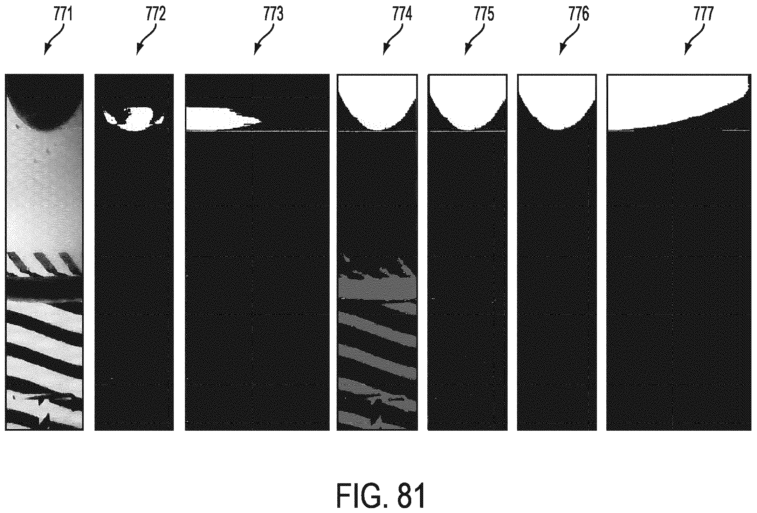

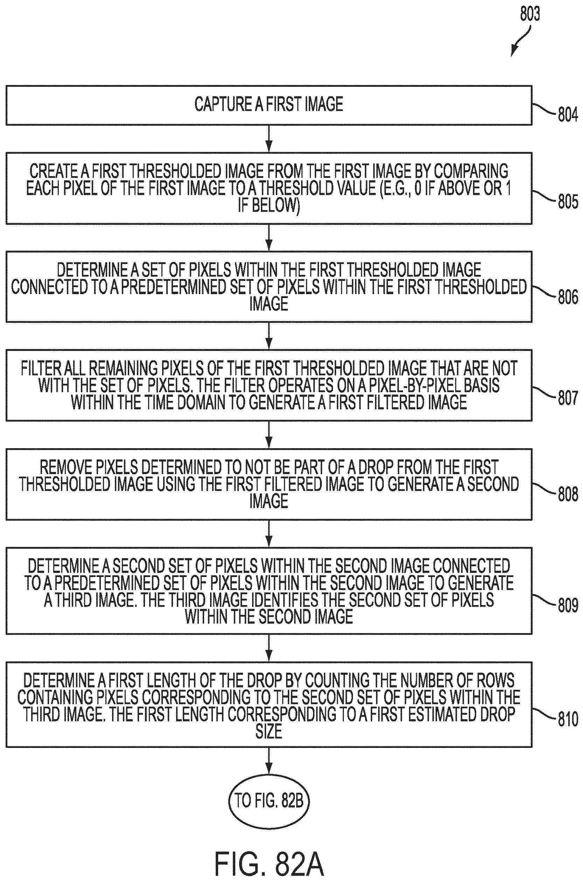

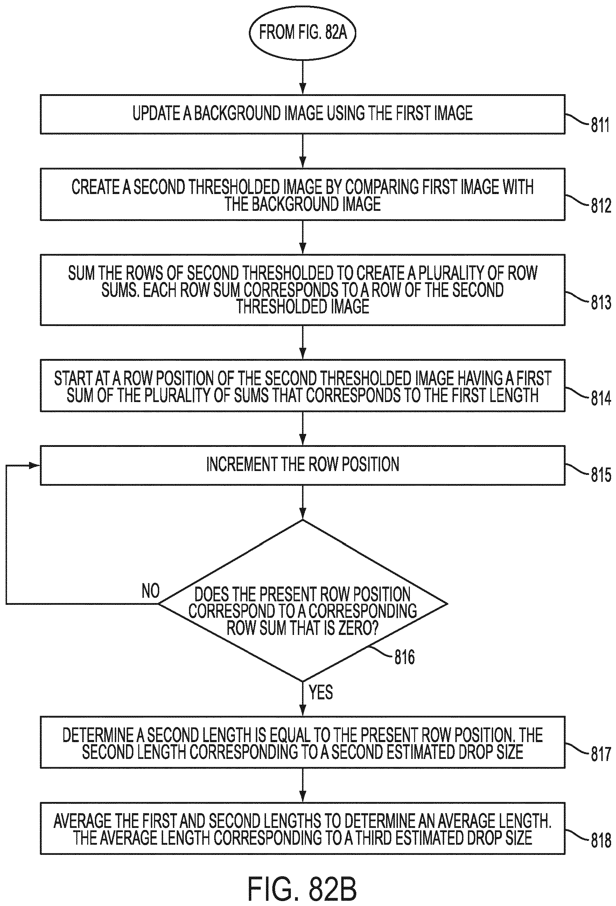

Fig. 81 shows several images for use to illustrate a method of estimating drop growth and/or fluid flow illustrated in Figs. 82A-82B in accordance with an embodiment of the present disclosure. Fig. 81 shows images 771-777 which are referred to below regarding Figs. 82A-82B.

Figs. 82A-82B show a flow chart diagram illustrating a method 803 of estimating drop growth and/or fluid flow. The method 803 includes acts 804-818.

Act 804 captures a first image (e.g., image 771 of Fig. 81). The first image may be a grey scale image of the drop changer. The drop chamber may be uniformly lit with a striped pattern on the bottom of the chamber (i.e., there is no back pattern on the top portion of the drop chamber).

Act 805 creates a first thresholded image using the first image. The first thresholded image may be the image 774 of Fig. 81. The first thresholded image may be made by comparing each pixel from the first image to a threshold value (e.g., setting a respective pixel of the thresolded to 0 if the respective pixel of the first image is above the threshold or setting a respective pixel of the thresholded to 1 if the respective pixel of the first image is below the threshold). This act is to highlight areas where there is water in front of the background.

In some specific embodiments, the threshold level is updated every time a new image is taken to ensure predetermined ratio of 1 to 0 pixels is maintained to highlight the drop. The ratio may be updated for use by act 805 when used again or the update may adjust the threshold until a predetermined ratio of 1 to 0 pixels is made and then use the first thresholded image for the rest of the method 803.

Act 806 determines a set of pixels within the first thresholded image connected to a predetermined set of pixels within the first thresholded image. The predetermined set of pixels may be determined by fiducials marked on the drip chamber or an opening in which drops are formed. The predetermined set of pixels may be a predetermined set of x, y values that correspond to pixels. Act 806 may use a connected component image analysis algorithm.

Act 807 filters all remaining pixels of the first thresholded image that are not with the set of pixels. The filter operates on a pixel-by-pixel basis within the time domain to generate a first filtered image. The first filtered image is an estimate of a non-active (e.g., a result from features not of interest in the image) portion of the first thresholded image (image 774 of Fig. 81). The filter may be any filter, e.g., any filter described herein.

Act 808 removes pixels determined to not be part of a drop from the first thresholded image using the first filtered image to generate a second image (e.g., image 775 of Fig. 81). A pixel within the second image will be set to 1 if a respective pixel in the first thresholded image is 1 and a respective pixel in the first filtered image is less than 0.5, otherwise, the pixel will be set to 0.

Act 809 determines a second set of pixels within the second image connected to a predetermined set of pixels within the second image to generate a third image (e.g., the image 776 of Fig. 81). The third image identifies the second set of pixels within the second image. Act 809 finds the set of "lit" pixels in the second image connected to the predetermined set of pixels (e.g., pixels representing the opening in which drops are formed).

Act 810 determines a first length of the drop by counting the number of rows containing pixels corresponding to the second set of pixels within the third image. That is, the drop length is determined to be equal to the last "lit" row in the set of pixels found in Act 809. The first length corresponds to a first estimated drop size.

Act 811 updates a background image using the first image. A low-pass filter may be used to update each pixel's value in the background image. An infinite impulse response filter may be used to update the background image using the first image. A pixel is only updated in the background image for rows below the first length plus a predetermined safety zone. A pixel in the background image is updated by low pass filtering the value from the corresponding pixel in the first image.

Act 812 creates a second thresholded image (e.g., image 772 of Fig. 81) by comparing first image with the background image. That is, the first image has the background image subtracted from it, and on a pixel-by-pixel basis, the absolute value of each pixel is set to 1 if it is above a second threshold value and set to a 0 if it is below the second threshold value to generate the second thresholded image.

Act 813 sums the rows of second thresholded to create a plurality of row sums (see image 773 of Fig. 81). Each row sum corresponds to a row of the second thresholded image.

Act 814 starts at a row position of the second thresholded image having a first sum of the plurality of sums that corresponds to the first length. The row position in incremented in act 815. Act 816 determines whether the present row position correspond to a corresponding row sum that is below a threshold, e.g., zero. If no, then act 815 is preformed again until the present row position corresponds to a corresponding row sum that is zero and then the method 803 proceeds to act 817.

Act 817 determines a second length is equal to the present row position. The second length corresponding to a second estimated drop size. Act 818 averages the first and second lengths to determine a average length. The average length corresponding to a third estimated drop size. By using the first and second lengths to determine an average length, the effects of condensation on the inner walls of the drip chamber are mitigated. That is, the purpose of creating two estimates of drop length is to compensate for how each length is affected by the presence of condensation. The first length tends to underestimate drop length if a drop of condensation intersects the growing drop from the spigot. The second length tends to overestimates the drop length if the drop of condensation intersects the growing drop from the spigot. Their average provides a better estimate when condensation is present. In the absence of condensation, the estimates are almost equal. In other embodiments, only either the first or second length is used to estimate the drop size.

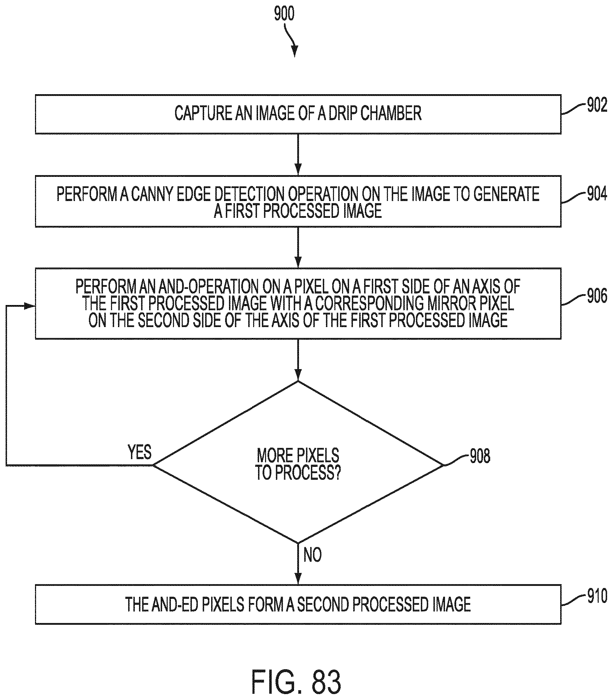

Fig. 83 shows a flow chart diagram of a method 900 for reducing noise from condensation in accordance with an embodiment of the present disclosure. Method 900 includes acts 902-910.

Act 902 captures an image of a drip chamber. Act 904 performs a canny, edge-detection operation on the image to generate a first processed image. Act 906 performs an AND-operation on a pixel on a first side of an axis of the first processed image with a corresponding mirror pixel on the second side of the axis of the first processed image. That is, Act 902 defines an axis in the first process image, and performs an AND on each pixel on one side with a pixel on the other side, such that the pixel on the other side is symmetrical with the pixel on first side. For example, a 40 (X-axis) by 40 (Y-axis) image may have an axis defined between pixel columns 19 and 20. The top, left pixel would be pixel (1,1) A pixel at location (1, 5) would be AND-ed by with a pixel at (40,5). The resulting pixel would be used for both locations (1, 5) and (40,5) to generate the second processed image.

That is, after act 906 is performed, act 908 determines whether all of the pixels have been processed. Act 908 repeats act 906 until all pixels have been processed. Act 910 provides a second processed image that is the results of all of the AND operations.

Various alternatives and modifications can be devised by those skilled in the art without departing from the disclosure. Accordingly, the present disclosure is intended to embrace all such alternatives, modifications and variances. Additionally, while several embodiments of the present disclosure have been shown in the drawings and/or discussed herein, it is not intended that the disclosure be limited thereto, as it is intended that the disclosure be as broad in scope as the art will allow and that the specification be read likewise. Therefore, the above description should not be construed as limiting, but merely as exemplifications of particular embodiments. And, those skilled in the art will envision other modifications within the scope and spirit of the claims appended hereto. Other elements, steps, methods and techniques that are insubstantially different from those described above and/or in the appended claims are also intended to be within the scope of the disclosure.

The embodiments shown in the drawings are presented only to demonstrate certain examples of the disclosure. And, the drawings described are only illustrative and are non-limiting. In the drawings, for illustrative purposes, the size of some of the elements may be exaggerated and not drawn to a particular scale. Additionally, elements shown within the drawings that have the same numbers may be identical elements or may be similar elements, depending on the context.

Where the term "comprising" is used in the present description and claims, it does not exclude other elements or steps. Where an indefinite or definite article is used when referring to a singular noun, e.g., "a," "an," or "the," this includes a plural of that noun unless something otherwise is specifically stated. Hence, the term "comprising" should not be interpreted as being restricted to the items listed thereafter; it does not exclude other elements or steps, and so the scope of the expression "a device comprising items A and B" should not be limited to devices consisting only of components A and B. This expression signifies that, with respect to the present disclosure, the only relevant components of the device are A and B.

Furthermore, the terms "first," "second," "third," and the like, whether used in the description or in the claims, are provided for distinguishing between similar elements and not necessarily for describing a sequential or chronological order. It is to be understood that the terms so used are interchangeable under appropriate circumstances (unless clearly disclosed otherwise) and that the embodiments of the disclosure described herein are capable of operation in other sequences and/or arrangements than are described or illustrated herein.

For the avoidance of doubt, the present application extends to the subject-matter described in the following numbered paragraphs (referred to as "Para" or "Paras"):

1. An apparatus for regulating fluid flow, the apparatus comprising:

a curved, elongated support member elastically deformable and having first and second ends; and
an opposing support member configured to position a tube against the curved, elongated support member between the first and second ends, wherein deformation of the curved, elongated support member by movement of the first and second ends toward each other reduces an internal cross-section along a length of the tube.

2. The apparatus according to Para 1, further comprising an actuator coupled to the curved, elongated support member to deform the curved, elongated support member by movement of the first and second ends toward each other by actuation of the actuator.

3. The apparatus according to Para 2, wherein the actuator, the curved, elongated support member, and the opposing support member are configured to regulate the fluid flow by actuation of the actuator in accordance with a Gompertz curve.

4. The apparatus according to Para 3, wherein the actuator is further configured to actuate the first and second ends toward each other along a predetermined portion of the Gompertz curve to regulate the fluid flow.

5. The apparatus according to Para 2, wherein the actuator, the curved, elongated support member, and the opposing support member are configured to regulate the fluid flow by actuation of the actuator in accordance with a sigmoid curve.

6. The apparatus according to Para 5, wherein the actuator is further configured to actuate the first and second ends toward each other along a predetermined portion of the sigmoid curve.

7. The apparatus according to Para 1, wherein the curved, elongated support member is semi-rigid.

8. The apparatus according to Para 1, wherein the curved, elongated support member consists essentially of a stretchable material.

9. The apparatus according to Para 1, wherein the curved, elongated support member is an arcuate, elongated support member.

10. The apparatus according to Para 1, wherein the curved, elongated support member is C-shaped.

11. The apparatus according to Para 1, wherein the apparatus further comprises an elongated connecting member operatively coupled to the first and second ends of the curved, elongated support member.

12. The apparatus according to Para 11, further comprising an actuator coupled to the elongated connecting member and the curved, elongated support member to apply an outward expanding force to thereby deform the first and second ends of the curved, elongated support members toward each other.

13. The apparatus according to Para 12, wherein the actuator is a lead screw, and a knob is coupled to the lead screw to actuate the lead screw.

14. The apparatus according to Para 1, wherein the opposing support member is another curved, elongated support member.

15. The apparatus according to Para 14, wherein the curved, elongated support member is disposed about parallel to the another curved, elongated support member along a substantial portion thereof.

16. The apparatus according to Para 14, wherein:

   the curved, elongated support member defines a length, and
   the another curved, elongated support member defines a length and the length of the another curved, elongated support member is disposed approximately parallel with the length of the curved, elongated support member.

17. The apparatus according to Para 14, further comprising an actuator operatively coupled to the curved, elongated support member at the first and second ends, and to the another curved, elongated support member at first and second ends, wherein actuation of the actuator causes the first and second ends of the curved, elongated support member to approach each other and causes the first and second ends of the another curved, elongated support member to approach each other thereby causing a reduction in distance between the curved, elongated support member and the another curved, elongated support member thereby compressing the tube.

18. The apparatus according to Para 1, wherein:

   the curved, elongated support member defines a length, and
   the opposing support member is disposed orthogonally from the length along a portion thereof.

19. The apparatus according to Para 1, wherein the curved, elongated support member includes a plurality of ridges disposed thereon to engage the tube.

20. The apparatus according to Para 1, wherein the opposing support member includes a plurality of ridges disposed thereon and configured to engage the tube.

21. The apparatus according to Para 1, wherein the curved, elongated support member includes a flange extending from a length thereof and configured to hold the tube.

22. The apparatus according to Para 21, wherein the opposing support member includes another flange extending from a length thereof and configured to hold the tube, wherein the flange and the another flange are about parallel to each other and are about parallel to a central axis defined by the tube when the tube is disposed therebetween.

23. An apparatus for regulating fluid flow, the apparatus comprising:

a first elongated support member defining a length;
a second elongated support member defining a length, wherein the length of the second elongated support member is disposed in spaced relation with the length of the first elongated support member to cooperate with the first elongated support member to compress a tube; and
an actuator in mechanical engagement with at least one of the first and second elongated support members to actuate the first and second elongated support members toward each other to thereby compress a tube disposed therebetween to regulate flow of fluid within the tube, wherein actuation of the actuator actuates the first and second elongated support members to regulate fluid flow within the tube in accordance with an approximate sigmoid curve.

24. The apparatus according to Para 23, wherein the length of the second elongated support member is disposed about parallel to the length of the first elongated support member.

25. The apparatus according to Para 23, wherein the first and second elongated support members are configured to cooperate with each other to compress the tube along a length of the tube at least substantially greater than the diameter of the tube.

26. The apparatus according to Para 23, wherein the actuator is further configured to actuate the first and second elongated support members to compress the tube to regulate fluid flow within the tube along a predetermined portion of the sigmoid curve.

27. An apparatus for regulating fluid flow, the apparatus comprising:

a first elongated support member defining a length;
a second elongated support member defining a length, wherein the length of the second elongated support member is disposed in spaced relation with the length of the first elongated support member to cooperate with the first elongated support member to compress a tube; and
an actuator in mechanical engagement with at least one of the first and second elongated support members to actuate the first and second elongated support members toward each other to thereby compress a tube disposed therebetween to regulate flow of fluid within the tube, wherein actuation of the actuator actuates the first and second elongated support members to regulate fluid flow within the tube in accordance with an approximate Gompertz curve.

28. The apparatus according to Para 27, wherein the length of the second elongated support member is disposed about parallel to the length of the first elongated support member.

29. The apparatus according to Para 27, wherein the first and second elongated support members are configured to cooperate with each other to compress the tube along a length at least substantially greater than the diameter of the tube.

30. The apparatus according to Para 27, wherein the actuator is further configured to actuate the first and second elongated support members to compress the tube to regulate fluid flow within the tube in accordance with a predetermined portion of the Gompertz curve.

31. An apparatus for regulating fluid flow, the apparatus comprising:

a first elongated support member defining a length;
a second elongated support member defining a length, wherein the length of the second elongated support member is disposed in spaced relation with the length of the first elongated support member to cooperate with the first elongated support member to compress a tube; and
an actuator in mechanical engagement with at least one of the first and second elongated support members to actuate the first and second elongated support members toward each other to thereby compress a tube disposed therebetween to regulate flow of fluid within the tube, wherein actuation of the actuator actuates the first and second elongated support members to regulate fluid flow within the tube in accordance with an approximate generalized logistic function.

32. The apparatus according to Para 31, wherein the length of the second elongated support member is disposed about parallel to the length of the first elongated support member.

33. The apparatus according to Para 31, wherein the first and second elongated support members are configured to cooperate with each other to compress the tube along a length of the tube at least substantially greater than the diameter of the tube.

34. The apparatus according to Para 31, wherein the actuator is further configured to actuate the first and second elongated support members to compress the tube to regulate fluid flow within the tube in accordance with a predetermined portion of the approximate generalized logistic function.

35. An apparatus for regulating fluid flow, the apparatus comprising:

a first support member that forms at least one of an arc, a plurality of arcs, a curve, a plurality of curves, an arcuate shape, a plurality of arcuate shapes, an S-shape, a C-shape, a convex shape, a plurality of convex shapes, a concave shape, and a plurality of convex shapes;
a second support member disposed in spaced relation with the first support member to cooperate with the first support member to compress a tube along a length of the tube at least substantially greater than the diameter of the tube; and
an actuator in mechanical engagement with at least one of the first and second support members to actuate the first and second support members toward each other to thereby compress a tube disposed therebetween to regulate flow of fluid within the tube, wherein actuation of the actuator actuates the first and second support members to regulate fluid flow within the tube in accordance with an approximate nonlinear function.

36. The apparatus according to Para 35, wherein the approximate nonlinear function is an approximate generalized logistic function.

37. The apparatus according to Para 35, wherein the approximate nonlinear function is an approximate sigmoid curve.

38. The apparatus according to Para 35, wherein the approximate nonlinear function is an approximate Gompertz curve.

39. The apparatus as in any one of Paras 35, 36, 37, and 38, wherein the actuator is further configured to actuate to thereby regulate the fluid flow within the tube in accordance with a predetermined portion of the approximate nonlinear function.

40. The apparatus according to Para 35, wherein the first support member forms the arc.

41. The apparatus according to Para 35, wherein the first support member has a shape consisting essentially of the arc.

42. The apparatus according to Para 35, wherein the first support member forms the plurality of arcs.

43. The apparatus according to Para 35, wherein the first support member has a shape consisting essentially of the plurality of arcs.

44. The apparatus according to Para 35, wherein the first support member forms the curve.

45. The apparatus according to Para 35, wherein the first support member has a shape consisting essentially of the curve.

46. The apparatus according to Para 35, wherein the first support member forms the plurality of curves.

47. The apparatus according to Para 35, wherein the first support member has a shape consisting essentially of the plurality of curves.

48. The apparatus according to Para 35, wherein the first support member forms the arcuate shape.

49. The apparatus according to Para 35, wherein the first support member has a shape consisting essentially of the arcuate shape.

50. The apparatus according to Para 35, wherein the first support member forms the plurality of arcuate shapes.

51. The apparatus according to Para 35, wherein the first support member has a shape consisting essentially of the plurality of arcuate shapes.

52. The apparatus according to Para 35, wherein the first support member forms the S-shape.

53. The apparatus according to Para 35, wherein the first support member has a shape consisting essentially of the S-shape.

54. The apparatus according to Para 35, wherein the first support member forms the C-shape.

55. The apparatus according to Para 35, wherein the first support member has a shape consisting essentially of the C-shape.

56. The apparatus according to Para 35, wherein the first support member forms the convex shape.

57. The apparatus according to Para 35, wherein the first support member has a shape consisting essentially of the convex shape.

58. The apparatus according to Para 35, wherein the first support member forms the plurality of convex shapes.

59. The apparatus according to Para 35, wherein the first support member has a shape consisting essentially of the plurality of convex shapes.

60. The apparatus according to Para 35, wherein the first support member forms the concave shape.

61. The apparatus according to Para 35, wherein the first support member has a shape consisting essentially of the concave shape.

62. The apparatus according to Para 35, wherein the first support member forms the plurality of concave shapes.

63. The apparatus according to Para 35, wherein the first support member has a shape consisting essentially of the plurality of concave shapes.

64. The apparatus according to Para 35, wherein a length of the second support member is disposed about parallel to a length of the first support member.

65. The apparatus according to Para 35, wherein the first and second support members are configured to cooperate with each other to compress the tube along a length of the tube at least substantially greater than the diameter of the tube.

66. An apparatus for regulating fluid flow, the apparatus comprising:

a curved, elongated support member elastically deformable and having first and second ends; and
an opposing support member configured to define a conduit with the curved, elongated support member, wherein the conduit is defined between the curved, elongated support member and the opposing member, wherein deformation of the curved, elongated support member by movement of the first and second ends toward each other reduces an internal volume of the conduit.

67. The apparatus according to Para 66, wherein the conduit is configured to receive a tube.

68. The apparatus according to Para 66, wherein:

the conduit is fluidly sealed, and
the apparatus further comprises first and second ports in fluid communication with the conduit each adapted for being coupled to a tube.

69. A system for regulating fluid flow, the system comprising:

a flexible fluid tube having a fluid path configured for passing fluid therethrough; and
an inverse-Bourdon-tube valve coupled to the flexible fluid tube to regulate the fluid flowing through the fluid path of the flexible fluid tube.

70. The system according to Para 69, further comprising an actuator coupled to the inverse-Bourdon-tube valve to actuate the inverse-Bourdon-tube valve to regulate the fluid flowing through the fluid path of the flexible fluid tube.

71. A system for regulating fluid flow, the system comprising:

a fluid tube defining a fluid path configured for passing fluid therethrough;
a valve operatively coupled to the fluid tube, the valve comprising:

a first flexible member; and
a second flexible member operatively coupled to the first flexible member;
wherein the fluid tube is disposed between the first and second flexible members, and wherein the first and second flexible members are configured to flex to thereby regulate flow of fluid passing through the fluid tube; and

an actuator coupled to at least a first end of the first flexible member and a second end of the first flexible member.

72. The system according to Para 71, wherein the actuator is a lead screw.

73. The system according to Para 71, further comprising an electrically powered motor coupled to the lead screw and configured to turn the lead screw.

74. The system according to Para 71, further comprising a knob coupled to the lead screw, wherein the knob is configured to rotate the lead screw.

75. The system according to Para 74, wherein the knob is configured for being engaged by a motor-driven actuator.

76. The system according to Para 71, wherein:

the actuator is coupled to a first end of the first flexible member and a second end of the first flexible member, and
the actuator is configured to at least one of flex the first and second ends together and flex the first and second ends away from each other.

77. The system according to Para 76, wherein the actuator flexes the first and second ends away from each other.

78. The system according to Para 76, wherein the actuator flexes the first and second flexible members such that the first and second ends approach each other.

79. The system according to Para 71, wherein the first and second flexible members are generally rectangular.

80. The system according to Para 71, wherein at least one of the first and second members is tensioned to at least substantially stop fluid flow when the actuator ceases application of a force.

81. The system according to Para 71, further comprising a flow meter coupled to a drip chamber, wherein the drip chamber is coupled to the fluid tube such that the flow meter estimates fluid flow through the drip chamber.

82. The system according to Para 81, wherein the flow meter is an image-sensor-based flow meter.

83. The system according to Para 81, wherein the flow meter is operatively coupled to a motor to actuate the valve.

84. The system according to Para 83, further comprising a control component to control the motor to actuate the valve to achieve a desired flow rate as estimated by the flow meter.

85. An apparatus for regulating fluid flow, the apparatus comprising:

a first C-shaped member defining inner and outer surfaces; and
a second C-shaped member defining inner and outer surfaces;
wherein:

at least one of the outer surface of the first C-shaped member and the inner surface of the second C-shaped member is configured to receive a tube; and
the inner surface of the second C-shaped member is disposed in spaced relation to the outer surface of the first C-shaped member.

86. The apparatus according to Para 85, wherein the second C-shaped member is flexible.

87. The apparatus according to Para 86, wherein the first C-shaped member is semi-rigid.

88. The apparatus according to Para 86, wherein the first C-shaped member is rigid.

89. The apparatus according to Para 86, wherein the second C-shaped member is an elastomer.

90. The apparatus according to Para 85, wherein a substantial area of the inner surface of the second C-shaped member abuts the outer surface of the first C-shaped member.

91. The apparatus according to Para 85, wherein at least one of the first and second C-shaped members is formed from a material selected from the group consisting of a plastic, a polymer, a monomer, a polypropylene, a thermoplastic polymer, a ceramic, a polyvinyl chloride, a polyethylene, a metal, and a composite.

92. An apparatus for regulating fluid flow, the apparatus comprising:

a first flexible sheet; and
a second flexible sheet operatively coupled to the first flexible sheet;
wherein:

the first and second flexible sheets are configured to receive a fluid tube therebetween, and
the first and second flexible sheets are configured to flex to thereby regulate flow of fluid passing through the fluid tube.

93. The apparatus according to Para 92, further comprising an actuator coupled to a first end of the first flexible sheet and a second end of the first flexible sheet.

94. The apparatus according to Para 93, further comprising a lead screw coupled to a first end of the first flexible sheet and a second end of the first flexible sheet.

95. The apparatus according to Para 94, further comprising a knob coupled to the lead screw, wherein rotation of the knob rotates the lead screw.

96. The apparatus according to Para 95, wherein the knob is configured for engagement with a motor-driven actuator whereby the motor-driven actuator actuates the knob.

97. The apparatus according to Para 93, wherein the actuator is configured to at least one of flex the first and second ends together and flex the first and second ends away from each other.

98. An apparatus for regulating fluid flow, the apparatus comprising:

a first curve-shaped member defining inner and outer surfaces; and
a second curve-shaped member defining inner and outer surfaces, wherein the inner surface of the second curve-shaped member is disposed in spaced relation to the outer surface of the first curve-shaped member.

99. The apparatus according to Para 98, wherein at least one of the first and second curve-shaped members is configured to position a fluid tube therebetween.

100. The apparatus according to Para 98, wherein the first curve-shaped member is at least one of semi-rigid and rigid.

101. The apparatus according to Para 100, wherein the second curve-shaped member is flexible.

102. The apparatus according to Para 100, wherein the second curve-shaped member comprises an elastomer.

103. The apparatus according to Para 98, wherein the first and second curve-shaped members are flexible.

104. The apparatus according to Para 98, further comprising a connecting member operatively coupled to at least one of a first end of the first curve-shaped member and a first end of the second curve-shaped member, wherein the connecting member is also operatively coupled to at least one of a second end of the first curve-shaped member and a second end of the second curve-shaped member.

105. The apparatus according to Para 104, further comprising an actuator positioned between the connecting member and the second curve-shaped member to apply a force therebetween when actuated.

106. The apparatus according to Para 105, wherein the actuator is a lead screw.

107. The apparatus according to Para 104, wherein the connecting member is flexible.

108. The apparatus according to Para 104, wherein the connecting member is semi-rigid.

109. The apparatus according to Para 104, wherein the connecting member is rigid.

110. An apparatus for regulating fluid flow, the apparatus comprising:

a first curve-shaped member defining inner and outer surfaces, the first curve-shaped member having first and second receiving members at opposite ends of the first curve-shaped member; and
a second curve-shaped member defining inner and outer surfaces, the second curve-shaped member having first and second fasteners at opposite ends of the second curve-shaped member,
wherein the first receiving member of the first curve-shaped member is configured to engage the first fastener of the second curve-shaped member and the second receiving member of the first curve-shaped member is configured to engage the second fastener of the second curve-shaped member.

111. The apparatus according to Para 110, wherein at least one of the first and second fasteners is a hook.

112. The apparatus according to Para 110, wherein at least one of the receiving members is a cylindrically-shaped member configured for coupling to a hook.

113. The apparatus according to Para 112, wherein the cylindrically-shaped member is a barrel nut.

114. The apparatus according to Para 110, wherein at least one of the receiving members is operatively coupled to an actuator.

115. The apparatus according to Para 110, wherein at least one of the receiving members is operatively coupled to an electric motor.

116. The apparatus according to Para 110, further comprising an electric motor coupled to the first receiving member, wherein:

the electric motor turns a rotor coupled to a shaft having threads on an outer surface thereof,

the second receiving member defines a threaded hole configured to receive the shaft, and
the threaded hole and shaft cooperate together to at least one of increase or decrease the distance between the first and second receiving members when the electric motor rotates the rotor to thereby rotate the shaft.

117. An apparatus for regulating fluid flow, the apparatus comprising:

a first curved, elongated support member elastically deformable and having first and second ends; and
a second curved, elongated support member elastically deformable and having first and second ends, wherein the second curved, elongated support member is configured to position a tube against the first curved, elongated support, wherein deformation of the first and second curved, elongated support members by movement of the first and second ends of the first curved, elongated support member toward each other reduces an internal volume of the tube;
a first connector coupled to the first end of the first curved, elongated support member and coupled to the first end of the second curved, elongated support member;
a second connector coupled to the second end of the first curved, elongated support member and coupled to the second end of the second curved, elongated support member, wherein the second connector defines a hole;
a connecting member having an end coupled to the first connector and another end configured for insertion into the hole of the second connector, wherein the connecting member defines a threaded rod at least along a portion thereof; and
a knob having a ratchet configured to ratchet onto the connector member when moved from the another end of the connecting member toward the end of the connector member, wherein the knob is further configured to engage the threaded rod of the connecting member.

118. The apparatus according to Para 117, the knob comprising a plurality of fingers configured to engage the threaded rod of the connecting member.

119. The apparatus according to Para 118, wherein the knob defines an outer periphery and includes a hole defined at the center of the outer periphery of the knob.

120. The apparatus according to Para 119, wherein the hole is configured to receive the threaded rod.

121. The apparatus according to Para 120, wherein the plurality of fingers each arc to engage the threaded rod at a respective end of each of the plurality of fingers.

122. The apparatus according to Para 117, wherein the first curved, elongated support member defines a first hole adjacent to the first end of the first curved, elongated support member, wherein the hole is configured to hold a fluid tube.

123. The apparatus according to Para 117, wherein the first curved, elongated support member defines a first notch adjacent to the first end of the first curved, elongated support member, wherein the notch is configured to receive a fluid tube.

124. The apparatus according to Para 123, wherein the notch includes a neck configured to receive the fluid tube and a circular region configured to retain the fluid tube.

125. An apparatus for regulating fluid flow, the apparatus comprising:

a base defining a hole configured to receive a fluid tube;
a plurality of fingers each having an end coupled to a base; and
a ring configured to slide from the base and along the plurality of fingers, wherein movement of the ring from the base compresses the fingers against the tube, wherein the ring is configured to frictionally lock against the plurality of fingers.

126. The apparatus according to Para 125, wherein each finger includes a elongated end coupled to the base and a curved end coupled to an opposite end to the base.

127. An apparatus for regulating fluid flow, the apparatus comprising:

a conically-shaped member having a surface for wrapping a tube therearound;
a complementing member configured to engage the conically-shaped member for compressing the tube; and
an actuator configured to compress the conically-shaped member against the complementing member to thereby compress the tube.

128. An intravenous administration set, comprising:

a flexible tube for directing fluid flow therewithin;
a first port at a first end of the flexible tube;
a second port a second end of the flexible tube;
a curved, elongated support member elastically deformable and having first and second ends; and
an opposing support member configured to position the flexible tube against the curved, elongated support member between the first and second ends, wherein deformation of the curved, elongated support member by movement of the first and second ends toward each other reduces an internal volume of the tube.

129. The intravenous administration set according to Para 128, further comprising a drip chamber coupled to the flexible tube.

130. The intravenous administration set according to Para 128, further comprising another port configured to receive a syringe for injection of fluid into the fluid flow within the flexible tube.

131. The intravenous administration set according to Para 128, further comprising a slide occluder coupled to the flexible tube configured to engage the flexible tube to occlude fluid flow therewithin.

132. The intravenous administration set according to Para 128, wherein: the first end of the curved, elongated support member defines a first hole to receive the flexible tube, and the second end of the curved, elongated support member defines a second hole to receive the flexible tube.

133. A flow meter, comprising:

a coupler adapted to couple to a drip chamber;
a support member operatively coupled to the coupler;
a first image sensor having a first field of view and operatively coupled to the support member, wherein the first image sensor is positioned to view the drip chamber within the first field of view;
a second image sensor having a second field of view and operatively coupled to the support member, wherein the second image sensor is positioned to view the drip chamber within the second field of view; and
at least one processor operatively coupled to the first and second image sensors, wherein:

the at least one processor receives a first image data from the first image sensor and a second image data from the second image sensor, and
the at least one processor estimates at least one parameter of liquid within the drip chamber using the first and second image data.

134. The flow meter according to Para 133, wherein the at least one parameter is one of a type of formation of the liquid, a volume of the liquid, and a shape of the liquid.

135. The flow meter according to Para 133, wherein the at least one processor determines an existence of a free flow condition using at least one of the first and second sets of image data.

136. The flow meter according to Para 133, further comprising a background pattern positioned within the field of view of the first image sensor wherein the drip chamber is between the first image sensor and the background pattern.

137. The flow meter according to Para 136, wherein the at least one processor estimates the at least one parameter using the first set of image data by analyzing a distortion of the background pattern caused by the liquid within the first field of view as viewed by the first image sensor.

138. The flow meter according to Para 136, wherein the background pattern is an array of lines having at least one angle relative to an opening of the drip chamber when viewed from the first image sensor within the first field of view using the first set of image data.

139. The flow meter according to Para 138, wherein the at least processor determines a free flow condition exists when the liquid causes the array of lines to change angles by distortion caused by the liquid when in the free flow condition as viewed within the first field of view from the first image sensor.

140. The flow meter according to Para 133, wherein the at least one processor compares at least one of the first and second image data to a background image to estimate the at least one parameter.

141. The flow meter according to Para 140, wherein the at least one processor compares the at least one of the first and second image data to the background image by calculating at least one of a difference between the at least one of the first and second image data to the background image, an absolute difference between the at least one of the first and second image data to the background image, and a squared absolute difference between the at least one of the first and second image data to the background image.

142. The flow meter according to Para 133, further comprising a non-transitory processor-readable memory in operative communication with the at least one processor, wherein the non-transitory processor-readable memory includes an operative set of processor executable instructions configured for execution by the at least one processor, wherein the operative set of processor executable instructions, when executed by the at least one processor, controls the operation of the at least one processor.

143. A flow meter, comprising:

a coupler adapted to couple to a drip chamber;
a support member operatively coupled to the coupler;
an image sensor having a field of view and operatively coupled to the support member, wherein the image sensor is positioned to view the drip chamber within the field of view;
a background pattern positioned within the field of the view of the image sensor, the background pattern positioned such that the drip chamber is between the background pattern and the image sensor; and
at least one processor operatively coupled to the image sensor to receive image data therefrom, wherein the at least one processor is configured to estimate at least one parameter of liquid within the drip chamber using distortion of the background pattern caused by the liquid as indicated by the image data, wherein the distortion is viewable within the field of view of the image sensor by the image sensor.

144. The flow meter according to Para 143, wherein the at least one parameter is at least one of a type of formation of the liquid, a volume of the liquid, and a shape of the liquid.

145. The flow meter according to Para 143, wherein the at least one processor determines an existence of a free flow condition using the distortion of the background pattern caused by the liquid as indicated by the image data.

146. The flow meter according to Para 143, wherein the background pattern is an array of lines having at least one angle relative to an opening of the drip chamber when viewed from the image sensor using the image data.

147. The flow meter according to Para 146, wherein the at least processor determines a free flow condition exists when the liquid causes the array of lines to change angles by distortion caused by the liquid when in the free flow condition as viewed within the field of view of the image sensor.

148. The flow meter according to Para 143, further comprising a non-transitory processor-readable memory in operative communication with the at least one processor, wherein the non-transitory processor-readable memory includes an operative set of processor executable instructions configured for execution by the at least one processor, wherein the operative set of processor executable instructions, when executed by the at least one processor, controls the operation of the at least one processor.

149. A flow meter, comprising:

a coupler adapted to couple to a drip chamber;
a support member operatively coupled to the coupler;
an image sensor having a field of view and operatively coupled to the support member, wherein the image sensor is positioned to view the drip chamber within the field of view; and

at least one processor operatively coupled to the image sensor to receive image data therefrom, wherein the at least one processor compares an image of the image data to a reference image to estimate at least one parameter of liquid within the drip chamber.

150. The flow meter according to Para 149, wherein the at least one processor uses a dynamic reference image.

151. The flow meter according to Para 149, wherein the at least one processor updates the reference image by multiplying each pixel of the reference image by a first constant and adding a corresponding pixel of the image multiplied by a second constant.

152. The flow meter according to Para 149, further comprising a non-transitory processor-readable memory in operative communication with the at least one processor, wherein the non-transitory processor-readable memory includes an operative set of processor executable instructions configured for execution by the at least one processor, wherein the operative set of processor executable instructions, when executed by the at least one processor, controls the operation of the at least one processor.

153. A method for exposing an image sensor implemented by an operative set of processor executable instructions configured for execution by at least one processor, the method comprising:

selecting a region of interest;
determining if a pixel is within the region of interest;
activating a light of a backlight if the pixel is within the region of interest; and
exposing the pixel.

154. The method according to Para 153, wherein the operative set of processor executable instructions is stored on a non-transitory processor-readable memory in operative communication with the at least one processor such that the at least one processor can perform the method.

155. The method according to Para 154, wherein the at least one processor is coupled to an image sensor, wherein the at least one processor performs the method using the image sensor.

156. The method according to Para 153, wherein the region of interest is a region of an image sensor that images a drip chamber.

157. The method according to Para 153, wherein the region of interest corresponds to a drip chamber.

158. The method according to Para 153, wherein the activating act activates a subset of lights including the light of the backlight.

159. The method according to Para 153, wherein the light of the backlight forms a uniform backlight.

160. The method according to Para 153, further comprising:

receiving a vertical sync signal from an image sensor; and
receiving a horizontal sync signal from the image sensor.

161. The method according to Para 160, wherein the at least one processor receives the vertical and horizontal sync signals from the image sensor.

162. The method according to Para 161, wherein the at least one processor activates the light of the backlight in accordance with at least one of the vertical and horizontal sync signals.

163. The method according to Para 161, wherein the light is a light-emitting diode.

164. The method according to Para 153, wherein an image sensor includes the region of interest and the pixel.

165. A flow meter, comprising:

a coupler adapted to couple to a drip chamber;
a support member operatively coupled to the coupler;
an image sensor having a field of view and operatively coupled to the support member, wherein the image sensor is positioned to view the drip chamber within the field of view;
a backlight having at least one light, wherein the backlight is coupled to the support member, wherein the backlight is adapted to illuminate the image sensor to expose the image sensor such that the field of view of the image sensor at least partially images at least a portion of the drip chamber; and
at least one processor operatively coupled to the image sensor to receive image data therefrom, wherein the at least one processor is configured to:

> select a region of interest of the image sensor;
> determine if a pixel of the image sensor is within the region of interest;
> activate the light of the backlight if the pixel of the image sensor is within the region of interest; and
> expose the pixel of the image sensor.

166. The flow meter according to Para 165, further comprising a non-transitory processor-readable memory readable by the at least one processor, wherein the non-transitory processor-readable memory includes an operative set of processor executable instructions stored thereon configured to cause the at least one processor, when executed, to: select the region of interest of the image sensor; determine if the pixel of the image sensor is within the region of interest; activate the light of the backlight if the pixel of the image sensor is within the region of interest; and expose the pixel of the image sensor.

167. The flow meter according to Para 165, wherein the at least one processor selects the region of interest and determines if the pixel of the image sensor is within the region of interest in accordance with the image data.

168. The flow meter according to Para 165, wherein the region of interest is a region of the image sensor that images the drip chamber.

169. The flow meter according to Para 165, wherein the region of interest corresponds to the drip chamber.

170. The flow meter according to Para 165, wherein the at least one processor activates a subset of lights including the light of the backlight.

171. The flow meter according to Para 165, wherein the light of the backlight forms a uniform backlight.

172. The flow meter according to Para 165, wherein the at least one processor is further configured to: receive a vertical sync signal from the image sensor; and receive a horizontal sync signal from the image sensor.

173. The flow meter according to Para 172, wherein the at least one processor is further configured to activate the light of the backlight in accordance with at least one of the vertical and horizontal sync signals.

174. A method, comprising:

> capturing an image including an image of a drip chamber using an image sensor having a field of view including the drip chamber;
> subtracting the image from a background image to thereby generate a difference image;
> converting each pixel of the difference image to a true value if an absolute value of a respective pixel is greater than a predetermined threshold or to a false value if the absolute value of the respective pixel is less than the predetermined threshold;
> summing each row of the converted difference image to generate a plurality of summation values, wherein each summation value of the plurality of summation values corresponds to a respective row of the converted difference image; and
> examining the plurality of summation values.

175. The method according to Para 174, wherein the act of examining the plurality of summation values includes determining if a free flow condition exists within the drip chamber.

176. The method according to Para 175, wherein the act of determining if the free flow condition exists includes determining if the plurality of summation values includes a plurality of contiguous summation values above another predetermined threshold.

177. The method according to Para 174, wherein the act of examining the plurality of summation values includes determining if a drop has been formed within the drip chamber.

178. The method according to Para 177, wherein the act of determining if the drop has been formed within the drip chamber includes determining if the plurality of summation values includes a plurality of contiguous summation values within a predetermined range greater than a minimum value and less than a maximum value.

179. The method according to Para 174, further comprising smoothing the plurality of summation values prior to the examining act.

180. The method according to Paras 179, wherein the smoothing act is in accordance with at least one of a spline function, a cubic spline function, a B-spline function, a Bezier spline function, a polynomial interpolation, a moving average, a data smoothing function, and a cubic-spline-type function.

181. The method according to Para 174, wherein the method is implemented by an operative set of processor executable instructions stored on a non-transitory processor-readable memory in operative communication with at least one processor such that the at least one processor performs the method.

182. The method according to Para 181, wherein:

the at least one processor is coupled to the image sensor, and
the at least one processor performs the method using the image sensor.

183. The method according to Para 174, further comprising converting each pixel of the difference image to an absolute value of each pixel after the subtracting act and prior to the converting act.

184. The method according to Para 174, further comprising converting each pixel of the difference image to a squared value of each pixel after the subtracting act and prior to the converting act.

185. A flow meter, comprising:

a coupler adapted to couple to a drip chamber;
a support member operatively coupled to the coupler;
an image sensor having a field of view and operatively coupled to the support member, wherein the image sensor is positioned to view the drip chamber within the field of view;
a light coupled to the support member and adapted to illuminate the image sensor to expose the image sensor such that the field of view of the image sensor at least partially images at least a portion of the drip chamber; and
at least one processor operatively coupled to the image sensor to receive image data therefrom, wherein the at least one processor is configured to:

capture an image including an image of a drip chamber using the image sensor having a field of view including the drip chamber;
subtract the image from a background image to thereby generate a difference image;
convert each pixel of the difference image to a true value if an absolute value of a respective pixel is beyond a predetermined threshold or to a false value if the absolute value of the respective pixel is less than the predetermined threshold;
sum each row of the converted difference image to generate a plurality of summation values, wherein each summation value of the plurality of summation values corresponds to a respective row of the converted difference image; and
examine the plurality of summation values.

186. The flow meter according to Para 185, further comprising a non-transitory processor-readable memory readable by the at least one processor, wherein the non-transitory processor-readable memory includes an operative set of processor executable instructions stored thereon configured to cause the at least one processor, when executed, to: capture the image including the image of a drip chamber using the image sensor having a field of view including the drip chamber; subtract the image from the background image to thereby generate the difference image; convert each pixel of the difference image to the true value if the absolute value of the respective pixel is beyond the predetermined threshold or to the false value if the absolute value of the respective pixel is less than the predetermined threshold; sum each row of the converted difference image to generate the plurality of summation values, wherein each summation value of the plurality of summation values corresponds to the respective row of the converted difference image; and examine the plurality of summation values.

187. The flow meter according to Para 185, wherein the at least one processor is further configured to determine if a free flow condition exists within the drip chamber when the processor examines the plurality of summation values.

188. The flow meter according to Para 187, wherein the at least one processor is further configured to determine if the plurality of summation values includes a plurality of contiguous summation values above another predetermined threshold when the at least one processor determines if the free flow condition exists.

189. The flow meter according to Para 185, wherein the at least one processor is further configured to determine if a drop has been formed within the drip chamber when the at least one processor examines the plurality of summation values.

190. The flow meter according to Para 189, wherein the at least one processor is further configured to determine that a drop has been formed if the plurality of summation values includes a plurality of contiguous summation values within a predetermined range greater than a minimum value and less than a maximum value and a location of the contiguous summation values corresponds to a predetermined range of locations in which a drop can form.

191. The flow meter according to Para 185, the at least one processor is further configured to smooth the plurality of summation values prior to when the at least one processor examines the plurality of summation values.

192. The flow meter according to Paras 191, wherein the at least one processor smoothes in accordance with at least one of a spline function, a cubic spline function, a B-spline function, a Bezier spline function, a polynomial interpolation, a moving average, a data smoothing function, and a cubic-spline-type function.

193. The flow meter according to Para 185, wherein the flow meter further includes a non-transitory processor-readable memory having an operative set of processor executable instructions stored thereon, the non-transitory processor-readable memory is in operative communication with at least one processor such that the operative set of processor executable instructions controls the operation of the at least one processor.

194. The flow meter according to Para 185, wherein the at least one processor is further configured to convert each pixel of the difference image to an absolute value of each pixel after the subtraction act and prior to the conversion.

195. The flow meter according to Para 185, wherein the at least one processor is further configured to convert each pixel of the difference image to a squared value of each pixel after the subtraction act and prior to the conversion.

196. A method, comprising:

    capturing an image of a drip chamber using an image sensor;
    identifying a plurality of pixels of interest within the image;
    determining a subset of pixels within the plurality of pixels of interest, wherein each pixel of the plurality of pixels is determined to be within the subset of pixels when there is a path to a baseline corresponding to the drip chamber;
    performing a rotation operation on the subset of pixels; and
    estimating a volume of the drop within the drip chamber by counting a number of pixels within the rotated subset of pixels.

197. The method according to Para 196, wherein the baseline is a predetermined set of pixels within the image sensor.

198. The method according to Para 196, wherein the plurality of pixels of interest are identified by comparing the image to a background image.

199. The method according to Para 198, further comprising initializing the background image.

200. The method according to Para 198, further comprising updating the background image using the image captured by the image sensor.

201. The method according to Para 200, wherein the background image is updated in accordance with:

$$P_{\text{background},i,j} = P_{\text{background},i,j}\left(1-\alpha_{\text{background}}\right) + \alpha_{\text{background}}P_{\text{input},i,j}\,.$$

202. The method as in Paras 196 or 198, further comprising updating an array of variances using the image captured by the image sensor.

203. The method according to Para 202, wherein the array of variances is updated in accordance with:

$$\sigma^2_{\text{temp}} = \left(P_{\text{background},i,j} - P_{\text{input},i,j}\right)^2$$

$$\sigma^2_{\text{background},i,j} = \sigma^2_{\text{background},i,j}\left(1-\beta_{\text{background}}\right) + \beta_{\text{background}}\sigma^2_{\text{temp}}\,.$$

204. The method according to Para 198, further comprising updating an array of integers in accordance with the image captured by the image sensor.

205. The method according to Para 204, wherein each integer of the array of integers corresponds to a number of updates of a pixel of the background image.

206. The method according to 205, wherein the comparison of the image to the background image only compares pixels within the image to pixels within the background image if a respective integer of the array of integers indicates a respective pixel within the background image has been updated at least a predetermined number of times.

207. The method according to Para 196, further comprising

identifying a drop in the image and a predetermined band near an edge of the drop; and
initializing the background image by setting each pixel of the background image to the image unless it is within the identified drop or the predetermined band near the edge of the drop.

208. The method according to Para 207, further comprising setting a pixel of the background image to a predetermined value if a corresponding pixel of the image is within the identified drop or the predetermined band near the edge of the drop.

209. The method according to Para 208, wherein the corresponding pixel of the image has a location corresponding to a location of the pixel of the background image.

210. The method according to Para 196, further comprising determining the baseline corresponding to an opening of the drip chamber.

211. The method according to Para 196, wherein the act of determining a subset of pixels within the plurality of pixels of interest that corresponds to a drop includes determining each of the plurality of pixels of interest is within the subset of pixels if the respective pixel of the plurality of pixels has a contiguous path back to the baseline of the drop forming at an opening of the drip chamber.

212. The method according to Para 196, further comprising:

capturing a first image using the image sensor;
identifying the drop within the first image and a predetermined band near an edge of the drop;
initializing the background image by setting each pixel to the first image unless it is within the identified drop or the predetermined band near the edge of the drop;
setting pixels within the region of the drop or within the predetermined band to a predetermined value;

initializing an array of integers; and
initializing an array of variances.

213. The method according to Para 212, further comprising updating the background image, the array of integers, and the array of variances using the image.

214. A flow meter, comprising:

a coupler adapted to couple to a drip chamber;
a support member operatively coupled to the coupler;
an image sensor having a field of view and operatively coupled to the support member, wherein the image sensor is positioned to view the drip chamber within the field of view;
at least one processor operatively coupled to the image sensor to receive image data therefrom, wherein the at least one processor is configured to:

capture an image of the drip chamber using the image sensor;
identify a plurality of pixels of interest within the image;
determine a subset of pixels within the plurality of pixels of interest, wherein each pixel of the plurality of pixels is determined to be within the subset of pixels when there is a path to a baseline corresponding to the drip chamber;
perform a rotation operation on the subset of pixels; and
estimate a volume of a drop within the drip chamber by counting a number of pixels within the rotated subset of pixels.

215. The flow meter according to Para 214, further comprising a non-transitory processor-readable memory having an operative set of processor executable instructions stored thereon, the non-transitory processor-readable memory is in operative communication with at least one processor such that the operative set of processor executable instructions controls the operation of the at least one processor.

216. The flow meter according to Para 214, further comprising a non-transitory processor-readable memory readable by the at least one processor, wherein the non-transitory processor-readable memory includes an operative set of processor executable instructions stored thereon configured to cause the at least one processor, when executed, to: capture an image of a drip chamber using the image sensor; identify a plurality of pixels of interest within the image; determine a subset of pixels within the plurality of pixels of interest, wherein each pixel of the plurality of pixels is determined to be within the subset of pixels when there is a path to a baseline corresponding to the drip chamber; perform a rotation operation on the subset of pixels; and estimate a volume of the drop within the drip chamber by counting a number of pixels within the rotated subset of pixels.

217. The flow meter according to Para 214, wherein the baseline is a predetermined set of pixels within the image sensor.

218. The flow meter according to Para 214, wherein the plurality of pixels of interests are identified by comparing the image to a background image.

219. The flow meter according to Para 214, wherein the at least one processor is further configured to initialize the background image.

220. The flow meter according to Para 214, wherein the at least one processor is further configured to update the background image using the image captured by the image sensor.

221. The flow meter according to Para 220, wherein the background image is updated in accordance with:

$$P_{\text{background},i,j} = P_{\text{background},i,j}\left(1-\alpha_{\text{background}}\right) + \alpha_{\text{background}}P_{\text{input},i,j}.$$

222. The flow meter as in Paras 214 or 218, wherein the at least one processor is further configured to update an array of variances using the image captured by the image sensor.

223. The flow meter according to Para 222, wherein the array of variances is updated in accordance with:

$$\sigma^2_{\text{temp}} = \left( P_{\text{background},i,j} - P_{\text{input},i,j} \right)^2$$

$$\sigma^2_{\text{background},i,j} = \sigma^2_{\text{background},i,j} \left( 1 - \beta_{\text{background}} \right) + \beta_{\text{background}} \sigma^2_{\text{temp}}.$$

224. The flow meter according to Para 218, wherein the at least one processor is further configured to update an array of integers in according with the image captured by the image sensor.

225. The flow meter according to Para 224, wherein each integer of the array of integers corresponds to a number of updates of a pixel of the background image.

226. The flow meter according to 225, wherein the comparison of the image to the background image only compares pixels within the image to pixels within the background image if a respective integer of the array of integers indicates a respective pixel within the background image has been updated at least a predetermined number of times.

227. The flow meter according to Para 214, wherein the at least one processor is further configured to:

identify a drop in the image and a predetermined band near an edge of the drop; and
initialize the background image by setting each pixel of the background image to the image unless it is within the identified drop or the predetermined band near the edge of the drop.

228. The flow meter according to Para 227, wherein the at least one processor is further configured to set a pixel of the background image to a predetermined value if a corresponding pixel of the image is within the identified drop or the predetermined band near the edge of the drop.

229. The flow meter according to Para 228, wherein the corresponding pixel of the image has a location corresponding to a location of the pixel of the background image.

230. The flow meter according to Para 214, wherein the at least one processor is further configured to determine the baseline corresponding to an opening of the drip chamber.

231. The flow meter according to Para 214, wherein the at least one processor is further configured to determine whether each of the plurality of pixels of interest is within the subset of pixels if the respective pixel of the plurality of pixels has a contiguous path back to the baseline of the drop forming at an opening of the drip chamber to determine if the subset of pixels are within the plurality of pixels of interest that corresponds to a drop.

232. The flow meter according to Para 214, wherein the at least one processor is further configured to:

capture a first image using the image sensor;
identify the drop within the first image and a predetermined band near an edge of the drop;
initialize the background image by setting each pixel to the first image unless it is within the identified drop or the predetermined band near the edge of the drop;
set pixels within the region of the drop or within the predetermined band to a predetermined value;
initialize an array of integers; and
initialize an array of variances.

233. The flow meter according to Para 232, wherein the at least one processor is further configured to update the background image, the array of integers, and the array of variances using at least one of the image and the first image.

234. A flow meter, comprising:

an image sensor means for capturing a plurality of images of a drip chamber; and
a flow rate estimator means for estimating the flow of fluid through the drip chamber using the plurality of images.

235. The flow meter according to Para 234, wherein the flow rate estimator means includes a processor means for estimating the flow of fluid through the drip chamber using the plurality of images.

236. The flow meter according to Para 235, further comprising a memory means in operative communication with the processor means to provide an operative set of processor executable instruction to cause the processor means to estimate the flow of fluid through the drip chamber using the plurality of images.

237. A flow meter, comprising:

a memory means having an operative set of processor executable instructions configured for being executed;
a processor means for executing the operative set of processor executable instructions for implementing a flow rate estimator means for estimating the flow of fluid through the drip chamber using the plurality of images.

238. A method, comprising

a step for capturing a plurality of images of a drip chamber; and
a step for estimating the flow of fluid through the drip chamber using the plurality of images.

239. The method according to Para 238, wherein the method is implemented by an operative set of processor executable instructions stored on a non-transitory memory and executed by at least one processor.

240. An apparatus, comprising:

a coupler adapted to couple to a drip chamber;
a support member operatively coupled to the coupler;
an image sensor having a field of view and operatively coupled to the support member, wherein the image sensor is positioned to view the drip chamber within the field of view;
a valve configured to couple to a fluid tube in fluid communication with the drip chamber, wherein the valve is configured to regulate flow through the fluid tube to thereby regulate the fluid flow through the drip chamber;
at least one processor operatively coupled to the image sensor to receive image data therefrom, wherein the at least one processor is configured to:

capture a plurality of images of the drip chamber using the image sensor;
estimate a volume growth rate of the drop within the drip chamber using the plurality of images;
receive a set point corresponding to a fluid flow rate through the fluid tube;
adjust a control system in accordance with the estimated volume growth rate of the drop to achieve the set point; and
output a control signal from the control system to an actuator of the valve to control actuation of the valve in accordance with the adjusted control system.

241. The apparatus according to Para 240, further comprising a non-transitory processor-readable memory having an operative set of processor executable instructions stored thereon, the non-transitory processor-readable memory is in operative communication with at least one processor such that the operative set of processor executable instructions controls the operation of the at least one processor.

242. The apparatus according to Para 240, further comprising a non-transitory processor-readable memory readable by the at least one processor, wherein the non-transitory processor-readable memory includes an operative set of processor executable instructions stored thereon configured to cause the at least one processor, when executed, to: capture the plurality of images of the drip chamber using the image sensor; estimate the volume growth rate of the drop within the drip chamber using the plurality of images; receive the set point corresponding to the fluid flow rate through the fluid tube; adjust the control system in accordance with the estimated volume growth rate of the drop to achieve the set point; and output the control signal from the control system to an actuator of the valve to control actuation of the valve in accordance with the adjusted control system.

243. The apparatus according to Para 240, wherein the control system is at least one of a proportional-integral-derivative control system, a proportional-integral control system, a proportional-derivative control system, a proportional control system, an integral control system, a neural net control system, a fuzzy logic control system, and a bang-bang control system.

244. The apparatus according to Para 240, wherein the control system correlates the estimated volume growth rate of the drop with the fluid flow through the fluid tube.

245. The apparatus according to Para 240, wherein the valve comprises:

a curved, elongated support member elastically deformable and having first and second ends; and
an opposing support member configured to position the fluid tube against the curved, elongated support member between the first and second ends, wherein deformation of the curved, elongated support member by movement of the first and second ends toward each other reduces an internal volume of the fluid tube,
wherein the actuator is configured to move the first and second ends toward each other.

246. The apparatus according to Para 240, wherein the valve comprises:

a first elongated support member defining a length; and
a second elongated support member defining a length, wherein the length of the second elongated support member is disposed in spaced relation with the length of the first elongated support member to cooperate with the first elongated support member to compress the fluid tube,
wherein the actuator is in mechanical engagement with at least one of the first and second elongated support members to actuate the first and second elongated support members toward each other to thereby compress the fluid tube disposed therebetween to regulate flow of fluid within the fluid tube, wherein actuation of the actuator actuates the first and second elongated support members to regulate fluid flow within the tube in accordance with an approximate sigmoid curve.

247. The apparatus according to Para 240, wherein the valve comprises:

a first elongated support member defining a length; and
a second elongated support member defining a length, wherein the length of the second elongated support member is disposed in spaced relation with the length of the first elongated support member to cooperate with the first elongated support member to compress the fluid tube,
wherein the actuator is in mechanical engagement with at least one of the first and second elongated support members to actuate the first and second elongated support members toward each other to thereby compress the fluid tube disposed therebetween to regulate flow of fluid within the tube, wherein actuation of the actuator actuates the first and second elongated support members to regulate fluid flow within the fluid tube in accordance with an approximate Gompertz curve.

248. The apparatus according to Para 240, wherein the valve comprises:

a first elongated support member defining a length; and
a second elongated support member defining a length, wherein the length of the second elongated support member is disposed in spaced relation with the length of the first elongated support member to cooperate with the first elongated support member to compress the fluid tube,
wherein the actuator is in mechanical engagement with at least one of the first and second elongated support members to actuate the first and second elongated support members toward each other to thereby compress the fluid tube disposed therebetween to regulate flow of fluid within the fluid tube, wherein actuation of the actuator actuates the first and second elongated support members to regulate fluid flow within the tube in accordance with an approximate generalized logistic function.

249. The apparatus according to Para 240, wherein the valve comprises:

a first support member that forms at least one of an arc, a plurality of arcs, a curve, a plurality of curves, an arcuate shape, a plurality of arcuate shapes, an S-shape, a C-shape, a convex shape, a plurality of convex shapes, a concave shape, and a plurality of convex shapes; and
a second support member disposed in spaced relation with the first support member to cooperate with the first support member to compress the fluid tube along a length of the fluid tube at least substantially greater than the diameter of the fluid tube,
wherein the actuator in is mechanical engagement with at least one of the first and second support members to actuate the first and second support members toward each other to thereby compress the fluid tube disposed therebetween to regulate flow of fluid within the fluid tube, wherein actuation of the actuator actuates the first and second support members to regulate fluid flow within the fluid tube in accordance with an approximate nonlinear function.

250. The apparatus according to Para 240, wherein the valve comprises:

a curved, elongated support member elastically deformable and having first and second ends; and
an opposing support member configured to define a conduit with the curved, elongated support member, wherein the conduit is defined between the curved, elongated support member and the opposing member, wherein the fluid tube is disposed within the conduit and deformation of the curved, elongated support member by movement of the first and second ends toward each other reduces an internal volume of the fluid tube within the conduit.

251. The apparatus according to Para 240, wherein the valve is an inverse-Bourdon-tube valve coupled to the fluid tube to regulate the fluid flowing through the fluid path of the fluid tube.

252. The apparatus according to Para 240, wherein the valve comprises:

a first flexible member; and
a second flexible member operatively coupled to the first flexible member, wherein:

the fluid tube is disposed between the first and second flexible members,
the first and second flexible members are configured to flex to thereby regulate flow of fluid passing through the fluid tube, and the actuator is coupled to at least a first end of the first flexible member and a second end of the first flexible member.

253. The apparatus according to Para 240, wherein the valve comprises:

a first C-shaped member defining inner and outer surfaces; and
a second C-shaped member defining inner and outer surfaces;

wherein:

at least one of the outer surface of the first C-shaped member and the inner surface of the second C-shaped member is configured to receive the fluid tube,
the inner surface of the second C-shaped member is disposed in spaced relation to the outer surface of the first C-shaped member, and
the actuator is coupled to the first and second C-shaped members to bend the first and second C-shaped members to compress the fluid tube.

254. The apparatus according to Para 240, wherein the valve comprises:

a first flexible sheet; and
a second flexible sheet operatively coupled to the first flexible sheet;
wherein:

the first and second flexible sheets are configured to receive the fluid tube therebetween,
the first and second flexible sheets are configured to flex to thereby regulate flow of fluid passing through the fluid tube, and
the actuator is coupled to the first and second flexible sheets to regulate flow of fluid passing through the fluid tube.

255. The apparatus according to Para 240, wherein the valve comprises:

a first curve-shaped member defining inner and outer surfaces; and
a second curve-shaped member defining inner and outer surfaces,
wherein:

the inner surface of the second curve-shaped member is disposed in spaced relation to the outer surface of the first curve-shaped member with the fluid tube disposed between the first and second curved-shaped members, and

the actuator is coupled to the first and second curve-shaped members to bend the first and second curve-shaped members to thereby regulate the flow of fluid within the fluid tube.

256. The apparatus according to Para 240, wherein the valve comprises:

a first curve-shaped member defining inner and outer surfaces, the first curve-shaped member having first and second receiving members at opposite ends of the first curve-shaped member; and

a second curve-shaped member defining inner and outer surfaces, the second curve-shaped member having first and second fasteners at opposite ends of the second curve-shaped member,

wherein:

the first receiving member of the first curve-shaped member is configured to engage the first fastener of the second curve-shaped member,

the second receiving member of the first curve-shaped member is configured to engage the second fastener of the second curve-shaped member, and

the actuator is coupled to the first and second curve-shaped members to bend the first and second curve-shaped members to regulate the flow of fluid of the fluid tube disposed therebetween.

257. The apparatus according to Para 240, wherein the valve comprises:

a first curved, elongated support member elastically deformable and having first and second ends; and

a second curved, elongated support member elastically deformable and having first and second ends, wherein the second curved, elongated support member is configured to position the fluid tube against the first curved, elongated support, wherein deformation of the first and second curved, elongated support members by movement of the first and second ends of the first curved, elongated support member toward each other reduces an internal volume of the fluid tube;

a first connector coupled to the first end of the first curved, elongated support member and coupled to the first end of the second curved, elongated support member;

a second connector coupled to the second end of the first curved, elongated support member and coupled to the second end of the second curved, elongated support member, wherein the second connector defines a hole;

a connecting member having an end coupled to the first connector and another end configured for insertion into the hole of the second connector, wherein the connecting member defines a threaded rod at least along a portion thereof; and

a knob having a ratchet configured to ratchet onto the connector member when moved from the another end of the connecting member toward the end of the connector member, wherein the knob is further configured to engage the threaded rod of the connecting member, wherein the actuator is coupled to the knob to rotate the knob.

258. The apparatus according to Para 240, wherein the valve comprises:

a base defining a hole configured to receive the fluid tube;

a plurality of fingers each having an end coupled to a base; and

a ring configured to slide from the base and along the plurality of fingers, wherein movement of the ring from the base compresses the fingers against the fluid tube, wherein the ring is configured to frictionally lock against the plurality of fingers, wherein the actuator is coupled to the ring to slide the ring.

259. The apparatus according to Para 240, wherein the valve comprises:

a conically-shaped member having a surface for wrapping the fluid tube therearound; and

a complementing member configured to engage the conically-shaped member for compressing the tube, wherein the actuator is configured to compress the conically-shaped member against the complementing member to thereby compress the fluid tube.

260. The apparatus according to Para 240, wherein the control system is implemented in hardware.

261. The apparatus according to Para 241, wherein the control system is implemented by at least one operational amplifier.

262. The apparatus according to Para 240, further comprising a non-transitory processor-readable memory, wherein:

the control system is implemented by an operative set of processor executable instructions configured for execution by the at least one processor,

the operative set of processor executable instructions is stored on the non-transitory processor-readable memory, and

the non-transitory processor-readable memory is in operative communication with the at least one processor to operatively communicate the operative set of processor executable instructions to the at least one processor for execution by the at least one processor.

263. The apparatus according to Para 240, wherein the set point is compared to the volume growth rate of the drop to adjust the control system.

264. The apparatus according to Para 240, wherein the set point is compared to the volume growth rate of the drop to determine an error signal.

265. The apparatus according to Para 264, wherein the error signal is the difference between the set point and the volume growth rate of the drop.

266. The apparatus according to Para 265, wherein the error signal is passed through a signal processing apparatus to generate the output signal.

267. The apparatus according to Para 266, wherein the signal processing apparatus implements a proportional-integral-derivative controller with at least one non-zero gain parameter.

268. An apparatus for regulating fluid flow, the apparatus comprising:

a curved, elongated support member elastically deformable and having first and second ends, wherein the first end is configured to pivotally couple to first and second dog bone linkers, and the second end is configured to pivotally couple to third and fourth dog bone linkers; and

an opposing support member configured to position a tube against the curved, elongated support member between the first and second ends, wherein deformation of the curved, elongated support member by movement of the first and second ends toward each other reduces an internal cross-section along a length of the tube, wherein a first end of the opposing support member is configured to pivotally couple to the first and second dog bone linkers, and a second end of the opposing support member is configured to pivotally couple to the third and fourth dog bone linkers.

269. The apparatus according to Para 268, wherein the first end of the curved, elongated support member includes an engagement finger configured to engage a rack, wherein the second end of the curved elongated is configured to pivotally couple to the rack.

270. The apparatus according to Para 269, further comprising a knob coupled to the first end of the curved, elongated support member to move the rack.

271. A flow meter, comprising:

a coupler adapted to couple to a drip chamber;
a support member operatively coupled to the coupler;
an image sensor having a field of view and operatively coupled to the support member, wherein the first image sensor is configured to view the drip chamber within the field of view;
a laser configured to shine the optical light onto the binary optics assembly; and
at least one processor operatively coupled to the image sensor, wherein:

the at least one processor receives data from the image sensor having at least a portion of the back pattern represented therein, and
the at least one processor estimates at least one parameter of liquid within the drip chamber using the image data.

272. A flow meter, comprising:

a coupler adapted to couple to a drip chamber;
a support member operatively coupled to the coupler;
a first electrode configured to couple to a fluid line in fluid communication with the drip chamber;
a second electrode configured to couple to the fluid line in fluid communication with the drip chamber; and
at least one processor operatively coupled to the first and second electrodes to measure a capacitance therebetween, wherein the at least one processor is configured to monitor the capacitance.

273. The flow meter according to Para 272, wherein the at least one processor is configured to determine if a streaming condition exists within the drip chamber using the monitored capacitance.

274. A safety valve, comprising:

a housing configured to hold a tube;
first and second occluding arms pivotally coupled together;
a first axle pivotally coupled to a distal end of the first occluding arm;
a second axle pivotally coupled to a distal end of the second occluding arm; and
a spring disposed adjacent to the first and second occluding arms on an opposite side to the tube configured to spring load the first and second occluding arm, wherein the safety valve is configured to discharge the spring and occlude the tube when the first and second occluding arms pivot away from the spring along their common pivot by a predetermined amount.

275. The safety valve according to Para 275, further comprising a solenoid configured to engage the first and second occluding arm to discharge the spring.

276. An apparatus, comprising:

a coupler adapted to couple to a drip chamber;
a support member operatively coupled to the coupler;
an image sensor having a field of view and operatively coupled to the support member, wherein the image sensor is configured to view the drip chamber within the field of view; and
at least one processor operatively coupled to the image sensor to receive image data therefrom, wherein the at least one processor is configured to:

capture an image of the drip chamber;
position a template within the captured image to a first position;
average the pixels within the template to determine a first average;
move the template to a second position;
average the pixels within the template to determine a second average;
determine that the template is located at an edge of a drop if a difference between the second average and the first average is greater than a predetermined threshold value; and
correlate the second position with a volume of the drop.

277. A method implemented by at least one processor executing an operative set of processor executable instructions configured for being executed by the at least one processor for estimating a flow rate, the method comprising:

capturing an image of the drip chamber;
positioning a template within the captured image to a first position;
averaging the pixels within the template to determine a first average;
moving the template to a second position;
averaging the pixels within the template to determine a second average;
determining that the template is located at an edge of a drop if a difference between the second average and the first average is greater than a predetermined threshold value; and
correlating the second position with a volume of the drop.

278. A flow meter, comprising:

a coupler adapted to couple to a drip chamber;
a support member operatively coupled to the coupler;
a modulatable backlight assembly configured to provide a first backlight and a second backlight;
an image sensor having a field of view and operatively coupled to the support member, wherein the image sensor is configured to view the drip chamber within the field of view and the modulatable backlight assembly; and
at least one processor operatively coupled to the image sensor and the modulatable backlight assembly, wherein:

the at least one processor receives data from the image sensor having at least a portion of the modulatable backlight assembly represented therein, and
the at least one processor is configured to modulate the backlight assembly to the first backlight when estimating a drop size and to modulate the backlight assembly to the second backlight.

279. The flow meter according to Para 278, wherein the first backlight is a diffuser backlight having no pattern and the second backlight is a diffuser backlight having a striped pattern.

280. A tube restorer, comprising:

a first gear; and
a second gear disposed abutted against the first gear, wherein the first and second gears define a space along radial portions of the first and second gears to allow a tube to flex therebetween, wherein the first and second gears are further configured to restore the tube when rotated such that the space is not positioned between the first and second gears.

281. A valve, comprising:

a first metallic strip; and
a second metallic strip;
a first guiding member coupled to distal ends of the first and second metallic strips; and
a second guiding member coupled to proximal ends of the first and second metallic strips, wherein when the first and second metallic strips are configured to compress a tube when the distal ends of the first and second metallic strips are actuated towards the proximal ends of the first and second metallic strips.

282. The valve according to Para 281, further comprising a string threaded through the first and second metallic strips to spiral around the tube.

283. A valve, comprising

first and second clamshell configured to provide a cavity between the first and second clamshells, the first and second clamshells configure to receive a tube therebetween and within the cavity;
a bladder disposed within the cavity;
a first elastomeric filler configured to position the tube within the first and second clamshells;
a second elastomeric filler configured to abut the first elastomeric filler; and
a pump configured to inflate or deflate the bladder to regulate flow of fluid within the tube.

284. An apparatus, comprising:

a coupler adapted to couple to a drip chamber;
a support member operatively coupled to the coupler; and
an image sensor having a field of view and operatively coupled to the support member, wherein the image sensor is configured to view the drip chamber within the field of view;
at least one processor operatively coupled to the image sensor to receive image data therefrom, wherein the at least one processor is configured to:

capture a first image;
create a first thresholded image from the first image by comparing each pixel of the first image to a threshold value;
determine a set of pixels within the first thresholded image connected to a predetermined set of pixels within the first thresholded image;

filter all remaining pixels of the first thresholded image that are not with the set of pixels, the filter operates on a pixel-by-pixel basis within the time domain to generate a first filtered image;

remove pixels determined to not be part of a drop from the first thresholded image using the first filtered image to generate a second image;

determine a second set of pixels within the second image connected to a predetermined set of pixels within the second image to generate a third image, the third image identifies the second set of pixels within the second image;

determine a first length of the drop by counting the number of rows containing pixels corresponding to the second set of pixels within the third image, the first length corresponding to a first estimated drop size;

update a background image using the first image;

create a second thresholded image by comparing first image with background image;

sum the rows of second thresholded to create a plurality of row sums, each row sum corresponds to a row of the second thresholded image;

start at a row position of the second thresholded image having a first sum of the plurality of sums that corresponds to the first length;

increment the row position until the row position corresponds to a corresponding row sum that is zero;

determine a second length is equal to the present row position, the

second length corresponding to a second estimated drop size; and

average the first and second lengths to determine a average length, the average length corresponding to a third estimated drop size.

285. A method implemented by at least one processor executing an operative set of processor executable instructions configured for being executed by the at least one processor for estimating a flow rate, the method comprising:

capturing a first image;

creating a first thresholded image from the first image by comparing each pixel of the first image to a threshold value;

determining a set of pixels within the first thresholded image connected to a predetermined set of pixels within the first thresholded image;

filtering all remaining pixels of the first thresholded image that are not with the set of pixels, the filter operates on a pixel-by-pixel basis within the time domain to generate a first filtered image;

removing pixels determined to not be part of a drop from the first thresholded image using the first filtered image to generate a second image;

determining a second set of pixels within the second image connected to a predetermined set of pixels within the second image to generate a third image, the third image identifies the second set of pixels within the second image;

determining a first length of the drop by counting the number of rows containing pixels corresponding to the second set of pixels within the third image, the first length corresponding to a first estimated drop size;

updating a background image using the first image;

creating a second thresholded image by comparing first image with background image;

summing the rows of second thresholded to create a plurality of row

sums, each row sum corresponds to a row of the second thresholded image;

starting at a row position of the second thresholded image having a first sum of the plurality of sums that corresponds to the first length;

incrementing the row position until the row position corresponds to a corresponding row sum that is zero;

determining a second length is equal to the present row position, the second length corresponding to a second estimated drop size; and

averaging the first and second lengths to determine a average length, the average length corresponding to a third estimated drop size.

286. A flow meter, comprising:

a coupler adapted to couple to a drip chamber;

a support member operatively coupled to the coupler;

a first loop antenna disposed adjacent to a fluid line in fluid communication with the drip chamber;

a second loop antenna disposed adjacent to the fluid line; and

at least one processor operatively coupled to the first and second loop antennas to measure a magnetic coupling therebetween, wherein the at least one processor is configured to monitor the magnetic coupling therebetween to determine if a streaming condition within the drip chamber exists.

287. An apparatus as in any one of Paras 1-38, 40-65, 67, 69-97, 99, 117-132, 268-270, 280-282, and 286 further comprising a safety valve coupled to the tube.

288. An apparatus as in any one of Paras 133-152, 165-173, 185-195, 214-237, 240-267, 271-273, 276, 278-279, and 286 further comprising a safety valve in fluid communication with the drip chamber.

290. A method implemented by an operative set of processor executable instructions, the method comprising:

determining a plurality of points of interest in an image;
randomly selecting N-points of interest of the plurality of points of interest; and
identifying a single, unique, geometric feature characterized by N-parameters corresponding to N-point of interest.

291. A system, comprising:

a non-transitory memory having stored thereon a plurality of instructions; and
at least one processor in operative communication with the non-transitory memory to execute the plurality of instruction, wherein the plurality of instructions is configured to cause the processor to:

determine a plurality of points of interest in an image;
randomly select N-points of interest of the plurality of points of interest; and
identify a single, unique, geometric feature characterized by N-parameters corresponding to N-point of interest.

292. A device substantially as shown and described.

**Claims**

1. A method comprising:

determining a baseline of a drop forming at an opening of a drip chamber;
capturing an image of a drip chamber (4, 59, 104) using an image sensor (11, 63);
identifying a plurality of pixels of interest within the image;
determining (211) a subset of pixels within the plurality of pixels of interest, wherein each pixel of the plurality of pixels of interest is determined to be within the subset of pixels when there is a path to the baseline (215), the baseline corresponding to the drip chamber (4, 59, 104);
performing (212) a rotation operation on the subset of pixels; and
estimating (213) a volume of the drop within the drip chamber (4, 59, 104) by counting a number of pixels within the rotated subset of pixels,
wherein the step of determining the subset of pixels within the plurality of pixels of interest includes determining each of the plurality of pixels of interest is within the subset of pixels if the respective pixel of the plurality of pixels of interest has a contiguous path back to the baseline (215).

2. The method according to claim 1, wherein the baseline (215) is a predetermined set of pixels within the image sensor (11, 63).

3. The method according to claim 1, wherein the plurality of pixels of interest are identified by comparing the image to a background image.

4. The method according to claim 3, further comprising initializing the background image.

5. The method according to claim 3, further comprising updating the background image using the image captured by the image sensor (11, 63).

6. The method as in claims 1 or 3, further comprising updating an array of variances using the image captured by the image sensor (11, 63).

7. The method according to claim 3, further comprising updating an array of integers in accordance with the image captured by the image sensor (11, 63).

8. The method according to claim 7, wherein each integer of the array of integers corresponds to a number of updates of a pixel of the background image.

9. The method according to claim 8, wherein the comparison of the image to the background image only compares pixels within the image to pixels within the background image if a respective integer of the array of integers indicates a respective pixel within the background image has been updated at least a predetermined number of times.

10. The method according to claim 1, further comprising
identifying the drop in the image and a predetermined band near an edge of the drop; and
initializing a background image by setting each pixel of the background image to the image unless it is within the identified drop or the predetermined band near the edge of the drop.

11. The method according to claim 10, further comprising setting a pixel of the background image to a predetermined value if a corresponding pixel of the image is within the identified drop or the predetermined band near the edge of the drop.

12. The method according to claim 11, wherein the corresponding pixel of the image has a location corresponding to a location of the pixel of the background image.

13. The method according to claim 1, further comprising determining the baseline (215) corresponding to an opening of the drip chamber (4, 59, 104).

14. The method according to claim 1, further comprising:

capturing (201) a first image using the image sensor (11, 63);
identifying (202) the drop within the first image and a predetermined band near an edge of the drop;
initializing (203) a background image by setting each pixel to the first image unless it is within the identified drop or the predetermined band near the edge of the drop;
setting (204) pixels within a region of the drop or within the predetermined band to a predetermined value;
initializing (205) an array of integers;
initializing (206) an array of variances; and
updating (209) the background image, the array of integers, and the array of variances using the image.

15. The method according to claim 1, the method further comprising the act of remotely controlling the flow rate by a monitoring client.

16. The method according to claim 15, wherein the monitoring client is a tablet.

17. A flow meter, comprising:

a coupler adapted to couple to a drip chamber (4, 59, 104);
a support member operatively coupled to the coupler;
an image sensor (11, 63) having a field of view and operatively coupled to the support member, wherein the image sensor (11, 63) is positioned to view the drip chamber (4, 59, 104) within the field of view; and
at least one processor operatively coupled to the image sensor (11, 63) to receive image data therefrom, wherein the at least one processor is configured to:

determine a baseline of a drop forming at an opening of a drip chamber;
capture an image of the drip chamber (4, 59, 104) using the image sensor (11, 63);
identify a plurality of pixels of interest within the image;
determine (211) a subset of pixels within the plurality of pixels of interest, wherein each pixel of the plurality of pixels of interest is determined to be within the subset of pixels when there is a path to the baseline (215), the baseline corresponding to the drip chamber (4, 59, 104);

perform (212) a rotation operation on the subset of pixels; and
estimate (213) a volume of the drop within the drip chamber (4, 59, 104) by counting a number of pixels within the rotated subset of pixels,

wherein the at least one processor is configured to determine the subset of pixels within the plurality of pixels of interest by determining each of the plurality of pixels of interest is within the subset of pixels if the respective pixel of the plurality of pixels of interest has a contiguous path back to the baseline (215).

**Patentansprüche**

1. Verfahren, das Folgendes umfasst:

   Bestimmen einer Basislinie für einen Tropfen, der sich an einer Öffnung einer Tropfkammer bildet;
   Aufnehmen eines Bilds einer Tropfkammer (4, 59, 104) unter Verwendung eines Bildsensors (11, 63);
   Identifizieren einer Vielzahl von Pixeln-von-Interesse innerhalb des Bilds;
   Bestimmen (211) einer Untermenge von Pixeln innerhalb der Vielzahl von Pixeln-von-Interesse, wobei jedes Pixel der Vielzahl von Pixeln-von-Interesse als innerhalb der Untermenge von Pixeln befindlich bestimmt wird, wenn es einen Pfad zu der Basislinie (215) gibt,
   wobei die Basislinie der Tropfkammer (4, 59, 104) entspricht;
   Durchführen (212) einer Rotationsoperation an der Untermenge von Pixeln; und
   Schätzen (213) eines Volumens des Tropfens innerhalb der Tropfkammer (4, 59, 104) durch Zählen einer Anzahl von Pixeln innerhalb der rotierten Untermenge von Pixeln,
   wobei der Schritt des Bestimmens der Untermenge von Pixeln innerhalb der Vielzahl von Pixeln-von-Interesse Bestimmen beinhaltet, dass sich jedes aus der Vielzahl von Pixeln-von-Interesse innerhalb der Untermenge von Pixeln befindet, wenn das jeweilige Pixel der Vielzahl von Pixeln-von-Interesse einen durchgehenden Pfad zurück zur Basislinie (215) aufweist.

2. Verfahren nach Anspruch 1, wobei die Basislinie (215) eine vorbestimmte Menge von Pixeln innerhalb des Bildsensors (11, 63) ist.

3. Verfahren nach Anspruch 1, wobei die Vielzahl von Pixeln-von-Interesse durch Vergleichen des Bilds mit einem Hintergrundbild identifiziert wird.

4. Verfahren nach Anspruch 3, ferner umfassend Initialisieren des Hintergrundbilds.

5. Verfahren nach Anspruch 3, ferner umfassend Aktualisieren des Hintergrundbilds unter Verwendung des durch den Bildsensor (11, 63) aufgenommenen Bilds.

6. Verfahren nach Anspruch 1 oder 3, ferner umfassend Aktualisieren eines Arrays von Varianzen unter Verwendung des durch den Bildsensor (11, 63) aufgenommenen Bilds.

7. Verfahren nach Anspruch 3, ferner umfassend Aktualisieren eines Arrays von ganzen Zahlen in Übereinstimmung mit dem durch den Bildsensor (11, 63) aufgenommenen Bild.

8. Verfahren nach Anspruch 7, wobei jede ganze Zahl des Arrays von ganzen Zahlen einer Anzahl von Aktualisierungen eines Pixels des Hintergrundbilds entspricht.

9. Verfahren nach Anspruch 8, wobei der Vergleich des Bilds mit dem Hintergrundbild nur Pixel innerhalb des Bilds mit Pixeln innerhalb des Hintergrundbilds vergleicht, wenn eine jeweilige ganze Zahl des Arrays von ganzen Zahlen angibt, dass ein jeweiliges Pixel innerhalb des Hintergrundbilds mindestens eine vorbestimmte Anzahl von Malen aktualisiert wurde.

10. Verfahren nach Anspruch 1, das ferner Folgendes umfasst:

    Identifizieren des Tropfens in dem Bild und eines vorbestimmten Bands nahe einem Rand des Tropfens; und
    Initialisieren eines Hintergrundbilds durch Einstellen von jedem Pixel des Hintergrundbilds auf das Bild, es sei denn, dass es sich innerhalb des identifizierten Tropfens oder des vorbestimmten Bands nahe dem Rand des Tropfens befindet.

**11.** Verfahren nach Anspruch 10, ferner umfassend Einstellen eines Pixels des Hintergrundbilds auf einen vorbestimmten Wert, wenn sich ein entsprechendes Pixel des Bilds innerhalb des identifizierten Tropfens oder des vorbestimmten Bands nahe dem Rand des Tropfens befindet.

**12.** Verfahren nach Anspruch 11, wobei das entsprechende Pixel des Bilds einen Ort aufweist, der einem Ort des Pixels des Hintergrundbilds entspricht.

**13.** Verfahren nach Anspruch 1, ferner umfassend Bestimmen der Basislinie (215), die einer Öffnung der Tropfkammer (4, 59, 104) entspricht.

**14.** Verfahren nach Anspruch 1, ferner umfassend:

Aufnehmen (201) eines ersten Bilds unter Verwendung des Bildsensors (11, 63);
Identifizieren (202) des Tropfens innerhalb des ersten Bilds und eines vorbestimmten Bands nahe einem Rand des Tropfens;
Initialisieren (203) eines Hintergrundbilds durch Einstellen von jedem Pixel auf das erste Bild, es sei denn, dass es sich innerhalb des identifizierten Tropfens oder des vorbestimmten Bands nahe dem Rand des Tropfens befindet;
Einstellen (204) von Pixeln innerhalb einer Region des Tropfens oder innerhalb des vorbestimmten Bands auf einen vorbestimmten Wert;
Initialisieren (205) eines Arrays von ganzen Zahlen;
Initialisieren (206) eines Arrays von Varianzen; und
Aktualisieren (209) des Hintergrundbilds, des Arrays von ganzen Zahlen und des Arrays von Varianzen unter Verwendung des Bilds.

**15.** Verfahren nach Anspruch 1, wobei das Verfahren ferner die Handlung der Fernsteuerung der Durchflussrate durch einen Überwachungsclient umfasst.

**16.** Verfahren nach Anspruch 15, wobei der Überwachungsclient ein Tablet ist.

**17.** Durchflussmessgerät, das Folgendes umfasst:

einen Koppler, eingerichtet zum Koppeln einer Tropfkammer (4, 59, 104);
ein Tragglied, das betriebsfähig mit dem Koppler gekoppelt ist;
einen Bildsensor (11, 63), der ein Sichtfeld aufweist und betriebsfähig mit dem Tragglied gekoppelt ist, wobei der Bildsensor (11, 63) dafür positioniert ist, die Tropfkammer (4, 59, 104) innerhalb des Sichtfelds zu betrachten; und
mindestens einen Prozessor, der betriebsfähig mit dem Bildsensor (11, 63) gekoppelt ist, um von diesem Bilddaten zu empfangen, wobei der mindestens eine Prozessor ausgelegt ist zum:

Bestimmen einer Basislinie für einen Tropfen, der sich an einer Öffnung der Tropfkammer bildet;
Aufnehmen eines Bilds der Tropfkammer (4, 59, 104) unter Verwendung des Bildsensors (11, 63);
Identifizieren einer Vielzahl von Pixeln-von-Interesse innerhalb des Bilds;
Bestimmen (211) einer Untermenge von Pixeln innerhalb der Vielzahl von Pixeln-von-Interesse, wobei jedes Pixel der Vielzahl von Pixeln-von-Interesse als innerhalb der Untermenge von Pixeln befindlich bestimmt wird, wenn es einen Pfad zu der Basislinie (215) gibt, wobei die Basislinie der Tropfkammer (4, 59, 104) entspricht;
Durchführen (212) einer Rotationsoperation an der Untermenge von Pixeln; und
Schätzen (213) eines Volumens des Tropfens innerhalb der Tropfkammer (4, 59, 104) durch Zählen einer Anzahl von Pixeln innerhalb der rotierten Untermenge von Pixeln,

wobei der mindestens eine Prozessor ausgelegt ist zum Bestimmen der Untermenge von Pixeln innerhalb der Vielzahl von Pixeln-von-Interesse durch Bestimmen, dass sich jedes aus der Vielzahl von Pixeln-von-Interesse innerhalb der Untermenge von Pixeln befindet, wenn das jeweilige Pixel der Vielzahl von Pixeln-von-Interesse einen durchgehenden Pfad zurück zur Basislinie (215) aufweist.

**EP 3 400 928 B1**

**Revendications**

1. Procédé comprenant :

   la détermination d'une ligne de base d'une goutte se formant au niveau d'une ouverture d'une chambre compte-gouttes ;
   la capture d'une image d'une chambre compte-gouttes (4, 59, 104) à l'aide d'un capteur d'image (11, 63) ;
   l'identification d'une pluralité de pixels d'intérêt à l'intérieur de l'image ;
   la détermination (211) d'un sous-ensemble de pixels à l'intérieur de la pluralité de pixels d'intérêt, chaque pixel de la pluralité de pixels d'intérêt étant déterminé comme étant dans le sous-ensemble de pixels lorsqu'il y a un chemin vers la ligne de base (215), la ligne de base correspondant à la chambre compte-gouttes (4, 59, 104) ;
   la réalisation (212) d'une opération de rotation sur le sous-ensemble de pixels ; et
   l'estimation (213) d'un volume de la goutte à l'intérieur de la chambre compte-gouttes (4, 59, 104) par comptage d'un nombre de pixels dans le sous-ensemble de pixels ayant subi une rotation,
   l'étape de détermination du sous-ensemble de pixels à l'intérieur de la pluralité de pixels d'intérêt comprenant la détermination que chaque pixel de la pluralité de pixels d'intérêt est dans le sous-ensemble de pixels si le pixel respectif de la pluralité de pixels d'intérêt a un chemin contigu de retour vers la ligne de base (215).

2. Procédé selon la revendication 1, la ligne de base (215) étant un ensemble prédéterminé de pixels à l'intérieur du capteur d'image (11, 63).

3. Procédé selon la revendication 1, la pluralité de pixels d'intérêt étant identifiés par comparaison de l'image avec une image d'arrière-plan.

4. Procédé selon la revendication 3, comprenant en outre l'initialisation de l'image d'arrière-plan.

5. Procédé selon la revendication 3, comprenant en outre la mise à jour de l'image d'arrière-plan à l'aide de l'image capturée par le capteur d'image (11, 63).

6. Procédé selon la revendication 1 ou 3, comprenant en outre la mise à jour d'un tableau de variances à l'aide de l'image capturée par le capteur d'image (11, 63).

7. Procédé selon la revendication 3, comprenant en outre la mise à jour d'un tableau de nombres entiers conformément à l'image capturée par le capteur d'image (11, 63) .

8. Procédé selon la revendication 7, chaque nombre entier du tableau de nombres entiers correspondant à un certain nombre de mises à jour d'un pixel de l'image d'arrière-plan.

9. Procédé selon la revendication 8, la comparaison de l'image avec l'image d'arrière-plan ne comparant les pixels à l'intérieur de l'image avec les pixels à l'intérieur de l'image d'arrière-plan que si un nombre entier respectif du tableau de nombres entiers indique qu'un pixel respectif à l'intérieur de l'image d'arrière-plan a été mis à jour au moins un nombre prédéterminé de fois.

10. Procédé selon la revendication 1, comprenant en outre :

    l'identification de la goutte dans l'image et d'une bande prédéterminée près d'un bord de la goutte ; et
    l'initialisation d'une image d'arrière-plan en définissant chaque pixel de l'image d'arrière-plan sur l'image, sauf s'il est à l'intérieur de la goutte identifiée ou de la bande prédéterminée près du bord de la goutte.

11. Procédé selon la revendication 10, comprenant en outre la définition d'un pixel de l'image d'arrière-plan à une valeur prédéterminée si un pixel correspondant de l'image est à l'intérieur de la goutte identifiée ou de la bande prédéterminée près du bord de la goutte.

12. Procédé selon la revendication 11, le pixel correspondant de l'image ayant un emplacement correspondant à un emplacement du pixel de l'image d'arrière-plan.

13. Procédé selon la revendication 1, comprenant en outre la détermination de la ligne de base (215) correspondant à une ouverture de la chambre compte-gouttes (4, 59, 104).

**14.** Procédé selon la revendication 1, comprenant en outre :

la capture (201) d'une première image à l'aide du capteur d'image (11, 63) ;

l'identification (202) de la goutte à l'intérieur de la première image et d'une bande prédéterminée près d'un bord de la goutte ;

l'initialisation (203) d'une image d'arrière-plan par définition de chaque pixel sur la première image, sauf s'il est à l'intérieur de la goutte identifiée ou de la bande prédéterminée près du bord de la goutte ;

la définition (204) de pixels à l'intérieur d'une région de la goutte ou à l'intérieur de la bande prédéterminée à une valeur prédéterminée ;

l'initialisation (205) d'un tableau de nombres entiers ;

l'initialisation (206) d'un tableau de variances ; et

la mise à jour (209) de l'image d'arrière-plan, du tableau de nombres entiers et du tableau de variances à l'aide de l'image.

**15.** Procédé selon la revendication 1, le procédé comprenant en outre l'action de commander à distance le débit par un client de surveillance.

**16.** Procédé selon la revendication 15, le client de surveillance étant une tablette.

**17.** Débitmètre, comprenant :

un coupleur adapté pour être couplé à une chambre compte-gouttes (4, 59, 104) ;

un élément de support couplé de manière fonctionnelle au coupleur ;

un capteur d'image (11, 63) ayant un champ de vision et couplé de manière fonctionnelle à l'élément de support, le capteur d'image (11, 63) étant positionné pour voir la chambre compte-gouttes (4, 59, 104) à l'intérieur du champ de vision ; et

au moins un processeur couplé de manière fonctionnelle au capteur d'image (11, 63) pour recevoir des données d'images en provenance de celui-ci, l'au moins un processeur étant configuré pour :

déterminer une ligne de base d'une goutte se formant au niveau d'une ouverture d'une chambre compte-gouttes ;

capturer une image de la chambre compte-gouttes (4, 59, 104) à l'aide du capteur d'image (11, 63) ;

identifier une pluralité de pixels d'intérêt à l'intérieur de l'image ;

déterminer (211) un sous-ensemble de pixels à l'intérieur de la pluralité de pixels d'intérêt, chaque pixel de la pluralité de pixels d'intérêt étant déterminé comme étant à l'intérieur du sous-ensemble de pixels lorsqu'il y a un chemin vers la ligne de base (215), la ligne de base correspondant à la chambre compte-gouttes (4, 59, 104) ;

réaliser (212) une opération de rotation sur le sous-ensemble de pixels ; et

estimer (213) un volume de la goutte à l'intérieur de la chambre compte-gouttes (4, 59, 104) par comptage d'un nombre de pixels à l'intérieur du sous-ensemble de pixels ayant subi une rotation,

l'au moins un processeur étant configuré pour déterminer le sous-ensemble de pixels à l'intérieur de la pluralité de pixels d'intérêt en déterminant que chacun de la pluralité de pixels d'intérêt est à l'intérieur du sous-ensemble de pixels si le pixel respectif de la pluralité de pixels d'intérêt a un chemin contigu de retour vers la ligne de base (215).

FIG. 1

EP 3 400 928 B1

FIG. 2

FIG. 3

FIG. 4A

FIG. 4B

IV BAG

69

71

73

74

72

68

409

70

75

67

FIG. 5

FIG. 6

FIG. 7

84

85

79

59

DRIP
CHAMBER

80

IR
FILTER

63

90

PROCESSOR

91

ARRAY OF
LINES

FIG. 8

FIG. 9

89

TYPICAL BACKGROUND IMAGE

FIG. 10

FIG. 13

FIG. 12

FIG. 11

FIG. 14

FIG. 17

FIG. 16

FIG. 15

FIG. 18

FIG. 19

FIG. 20

FIG. 21

FIG. 22

FIG. 23

FIG. 24

FIG. 25

FIG. 26

DETERMINE A BASELINE OF A DROP FORMING AT AN OPENING OF A DRIP CHAMBER — 200

CAPTURE A FIRST IMAGE — 201

IDENTIFY THE DROP WITHIN THE FIRST IMAGE AND A PREDETERMINED BAND NEAR AN EDGE OF THE DROP — 202

INITIALIZE A BACKGROUND IMAGE BY SETTING EACH PIXEL TO THE FIRST IMAGE UNLESS IT IS WITHIN THE IDENTIFIED DROP OR A PREDETERMINED BAND NEAR THE EDGE OF THE DROP — 203

SET PIXELS WITHIN THE REGION OF THE DROP OR WITHIN THE PREDETERMINED BAND TO A PREDETERMINED VALUE — 204

INITIALIZE AN INTEGER ARRAY OF INTEGERS TO ZERO — 205

INITIALIZE AN ARRAY OF VARIANCES TO ZERO — 206

CAPTURE ANOTHER IMAGE — 207

IDENTIFY THE DROP IN THE ANOTHER IMAGE AND ANOTHER PREDETERMINED BAND NEAR AN EDGE OF THE DROP — 208

UPDATE THE BACKGROUND IMAGE, THE ARRAY OF INTEGERS, AND THE ARRAY OF VARIANCES — 209

COMPARE THE ANOTHER IMAGE TO THE BACKGROUND IMAGE AND IDENTIFY A PLURALITY OF PIXELS OF INTEREST — 210

TO FIG. 28

FIG. 27

214

214

FROM FIG. 27

DETERMINE A SUBSET OF PIXELS WITHIN THE PLURALITY OF PIXELS OF INTEREST THAT CORRESPOND TO A DROP —211

PERFORM A ROTATION OPERATION ON THE SUBSET OF PIXELS —212

ESTIMATE A VOLUME OF THE DROP WITHIN THE DRIP CHAMBER BY COUNTING THE NUMBER OF PIXELS WITHIN THE ROTATED SUBSET OF PIXELS —213

FIG. 28

FIG. 29

216

FIG. 30

217

FIG. 31

1. IF THE INPUT PIXEL IS TO THE LEFT OR RIGHT OF THE BASELINE (GREEN LINE) IN THE IMAGE, THEN ITS OUTPUT VALUE IS SET TO ZERO.

2. IF THE INPUT PIXEL'S BACKGROUND COUNT ARRAY INDICATES THAT FEWER THAN A PRE-DETERMINED NUMBER OF IMAGES (E.G., 100) HAVE BEEN USED TO MAKE THIS PIXEL'S BACKGROUND VALUE, THEN

    a. IF THE INPUT PIXEL'S INTENSITY IS LESS THAN THE THRESHOLD INTENSITY (E.G., 140 IN AN RANGE OF 0 - 255), THEN SET THE PIXEL'S OUTPUT VALUE TO NOT - ZERO (255).

    b. IF THE INPUT PIXEL'S INTENSITY IS GREATER THAN OR EQUAL TO THE THRESHOLD INTENSITY, THEN SET THE PIXEL'S OUTPUT VALUE TO ZERO.

3. IF THE INPUT PIXEL'S BACKGROUND COUNT ARRAY IS GREATER THAN THE PRE-DETERMINED NUMBER OF IMAGES , THEN:

    a. IF THE SQUARE OF THE DIFFERENCE BETWEEN THE INPUT PIXEL INTENSITY AND THE BACKGROUND PIXEL INTENSITY IS GREATER THAN THE PIXEL'S ESTIMATE OF BACKGROUND VARIANCE TIMES A

    CONSTANT $\gamma^2$ , THEN SET THE PIXEL'S OUTPUT VALUE TO NOT-ZERO (255). *(THIS EFFECTIVELY SAYS IF THE CURRENT PIXEL VALUE IS MORE THAN $\gamma$, THEN THE PIXEL IS DISTINCT.)*

    b. IF THE SQUARE OF THE DIFFERENCE BETWEEN THE INPUT PIXEL INTENSITY AND THE BACKGROUND PIXEL INTENSITY IS LESS THAN OR EQUAL TO THE PIXEL'S ESTIMATE OF BACKGROUND VARIANCE TIMES

    A CONSTANT $\gamma^2$, THEN SET THE PIXEL'S OUTPUT VALUE TO ZERO.

# FIG. 32

FIG. 33

FIG. 34

FIG. 35

FIG. 36

1. PUSH THE LOCATION OF THE FIRST PIXEL ONTO A STACK

2. WHILE THE STACK IS NOT EMPTY:

    a. POP THE NEXT LOCATION $(i,j)$ OFF OF THE STACK

    b. MAKE THE OUTPUT PIXEL VALUE AT $(i,j)$ WHITE

    c. EXAMINE THE EIGHT PIXELS ADJACENT TO $(i,j)$ :

        i. IF THE ADJACENT INPUT PIXEL $(\iota, \varphi)$ IS WHITE, BUT THE OUTPUT PIXEL $(\iota, \varphi)$ IS BLACK ADD THE LOCATION $(\iota, \varphi)$ TO THE STACK

    d. RETURN TO 2

# FIG. 37

FIG. 38

FIG. 39

CAMERA TO FOCUS DISTANCE (m)

BLUR CIRCLE DIVIDED BY PIXEL SIZE FOR A 20mm FOCAL LENGTH LENS

FIG. 40

CAMERA TO FOCUS DISTANCE (m)
BLUR CIRCLE DIVIDED BY PIXEL SIZE FOR A 40mm FOCAL LENGTH LENS

FIG. 41

| LENS FOCAL LENGTH | LENS SEPARATION FROM THE CAMERA | FOCUS SEPARATION FROM THE CAMERA | FIELD OF VIEW | DEPTH OF FIELD $(D_{BLUR}/D_{PIXEL})=1$ | DEPTH OF FIELD $(D_{BLUR}/D_{PIXEL})=2$ |
|---|---|---|---|---|---|
| 20mm | 0mm | 18.6mm | ±9.7mm | ±2mm | ±4mm |
| 20mm | 60mm | 78.2mm | ±12.3mm | ±3mm | ±6mm |
| 40mm | 0mm | 34.8mm | ±18.2mm | ±5mm | >10mm |
| 40mm | 60mm | 93.5mm | ±22.6mm | ±10mm | >10mm |
| 60mm | 0mm | 48.9mm | ±25.6mm | >10mm | >10mm |
| 60mm | 60mm | 106.4mm | ±31.4mm | >10mm | >10mm |

# FIG. 42

125 mL/hr

0.9%
Sodium Chloride

FIG. 43

FIG. 44

FIG. 45

FIG. 46

FIG. 47

FIG. 48

FIG. 49

FIG. 50A

FIG. 50B

FIG. 51A

FIG. 51B

FIG. 51C

FIG. 51D

FIG. 52A

FIG. 52B

FIG. 52C

FIG. 52D

FIG. 53A

FIG. 53B

FIG. 53C

352

368

FIG. 54

FIG. 55

FIG. 56A

FIG. 56B

FIG. 56C

FIG. 57A

FIG. 57B

FIG. 57C

FIG. 57D

FIG. 57E

FIG. 58A          FIG. 58B

FIG. 58C

FIG. 58D

FIG. 59A

FIG. 59B

FIG. 59C

FIG. 59D

FIG. 59E

FIG. 59F

FIG. 59G

FIG. 59H

FIG. 60

FIG. 61

FIG. 62

FIG. 63

FIG. 64A

FIG. 64B

FIG. 65

FIG. 66A

FIG. 66B

EP 3 400 928 B1

FIG. 66C

FIG. 66D

FIG. 66E

FIG. 66F

FIG. 66G

EP 3 400 928 B1

408

mL/HR

FLOW

◇ mL/HR

DISTANCE

FIG. 67

FIG. 68A

EP 3 400 928 B1

2.5 GHz
LOOP

2.5 GHz
COUPLING
RESONATOR

TX

2.5 GHz
TRANSCEIVER

RX

WATER
COLUMN

2.5 GHz
LOOP

2.5 GHz
COUPLING
RESONATOR

FIG. 68B

706

708

707

709

EP 3 400 928 B1

712

720

710

711

715

713

714

FIG. 69A

FIG. 69B

EP 3 400 928 B1

706

708

709

707

713

715

714

712

720

FIG. 69C

154

706

708

709

707

710

713

715

712

711

714

720

FIG. 69D

FIG. 69E

706

708

713

709

715

707

710

711

712

720

714

FIG. 69F

728

```
CAPTURE AN IMAGE OF A DRIP CHAMBER                    ~729

            ↓

POSITION A TEMPLATE WITHIN THE CAPTURED IMAGE TO     ~730
            A FIRST POSITION

            ↓

AVERAGE THE PIXELS WITH THE TEMPLATE TO             ~731
          DETERMINE A FIRST AVERAGE

            ↓

MOVE THE TEMPLATE TO A SECOND POSITION              ~732

            ↓

AVERAGE THE PIXELS WITHIN THE TEMPLATE TO           ~733
         DETERMINE A SECOND AVERAGE

            ↓

IF A DIFFERENCE BETWEEN THE SECOND AVERAGE AND
THE FIRST AVERAGE IS GREATER THAN A PREDETERMINED    ~734
THRESHOLD VALUE, DETERMINE THAT THE TEMPLATE IS
        LOCATED AT AN EDGE OF A DROP

            ↓

CORRELATE THE SECOND POSITION WITH A VOLUME OF      ~735
              THE DROP
```

FIG. 70

FIG. 71A

FIG. 71B

FIG. 71C

FIG. 71D

FIG. 71E

FIG. 72

FIG. 73A

741

744

742     745     743

FIG. 73B

FIG. 73C

746

747

FIG.74

FIG.75

FIG. 76A

755

1000

1002

1004

756

757

758

Fig. 76B

FIG. 77

FIG. 78

FIG. 79

FIG. 80

FIG. 81

EP 3 400 928 B1

803

| CAPTURE A FIRST IMAGE | 804 |

↓

| CREATE A FIRST THRESHOLDED IMAGE FROM THE FIRST IMAGE BY COMPARING EACH PIXEL OF THE FIRST IMAGE TO A THRESHOLD VALUE (E.G., 0 IF ABOVE OR 1 IF BELOW) | 805 |

↓

| DETERMINE A SET OF PIXELS WITHIN THE FIRST THRESHOLDED IMAGE CONNECTED TO A PREDETERMINED SET OF PIXELS WITHIN THE FIRST THRESHOLDED IMAGE | 806 |

↓

| FILTER ALL REMAINING PIXELS OF THE FIRST THRESHOLDED IMAGE THAT ARE NOT WITH THE SET OF PIXELS. THE FILTER OPERATES ON A PIXEL-BY-PIXEL BASIS WITHIN THE TIME DOMAIN TO GENERATE A FIRST FILTERED IMAGE | 807 |

↓

| REMOVE PIXELS DETERMINED TO NOT BE PART OF A DROP FROM THE FIRST THRESHOLDED IMAGE USING THE FIRST FILTERED IMAGE TO GENERATE A SECOND IMAGE | 808 |

↓

| DETERMINE A SECOND SET OF PIXELS WITHIN THE SECOND IMAGE CONNECTED TO A PREDETERMINED SET OF PIXELS WITHIN THE SECOND IMAGE TO GENERATE A THIRD IMAGE. THE THIRD IMAGE IDENTIFIES THE SECOND SET OF PIXELS WITHIN THE SECOND IMAGE | 809 |

↓

| DETERMINE A FIRST LENGTH OF THE DROP BY COUNTING THE NUMBER OF ROWS CONTAINING PIXELS CORRESPONDING TO THE SECOND SET OF PIXELS WITHIN THE THIRD IMAGE. THE FIRST LENGTH CORRESPONDING TO A FIRST ESTIMATED DROP SIZE | 810 |

↓

( TO FIG. 82B )

## FIG. 82A

FROM FIG. 82A

UPDATE A BACKGROUND IMAGE USING THE FIRST IMAGE ──811

CREATE A SECOND THRESHOLDED IMAGE BY COMPARING FIRST IMAGE WITH THE BACKGROUND IMAGE ──812

SUM THE ROWS OF SECOND THRESHOLDED TO CREATE A PLURALITY OF ROW SUMS. EACH ROW SUM CORRESPONDS TO A ROW OF THE SECOND THRESHOLDED IMAGE ──813

START AT A ROW POSITION OF THE SECOND THRESHOLDED IMAGE HAVING A FIRST SUM OF THE PLURALITY OF SUMS THAT CORRESPONDS TO THE FIRST LENGTH ──814

INCREMENT THE ROW POSITION ──815

DOES THE PRESENT ROW POSITION CORRESPOND TO A CORRESPONDING ROW SUM THAT IS ZERO? ──816

NO

YES

DETERMINE A SECOND LENGTH IS EQUAL TO THE PRESENT ROW POSITION. THE SECOND LENGTH CORRESPONDING TO A SECOND ESTIMATED DROP SIZE ──817

AVERAGE THE FIRST AND SECOND LENGTHS TO DETERMINE AN AVERAGE LENGTH. THE AVERAGE LENGTH CORRESPONDING TO A THIRD ESTIMATED DROP SIZE ──818

FIG. 82B

900

CAPTURE AN IMAGE OF A DRIP CHAMBER ──902

PERFORM A CANNY EDGE DETECTION OPERATION ON THE IMAGE TO GENERATE A FIRST PROCESSED IMAGE ──904

PERFORM AN AND-OPERATION ON A PIXEL ON A FIRST SIDE OF AN AXIS OF THE FIRST PROCESSED IMAGE WITH A CORRESPONDING MIRROR PIXEL ON THE SECOND SIDE OF THE AXIS OF THE FIRST PROCESSED IMAGE ──906

YES     MORE PIXELS TO PROCESS?     ──908

NO

THE AND-ED PIXELS FORM A SECOND PROCESSED IMAGE ──910

FIG. 83

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 20050039780
- US 6159186 A
- US 4936828 A

**Non-patent literature cited in the description**

- **J. MATAS ; C. GALAMBOS ; J. KITTLER.** *Progressive Probabilistic Hough Transform,* 1998